# EUROPÄISCHE PATENTSCHRIFT

(11) **EP 2 683 701 B1**
(45) Veröffentlichungstag und Bekanntmachung des Hinweises auf die Patenterteilung: **24.12.2014**
(21) Anmeldenummer: 12707597.6
(22) Anmeldetag: 07.03.2012
(51) Int. Cl.: C07D 291/06, C07D 419/10, C07D 419/14, A61K 31/54, A61P 3/10

(54) **MIT BENZYL- ODER HETEROMETHYLENGRUPPEN SUBSTITUIERTE OXATHIAZINDERIVATE, VERFAHREN ZU DEREN HERSTELLUNG, IHRE VERWENDUNG ALS MEDIKAMENT SOWIE SIE ENTHALTENDES ARZNEIMITTEL UND DEREN VERWENDUNG**
OXATHIAZINE DERIVATIVES SUBSTITUTED WITH BENZYL OR HETEROMETHYLENE GROUPS, METHOD FOR THEIR PREPARATION, THEIR USAGE AS MEDICAMENT, MEDICAMENT CONTAINING SAME AND ITS USE
DÉRIVÉS D'OXATHIAZINE SUBSTITUÉS PAR DES GROUPES DE BENZYLE-MÉTHYLES OU D'HÉTÉRO-MÉTHYLES, LEUR PROCÉDÉ DE FABRICATION, LEUR UTILISATION COMME MÉDICAMENT AINSI QUE MÉDICAMENTS EN ÉTANT POURVU ET LEUR UTILISATION

(30) Priorität: 08.03.2011 EP 11305246
(43) Veröffentlichungstag der Anmeldung: 15.01.2014
(73) Patentinhaber: SANOFI, 75008 Paris (FR)
(72) Erfinder: BOEHME, Thomas, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); RITTER, Kurt, 65926 Frankfurt am Main (DE); TSCHANK, Georg, 65926 Frankfurt amMain (DE)
(74) Vertreter: Essler, Frank
(86) Internationale Anmeldenummer: PCT/EP2012/053941
(87) Internationale Veröffentlichungsnummer: WO 2012/120058

(56) Entgegenhaltungen:
- JP-A- 60 214 743
- DATABASE CAPLUS [Online] CHEMICAL ABSTRACTS SERVICE, COLUMBUS, OHIO, US; SUZUE, SEIGO ET AL: "Hypoglycemic agents. IV. Synthesis of 1,4,3-benzoxathiazine 4,4-dioxides", XP002656994, gefunden im STN Database accession no. 1968:467344

## Beschreibung

Die Erfindung betrifft substituierte Oxathiazinderivate und deren physiologisch verträgliche Salze.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Diabetes, Hyperglykämie, Insulinresistenz, Adipositas oder Lipidstoffwechselstörungen geeignet sind.

Die Erfindung betrifft daher Verbindungen der Formel I 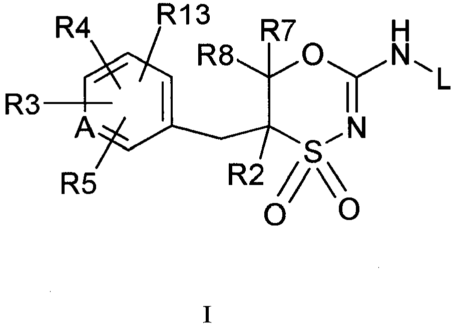 worin bedeuten
- A: CH, N;
- L: R1, -CH(R10)(R11);
- R10, R11: unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆-Alkyl)₂, COOH, COO-(C₁-C₆-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R6: H, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
- R1: 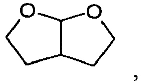 (C₆-C₁₀)-Aryl, (C₃-C₈)-Carbocyclyl, wobei der 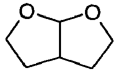 -rest, Arylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: H, F, (C₁-C₃)-Alkyl, wobei der Alkylrest ein bis 3-fach mit Fluor substituiert sein kann;
- R3, R4, R5, R13: unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂,-O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, -(C₁-C₆)-Alkylen-(C₆-C₁₀)-Aryl,, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
oder R4 und R5 bilden gemeinsam eine -CH=CH-CH=CH- Kette.
- R7, R8: unabhängig voneinander H, (C₁-C₃)-Alkyl einfach oder mehrfach mit Fluor substituiert, oder R7 und R8 bilden gemeinsam mit dem Kohlenstoffatom an das sie gebunden sind einen 3-8 gliedrigen Carbocyclus oder Heterocyclus;
- R9: H, OH, OCH₃, OCF₃, CHF₂, CF₃;
sowie deren pharmazeutisch verträgliche Salze.

Bevorzugt sind Verbindungen der Formel I, worin bedeuten
- A: CH, N;
- L: R1, -CH(R10)(R11);
- R10, R11: unabhängig voneinander H, F, Cl, Br, I, OH, CF₃, CHF₂, CH₂F, NO₂, CN, OCF₃, OCHF₂, O-(C₁-C₆-Alkyl, C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R6: H, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
- R1: (C₆-C₁₀)-Aryl, (C₃-C₈)-Carbocyclyl, wobei der Arylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: H, F, (C₁-C₃)-Alkyl, wobei der Alkylrest ein bis 3-fach mit Fluor substituiert sein kann;
- R3, R4, R5, R13: unabhängig voneinander H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅.;
oder R4 und R5 bilden gemeinsam eine -CH=CH-CH=CH- Kette;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

Weiter bevorzugt sind Verbindungen der Formel I, worin bedeuten
- A: CH, N;
- L: R1, -CH(R10)(R11);
- R10, R11: unabhängig voneinander (C₁-C₆)-Alkyl, (C₁-C₆)-Alkylen-(R6), (C₆-C₁₀)-Aryl, -(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R6: H, OH, O-(CO)-NH₂, SO₂NH₂;
- R1: (C₃-C₈)-Carbocyclyl, wobei der Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2: H, F, (C₁-C₃)-Alkyl, wobei der Alkylrest ein bis 3-fach mit Fluor substituiert sein kann;
- R3: F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R4, R5, R13: unabhängig voneinander H, F, Cl, Br, I, OH, CF₃, NO₂, CN, OCF₃, -(C₁-C₆)-Alkylen-(R9), O-(C₁-C₆)-Alkylen-(R9), NH₂;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

Weiter bevorzugt sind Verbindungen der Formel I, worin bedeuten
- A: CH, N;
- L: R1, -CH(R10)(R11);
- R10: (C₁-C₆)-Alkyl, (C₁-C₆)-Alkylen-(R6);
- R11: (C₆-C₁₀)-Aryl;
wobei der Arylrest oder Heteroarylrest ein bis 3-fach substituiert sein kann mit F, Cl, Br, (C₁-C₆)-Alkyl;
- R6: OH;
- R1: 2,2,2-Bicyclooctyl,
wobei der 2,2,2-Bicyclooctylrest ein bis 3-fach substituiert sein kann mit F, Cl, Br;
- R2: H, (C₁-C₃)-Alkyl;
- R3: F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Pyridin, Tetrahydropyridin, Piperidin, Morpholin, Piperazin, wobei der Pyridin-, Tetrahydropyridin-, Piperidin-, Morpholin- oder Piperazinrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, -(C₁-C₆)-Alkylen-(C₃-C₈)-Cycloalkyl, 4 bis12-gliedriger Heterocyclus;
- R4, R5, R13: unabhängig voneinander H, F, Cl, Br, I, OH, CF₃, NO₂, CN, OCF₃, -(C₁-C₆)-Alkylen-(R9), O-(C₁-C₆)-Alkylen-(R9), NH₂;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

Weiter bevorzugt sind Verbindungen der Formel I, worin bedeuten
- A: CH, N;
- L: R1, -CH(R10)(R11);
- R10: (C₁-C₆)-Alkyl, (C₁-C₆)-Alkylen-(R6);
- R11: (C₆-C₁₀)-Aryl;
wobei der Arylrest oder Heteroarylrest ein bis 3-fach substituiert sein kann mit F, Cl, Br, (C₁-C₆)-Alkyl;
- R6: OH;
- R1: 2,2,2-Bicyclooctyl,
wobei der 2,2,2-Bicyclooctylrest ein bis 3-fach substituiert sein kann mit F, Cl, Br;
- R2: H, (C₁-C₃)-Alkyl;
- R3: F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, CF₃;
Pyridin, Tetrahydropyridin, Piperidin, Morpholin, Piperazin,
wobei der Pyridin-, Tetrahydropyridin-, Piperidin-, Morpholin- oder Piperazinrest ein bis 3-fach substituiert sein kann mit F, Cl, Br, CF₃, (C₁-C₆)-Alkylen-(R9), COO-(C₁-C₆)-Alkylen-(R9), (C₃-C₈)-Cycloalkyl, Oxetan;
- R4, R5, R13: unabhängig voneinander
H, F, Cl, Br, I, OH, CF₃, NO₂, CN, OCF₃, -(C₁-C₆)-Alkylen-(R9), O-(C₁-C₆)-Alkylen-(R9), NH₂;
- R7, R8: unabhängig voneinander (C₁-C₃)-Alkyl;
- R9: H, OH, OCH₃, OCF₃, CHF₂, CF₃;
sowie deren pharmazeutisch verträgliche Salze.

In einer weiteren Ausführungsform sind Verbindungen der Formel I bevorzugt, die einen Rest

R3 ungleich H in Parastellung aufweisen.

Können Reste oder Substituenten mehrfach in den Verbindungen der Formeln I auftreten, so können sie alle unabhängig voneinander die angegebene Bedeutung haben und gleich oder verschieden sein.

Falls der Rest A in den Verbindungen der Formel I die Defintion -CH- aufweist, kann das Kohlenstoffatom dieser Gruppe durch R3 oder R5 substituiert sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Alkylenrest wird eine geradkettige oder verzweigte Kohlenwasserstoffkette mit zwei freien Valenzen verstanden, wie z.B. Methylen, Ethylen, iso-Propylen, tert.-Butylen.

Unter einem Carbozyklus bzw. Carbocyclylrest wird ein Ring, welcher gesättigt oder partiell ungesättigt (mit einer oder zwei Doppelbindungen) vorliegt, verstanden, der ausschließlich aus Kohlenstoffatomen aufgebaut ist.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydrobenzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterocyclen bzw. heterocyclischen Reste können ein oder mehrfach mit geeigneten Gruppen wie oben beschrieben substituiert sein.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung.

Bevorzugt sind säure- und magensaftresistente Formulierungen. Geeignete magensaftresistente Beschichtungen umfassen Celluloseacetatphthalat, Poylvinylacetatphthalat, Hydroxyopylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2009, Kapitel 12 genannt sind; alle Abmagerungsmittel/Appetitzügler, die in der Roten Liste 2009, Kapitel 1 genannt sind; alle Lipidsenker, die in der Roten Liste 2009, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder Levemir® (insulin detemir) oder solche, wie sie in WO2005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe,
Biguanidine,
Meglitinide,
Oxadiazolidindione,
Thiazolidindione,
Glukosidase-Inhibitoren,
Hemmstoffe der Glykogenphosphorylase,
Glukagon-Antagoni sten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden oder die, die in WO2006045799 beschrieben sind (Solvay),
Inhibitoren der Dipeptidylpeptidase-IV (DPP-IV),
Insulin-Sensitizer,
Inhibitoren von Leberenzymen, die an der Stimulation der Glukoneogenese und/oder
Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1B (PTP1B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699
verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, G1 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit duetact™, einer festen Kombination von Pioglitazon Hydrochlorid mit Glimepirid, verabreicht.

Bei einer Ausführungsform der Erfindung wird die Verbindung der Formel I in Kombination mit Avandamet^{®}, einer festen Kombination von Rosiglitazon Maleat mit Metformin Hydrochlorid, verabreicht.

Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in WO2005085226, WO2005121091, WO2006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in WO2006002342, WO2006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in WO2006045565, WO2006045564, WO2006069242 beschrieben sind, verabreicht.

Bei einer anderen Ausführungsform der Erfindung wird die Verbindung der Formel I in Kombination mit einem Aqonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einer die Insulinsekretion verstärkende Substanz, wie z. B. KCP-265 (WO2003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in WO2003074500, WO2003106456, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia™, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht.

Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814, WO2005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804, S-2367 oder wie sie z. B. in WO2006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
CB1R (Cannabinoid Rezeptor 1) Antagonisten (wie z.B. Rimonabant, SR147778, SLV-319, AVE-1625, MK-0364 oder Salze davon oder solche wie sie in z. B. EP 0656354, WO 00/15609, WO2001/64632, WO2001/64633, WO2001/64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind); Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002089729, WO2002002744, WO2003004027, FR2868780, WO2006010446, WO2006038680, WO2006044293, WO2006044174 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. Lorcaserin Hydrochlorid (APD-356) oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189 beschrieben;
Inhibitoren der Fettsäuresynthase (FAS) wie z.B. C75 oder solchen, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIR1, eines Mitglieds der humanen Sirtuinenzymfamilie.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

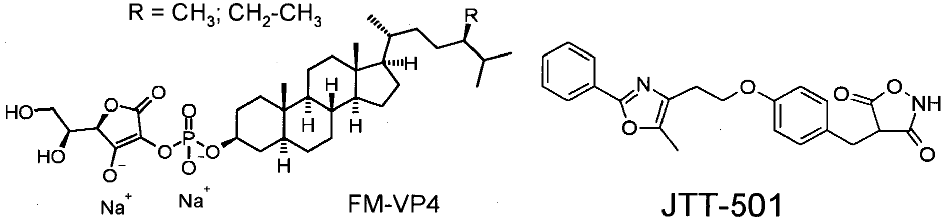

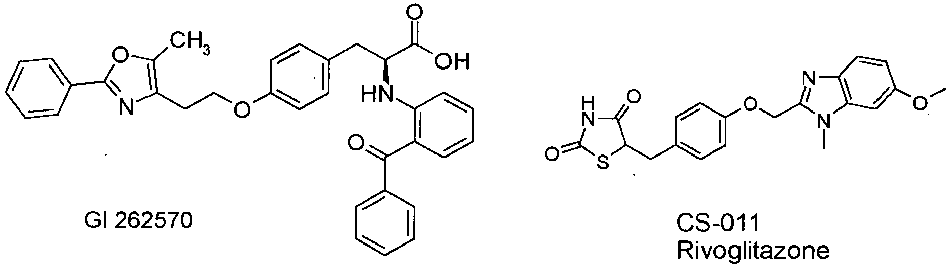

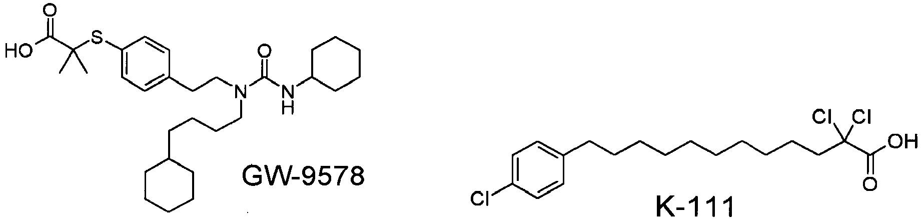

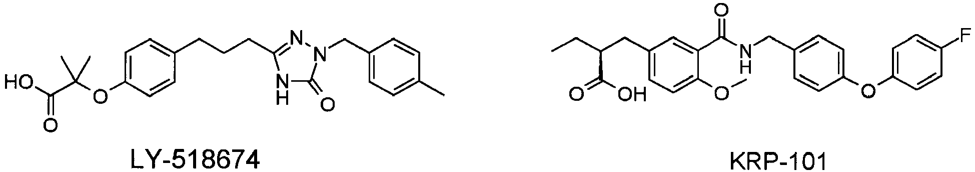

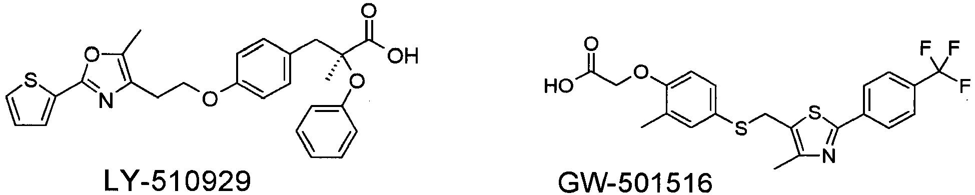

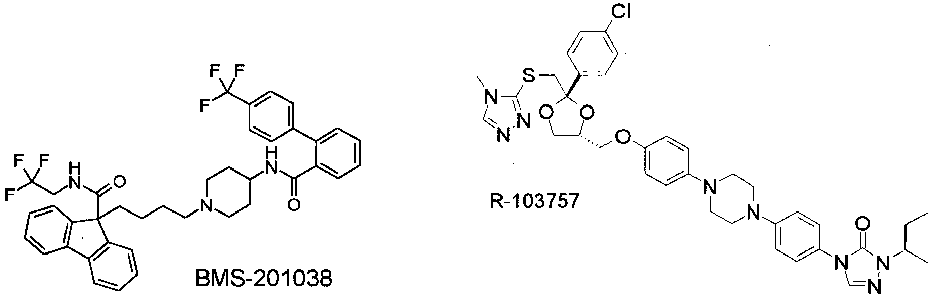

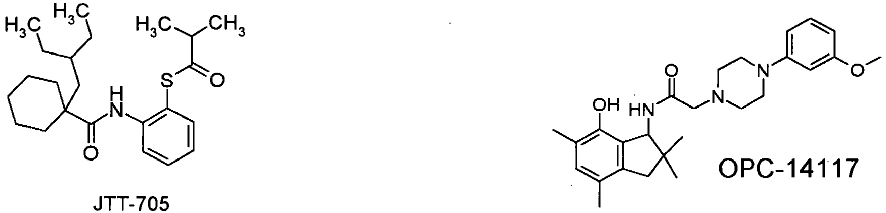

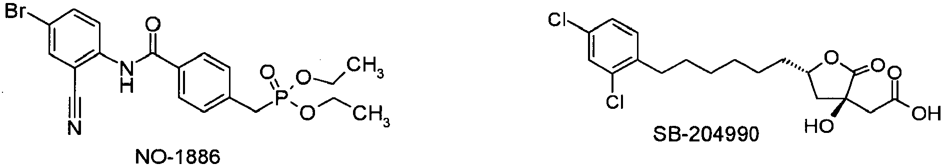

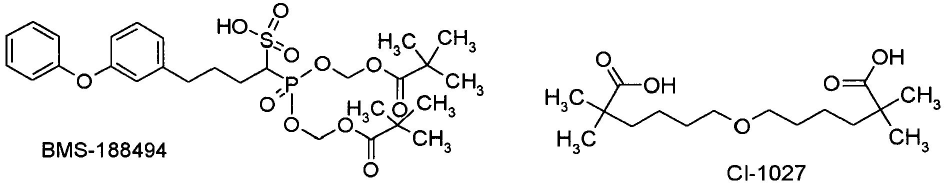

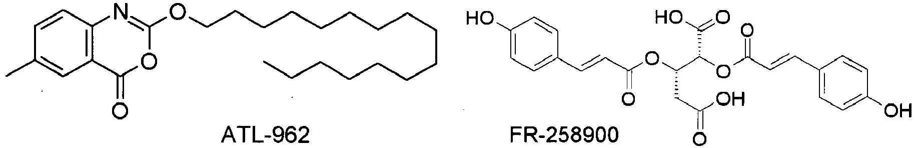

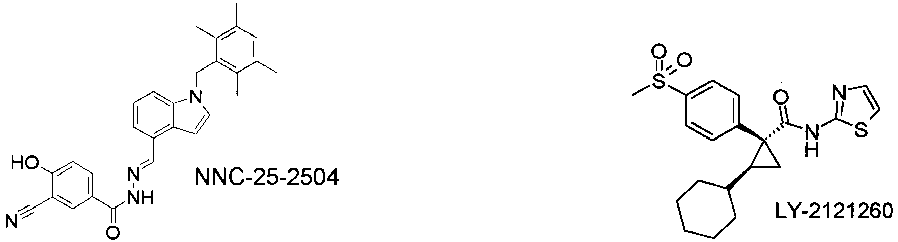

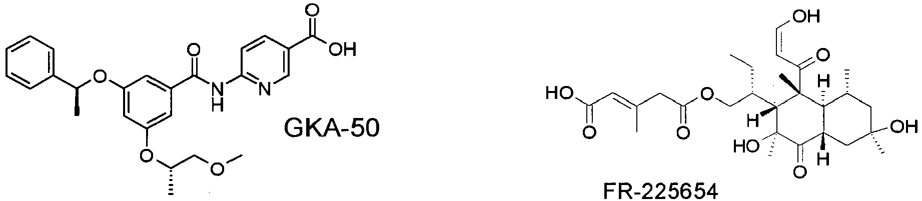

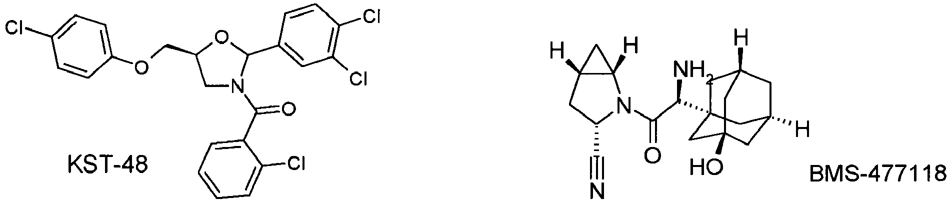

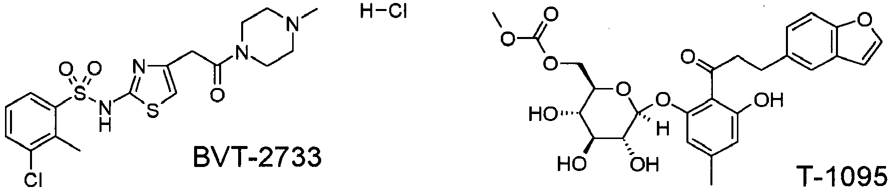

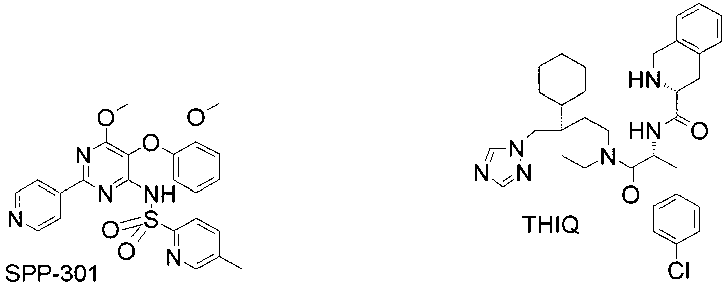

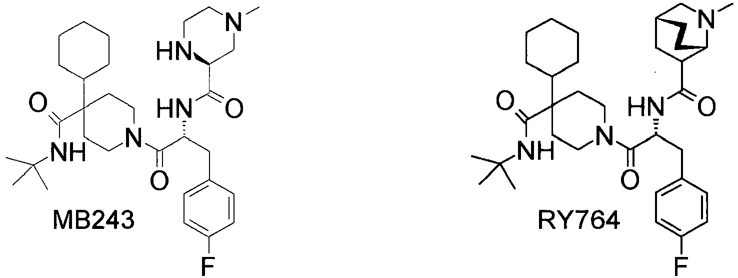

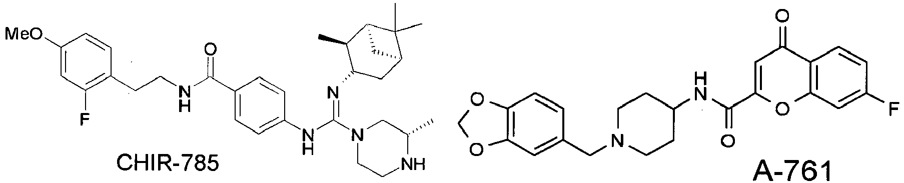

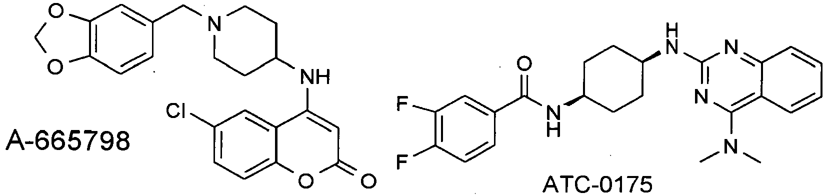

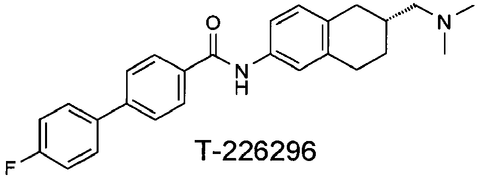

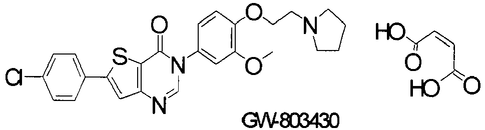

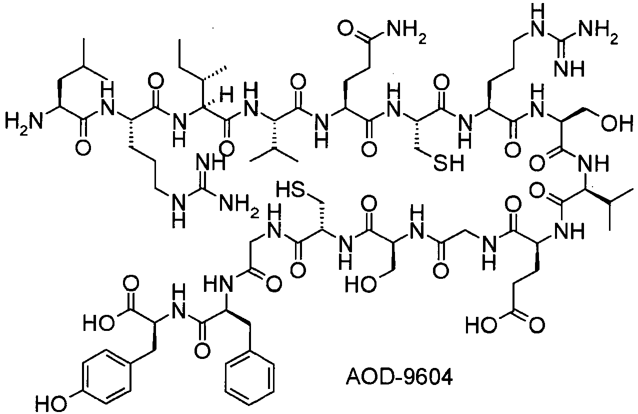

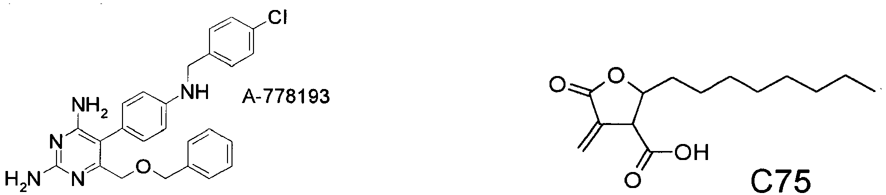

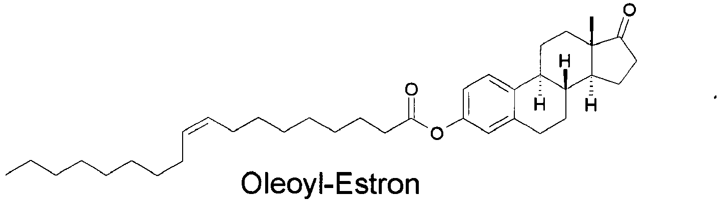

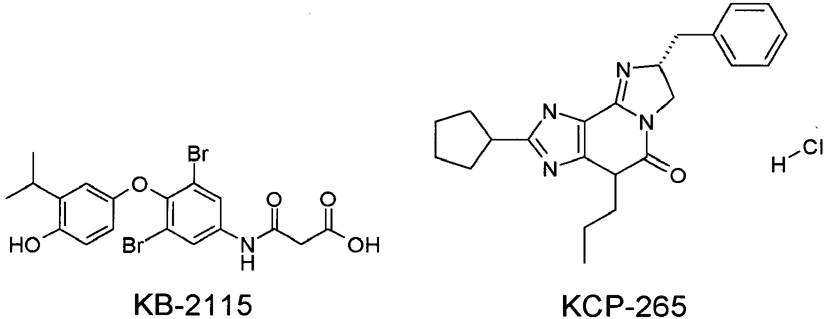

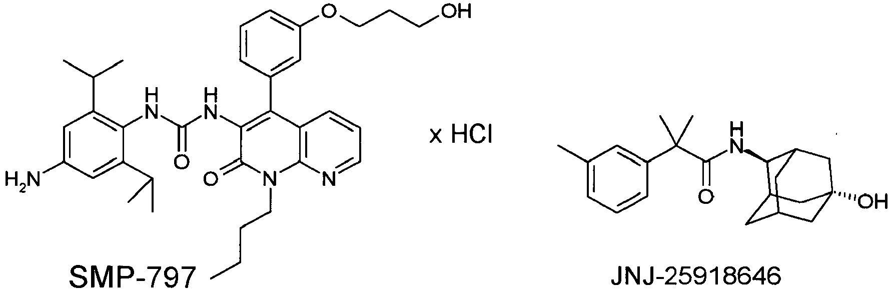

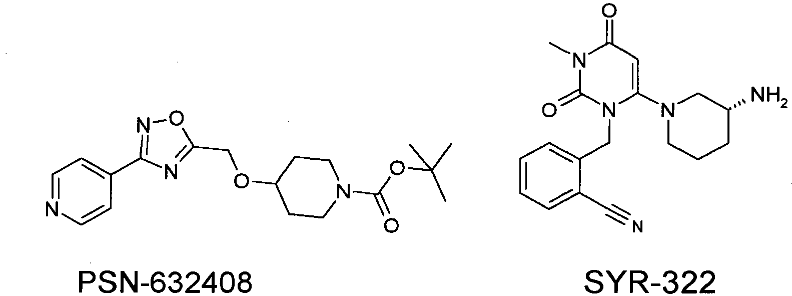

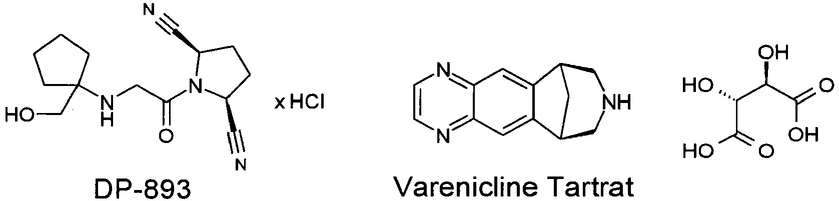

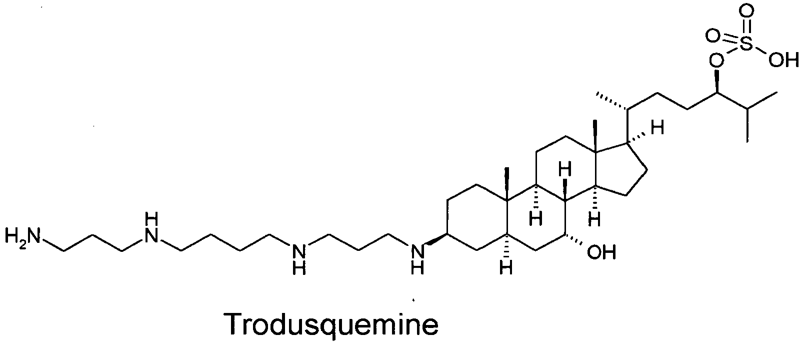

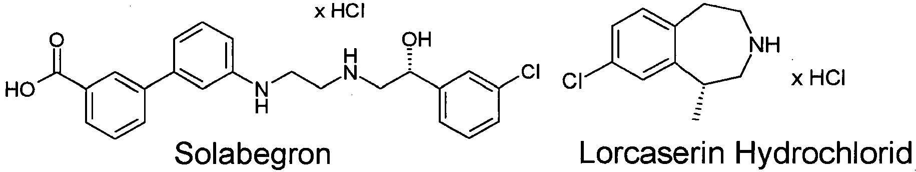

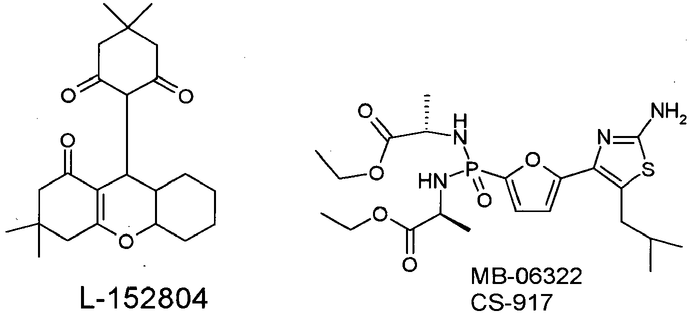

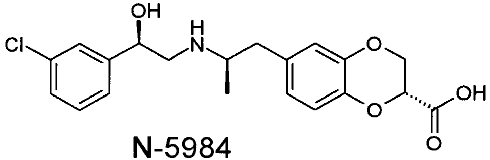

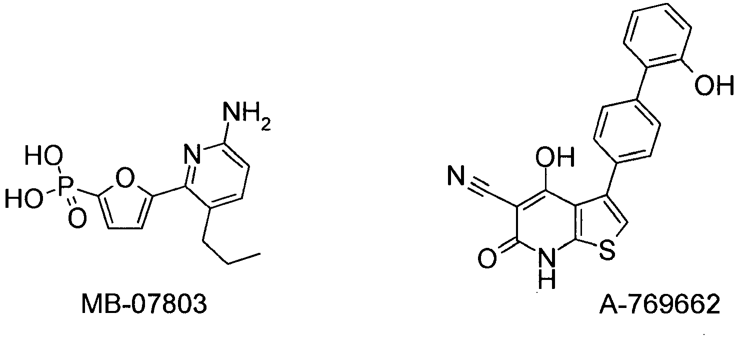

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

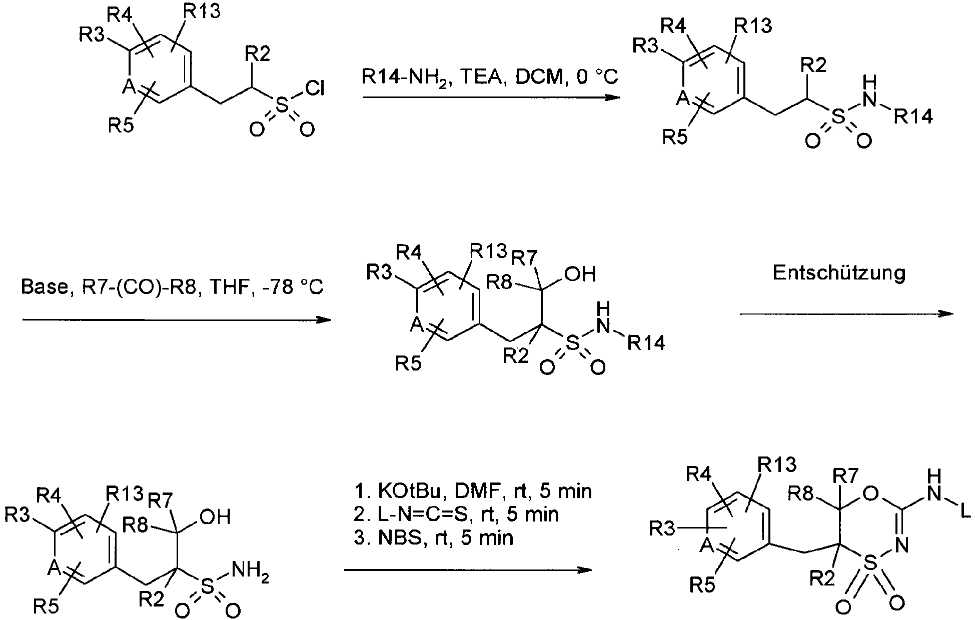

Aus einem mit Benzyl- oder Heteromethylen- sowie R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechend substituiertes und mit R14 (z.B. Boc, Benzyl, 2,4-Dimethoxybenzyl) geschütztes Methansulfonsäureamid hergestellt. Durch Behandlung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R14 geschützten Hydroxysulfonamid umgesetzt wird. Durch Entschützung von R14 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhaltenen Hydroxysulfonamids mit Base und einem Isothiocyanat (L-N=C=S) in einem geeigneten Lösungsmittel z.B. Dimethylformamid (DMF) sowie nachfolgendem oxidativen Ringschluss mit N-Bromsuccinimid (NBS) liefert die gewünschten 4,4-dioxo-oxathiazine.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Die verwendeten Isothiocyanate werden durch die Reaktion eines primären Amins mit Thiocarbonyldiimidazol erhalten, wobei eventuell vorhandene störende funktionelle Gruppen, z.B. Hydroxylgruppen mit geeigneten Schutzgruppen, z.B. Silylether blockiert werden. Die Schutzgruppen werden am Ende der Sequenz durch geeignete Verfahren entfernt, z.B. Silylgruppen durch Behandlung mit methanolischer Salzsäure.

In den Fällen, in denen während der Synthese Diastereomere oder Racemate entstehen, können diese durch präparative HPLC voneinander getrennt werden.

Einige der eingesetzten primären Amine sind kommerziell erhältlich.

4-Fluoro-bicyclo[2.2.2]octan-1-amin kann hergestellt werden wie in der Literatur beschrieben (JOC 1982, 47, 1952-7).

Andere erfindungsgemäße Verbindungen können auf weiteren Wegen erhalten werden, die im folgenden Schema beispielhaft skizziert sind.

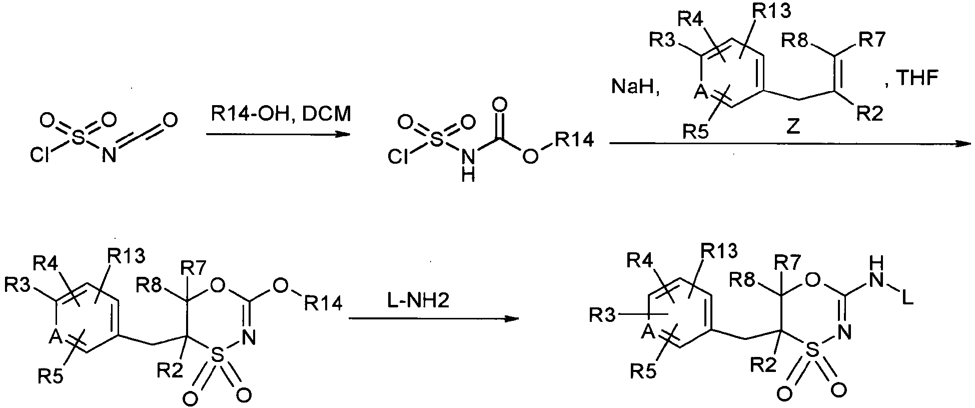

In Analogie zu einem literaturbekannten Verfahren (JACS 1972, 94, 4386-7) wird Chrosulfonylisocyanat mit einem Alkohol (R14-OH, z.B. Methanol) umgestzt, wobei ein entsprechendes Carboalkoxysulfamoylchlorid entsteht (z.B. Carbomethoxysulfamoylchlorid). Dieses wird mit Natriumhydrid deprotoniert und die nach Chloridabspaltung entstehende Zwischenstufe (z.B. Methyl-N-Sulfonylurethan) wird in einer 2+4-Cycloaddition mit einem geeigneten Alken der Formel Z zum alkoxysubstitierten (z.B. methoxysubstitierten) 4,4-Dioxooxathiazins umgesetzt. Die Alkoxygruppe (-O-R14) wird nun in einem geeigneten Lösemittel (z.B. Dichlormethan) durch ein Amin (L-NH₂) ersetzt, wobei die gewünschten 4,4-Dioxooxathiazine entstehen.

In den Fällen, in denen während der Synthese Diastereomere oder Racemate entstehen, können diese durch präparative HPLC voneinander getrennt werden.

Andere erfindungsgemäße Verbindungen können auf weiteren Wegen erhalten werden, die im folgenden Schema beispielhaft skizziert sind.

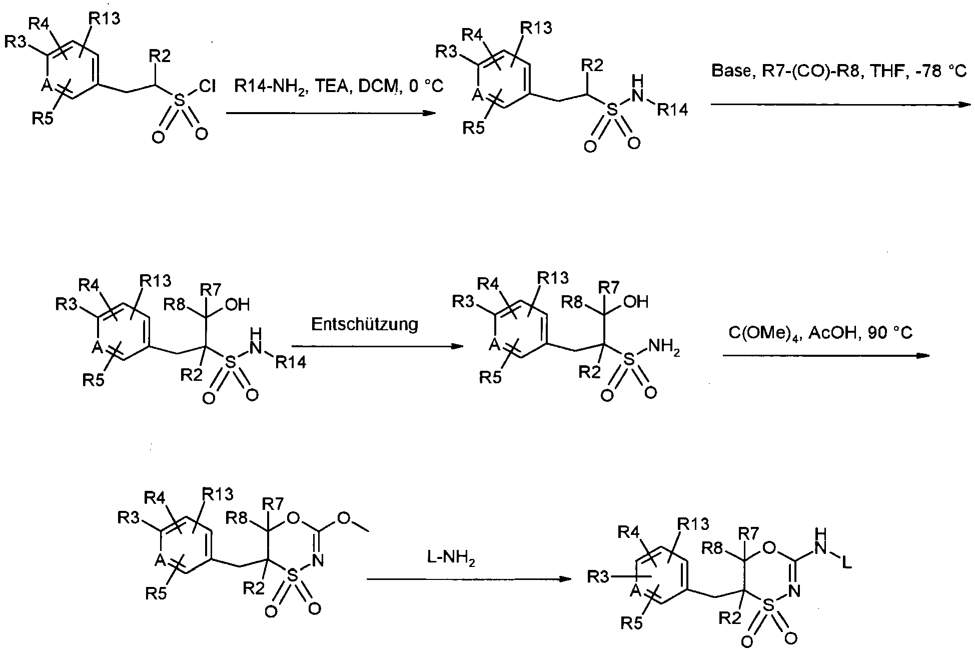

Aus einem mit Benzyl- oder Heteromethylen- sowie R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechend substituiertes und mit R14 (z.B. Boc, Benzyl, 2,4-Dimethoxybenzyl) geschütztes Methansulfonsäureamid hergestellt. Durch Behandlung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R14 geschützten Hydroxysulfonamid umgesetzt wird. Durch Entschützung von R14 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhatenen Hydroxysulfonamids mit Orthokohlensäuretetramethylester liefert methoxysubstitierte 4,4-Dioxooxathiazine. Die Methoxygruppe (-O-CH₃) wird nun in einem geeigneten Lösemittel (z.B. Dichlormethan) durch ein Amin (L-NH₂) ersetzt, wobei die gewünschten 4,4-Dioxooxathiazine entstehen.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Falls das entstehende Molekül eine Halogenaryleinheit beinhaltet, kann das Halogen durch gängige metallkatalyiserte Kupplungsverfahren ersetzt werden. So kann z.B. ein Bromid mit Hilfe einer Suzuki-Reaktion oder eine Sonogashira-Reaktion oder einer palladiumkatalysierten Aminierung weiter umgesetzt werden. Weiterhin lassen sich in einigen Fällen einfache Syntheseschritte anschließen, so kann z.B. eine Dreifachbindung zur Einfachbindung hydriert werden oder ein Bocgeschütztes Amin kann entschützt und anschließend alkyliert werden.

Einige der verwendeten Amine sind kommerziell erhältlich oder können durch literaturbekannte Verfahren hergestellt werden.

Andere der verwendeten primären Amine wurden hergestellt wie im folgenden Schema beispielhaft skizziert ist.

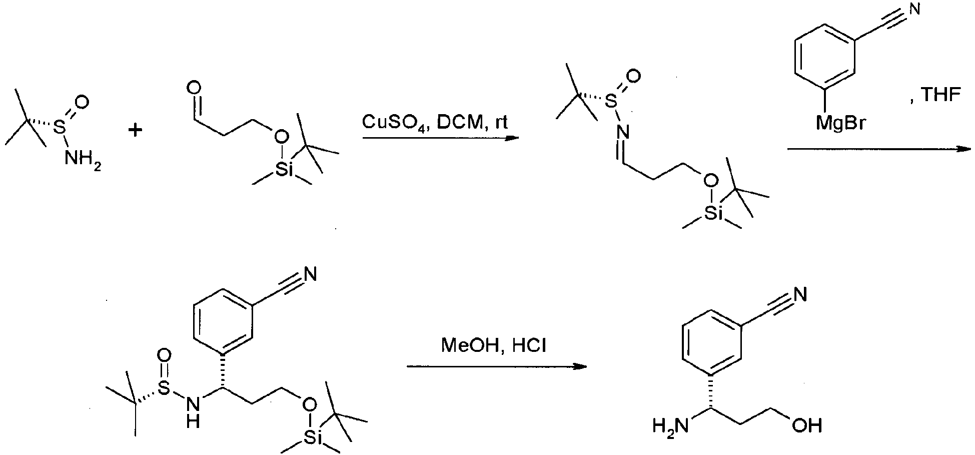

Die nachfolgend aufgeführten Beispiele dienen zur Erläuterung der Erfindung, ohne diese jedoch einzuschränken. Tabelle 1:

| Beispiel | CHEMISTRY | Methode | Retentionsz eit | Molgewicht (g/mol) | Name |
|---|---|---|---|---|---|
| 1 | 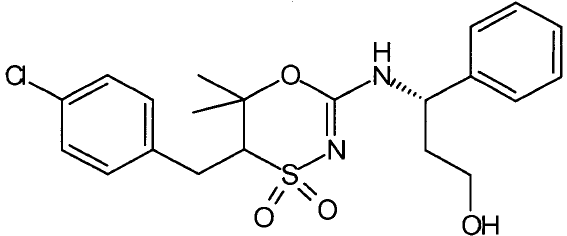 | A | 1.046 | 437,0 | (S)-3-[5-(4-Chlor-benzyl)-6,6-d imethyl-4,4-dioxo-5,6-dihydro-4H-4lambda6-[1,4,3]oxathiazin-2-ylamino]-3-phenyl-propan-1-ol |
| 2 | 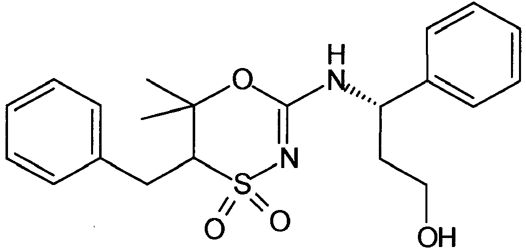 | A | 0.992 | 402,5 | (S)-3-(5-Benzyl-6,6-dimethyl-4,4-dioxo-5,6-dihydro-4H-4lambda6-[1,4,3]oxathiazin-2-ylamino)-3-phenyl-propan-1-ol |
| 3 | 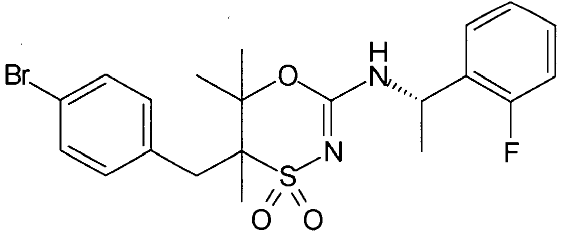 | A | 1.174 | 483,4 | [5-(4-Brom-benzyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin |
| 4 | 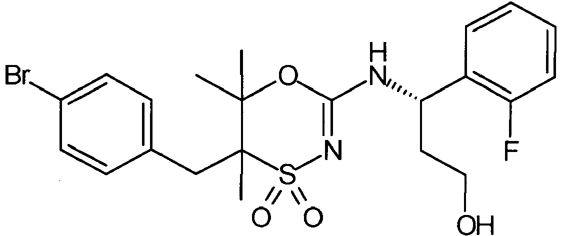 | A | 1.083 | 513,4 | (S)-3-[5-(4-Brom-benzyl)-5,6,6-trimethyl-4,4-dioxo 5,6-dihydro-4H-4lambda6-[1,4,3]oxathiazin-2-ylamino]-3-(2-fluor-phenyl)-propan-1-ol |
| 5 | 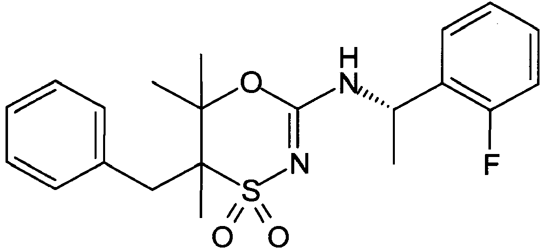 | A | 1.115 | 404,5 | (5-Benzyl-5,6,6-trimethyl-4,4-dioxo-5,6-dihydro-4H-4lambda6-[1,4,3]oxathiazin-2-yl)-[(S)-1-(2-fluor-phenyl)-ethyl]-amin |
| 6 | 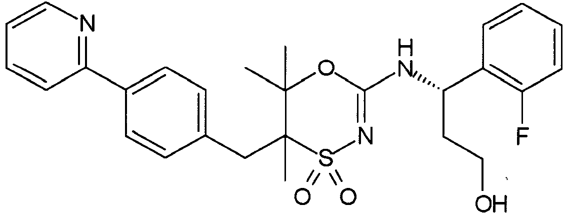 | A | 0.827 | 511,6 | (S)-3-(2-Fluor-phenyl)-3-[5,6,6-trimethyl-4,4-dioxo 5-(4-pyridin-2-yl-benzyl)-5,6-dihydro-4H-4lambda6 [1,4,3]oxathiazin-2-ylamino]-propan-1-ol |
| 7 | 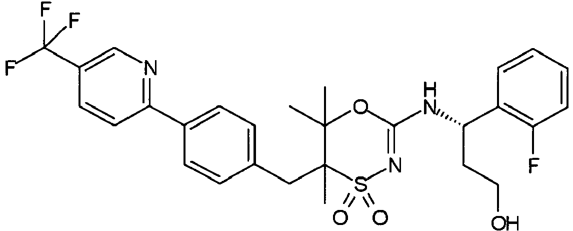 | A | 1.135 | 579,6 | (S)-3-(2-Fluor-phenyl)-3-{5,6,6-trimethyl-4,4-dioxo-5-[4-(5-trifluormethyl-pyridin-2-yl)-benzyl]-5,6-dihydro-4H-4lambda6-[1,4,3]oxathiazin-2-ylamino}-propan-1-ol |
| 8 | 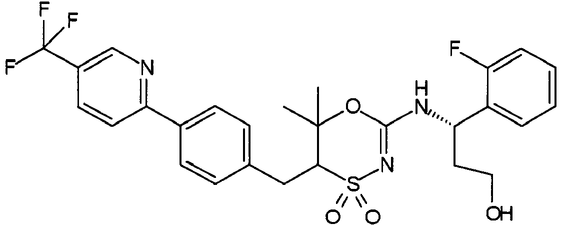 | A | 1.115 | 565,6 | (S)-3-{6,6-Dimethyl-4,4-dioxo-5-[4-(5-trifluormethyl-pyridin-2-yl)-benzyl]-5,6-dihydro-4H-4lambda6-[1,4,3]oxathiazin-2-ylamino}-3-(2-fluor-phenyl)-propan-1-ol |
| 9 | 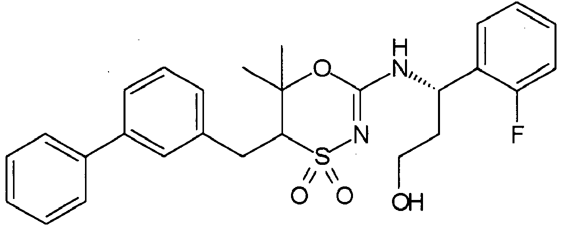 | A | 1,09 | 496,6 | (S)-3-(5-Biphenyl-3-ylmethyl-6,6-dimethyl-4,4-dioxo-5,6-dihydro-4H-4lambda6-[1,4,3]oxathiazin-2-ylamino)-3-(2-fluoro-phenyl)-propan-1-ol |
| 10 | 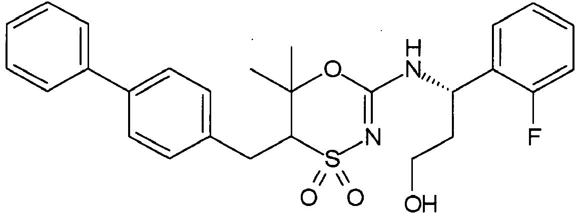 | A | 1,114 | 496,6 | (S)-3-(5-Biphenyl-4-ylmethyl-6,6-dimethyl-4,4-dioxo-5,6-dihydro-4H-4lambda6-[1,4,3]oxathiazin-2-ylamino)-3-(2-fluoro-phenyl)-propan-1-ol |
| 11 | 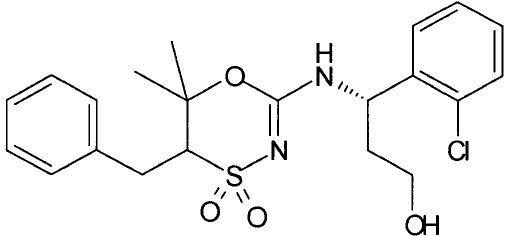 | A | 1,023 | 437,0 | (S)-3-(5-Benzyl-6,6-dimethyl-4,4-dioxo-5,6-dihydro-4H-4lambda6-[1,4,3]oxathiazin-2-ylamino)-3-(2-chlor-phenyl)-propan-1-ol |
| 12 | 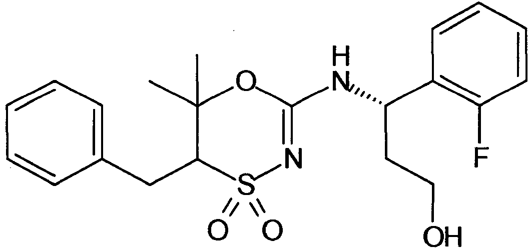 | A | 0,998 | 420,5 | (S)-3-(5-Benzyl-6,6-dimethyl-4,4-dioxo-5,6-dihydro-4H-4lambda6-[1,4,3]oxathiazin-2-ylamino)-3-(2-fluor-phenyl)-propan-1-ol |
| 13 | 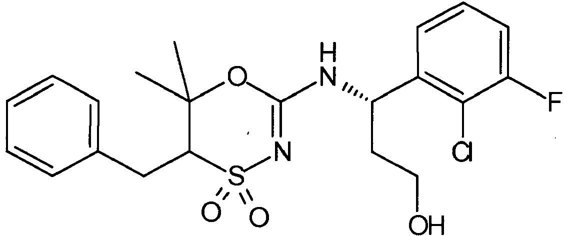 | A | 1,032 | 454,9 | (S)-3-(5-Benzyl-6,6-dimethyl-4,4-dioxo-5,6-dihydro-4H-4lambda6-[1,4,3]oxathiazin-2-ylamino)-3-(2-chlor-3-fluor-phenyl)-propan-1-ol |
| 14 | 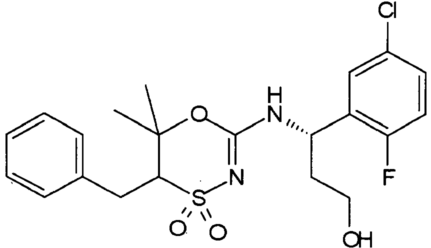 | A | 1,039 | 454,9 | (S)-3-(5-Benzyl-6,6-dimethyl-4,4-dioxo-5,6-dihydro-4H-4lambda6-[1,4,3]oxathiazin-2-ylamino)-3-(5-chlor-2-fluor-phenyl)-propan-1-ol |
| 15 | 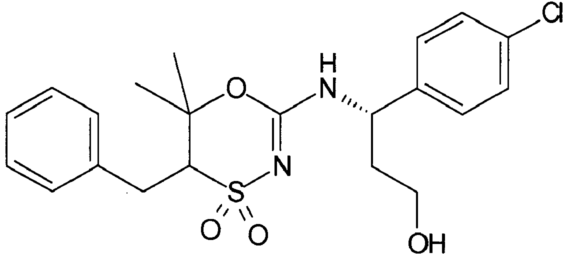 | A | 1,042 | 437,0 | (S)-3-(5-Benzyl-6,6-dimethyl-4,4-dioxo-5,6-dihydro-4H-4lambda6-[1,4,3]oxathiazin-2-ylamino)-3-(4-chlor-phenyl)-propan-1-ol |
| 16 | 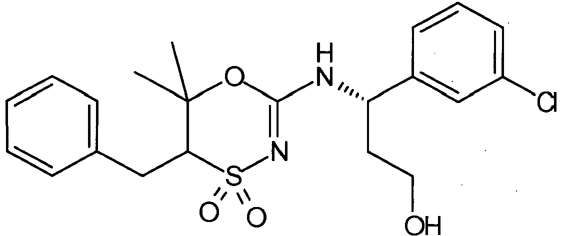 | A | 1,034 | 437,0 | (S)-3-(5-Benzyl-6,6-dimethyl-4,4-dioxo-5,6-dihydro-4H-4lambda6-[1,4,3]oxathiazin-2-ylamino)-3-(3-chlor-phenyl)-propan-1-ol |
| 17 | 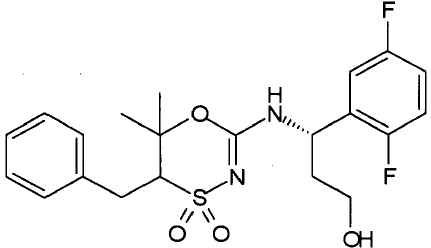 | A | 0,989 | 438,5 | (S)-3-(5-Benzyl-6,6-dimethyl-4,4-dioxo-5,6-dihydro-4H-4lambda6-[1,4,3]oxathiazin-2-ylamino)-3-(2,5-difluor-phenyl)-propan-1-ol |
| 18 | 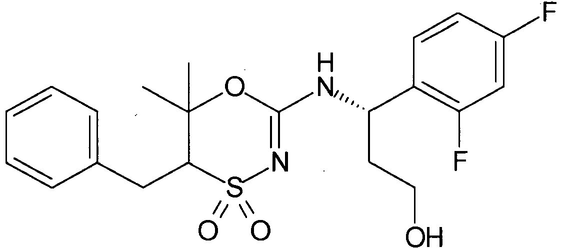 | A | 0,998 | 438,5 | (S)-3-(5-Benzyl-6,6-dimethyl-4,4-dioxo-5,6-dihydro-4H-4lambda6-[1,4,3]oxathiazin-2-ylamino)-3-(2,4-difluor-phenyl)-propan-1-ol |
| 19 | 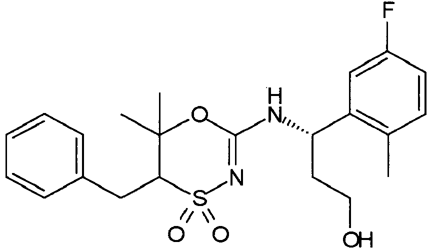 | A | 1,007 | 434,5 | (S)-3-(5-Benzyl-6,6-dimethyl-4,4-dioxo-5,6-dihydro-4H-4lambda6-[1,4,3]oxathiazin-2-ylamino)-3-(5-fluor-2-methyl-phenyl)-propan-1-ol |
| 20 | 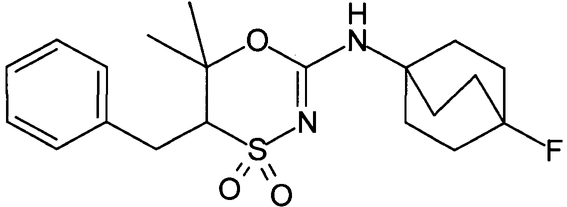 | A | 1,075 | 394,5 | (5-Benzyl-6,6-dimethyl-4,4-d ioxo-5,6-dihydro-4H-4lambda6-[1,4,3]oxathiazin-2-yl)-(4-fluor-bicyclo[2.2.2]oct-1-yl)-amin |
| 21 | 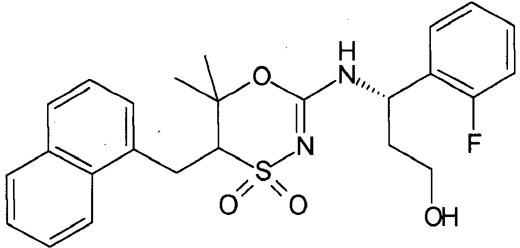 | A | 1,052 | 470,5 | (S)-3-(6,6-Dimethyl-5-naphthalen-1-ylmethyl-4,4-dioxo-5,6-dihydro-4H-4lambda6-[1,4,3]oxathiazin-2-ylamino)-3-(2-fluor-phenyl)-propan-1-ol |
| 22 | 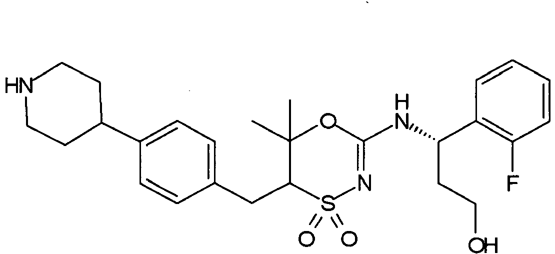 | A | 0.798 | 503.6 | (S)-3-[6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol |
| 23 | 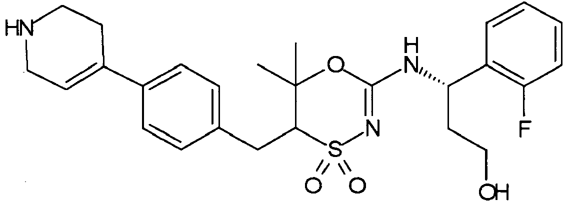 | A | 0.798 | 501.6 | (S)-3-{6,6-Dimethyl-4,4-dioxo-5-[4-(1,2,3,6-tetrahydro-pyridin-4-yl)-benzyl]-5,6-dihydro-4H-4lambda6-[1,4,3]oxathiazin-2-ylamino}-3-(2-fluor-phenyl)-propan-1-ol |
| 24 | 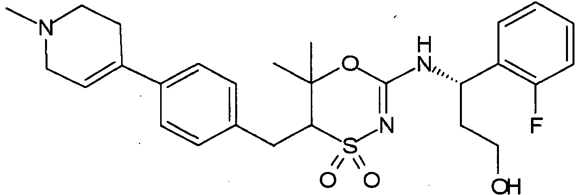 | A | 0.812 | 515.6 | (S)-3-{6,6-Dimethyl-5-[4-(1-methyl-1,2,3,6-tetrahydro-pyridin-4-yl)-benzyl]-4,4-dioxo-5,6-dihydro-4H-4lambda6-[1,4,3]oxathiazin-2-ylamino}-3-(2-fluor-phenyl)-propan-1-ol |
| 25 | 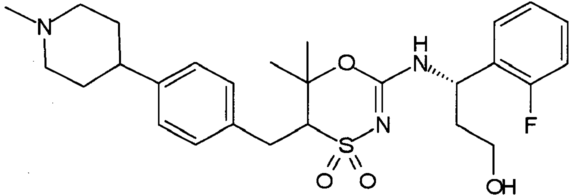 | A | 0.812 | 517.7 | (S)-3-{6,6-Dimethyl-5-[4-(1-methyl-pi peridin-4-yl)-benzyl]-4,4-dioxo-5,6-dihydro-4H-4lambda6-[1,4,3]oxathiazin-2-ylamino}-3-(2-fluor-phenyl)-propan-1-ol |
| 26 | 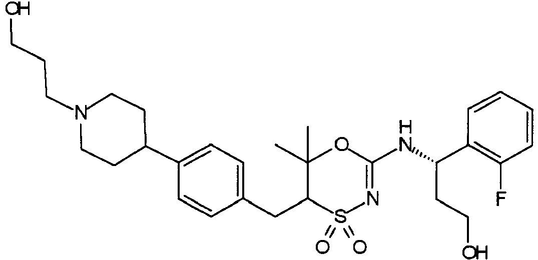 | A | 0.811 | 561.7 | (S)-3-(2-fluor-phenyl)-3-(5-{4-[1-(3-hydroxy-propyl)-piperidin-4-yl]-benzyl}-6,6-dimethyl-4,4-dioxo-5,6-dihydro-4H-4lambda6-[1,4,3]oxathiazin-2-ylamino)-propan-1-ol |
| 27 | 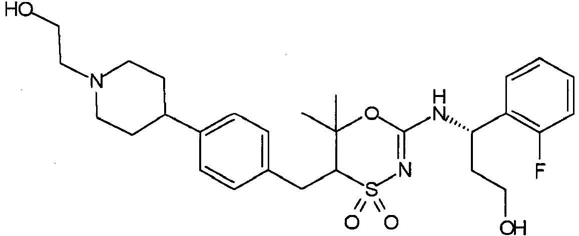 | A | 0.808 | 547.7 | (S)-3-(2-fluor-phenyl)-3-(5-{4-[1-(2-hydroxy-ethyl)-piperidin-4-yl]-benzyl}-6,6-dimethyl-4,4-dioxo-5,6-dihydro-4H-4lambda6-[1,4,3]oxathiazin-2-ylamino)-propan-1-ol |
| 28 | 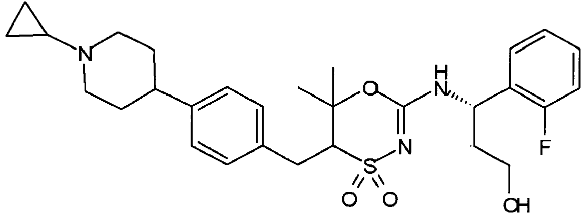 | A | 0.832 | 543.7 | (S)-3-{5-[4-(1-Cyclopropyl-piperidin-4-yl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol |
| 29 | 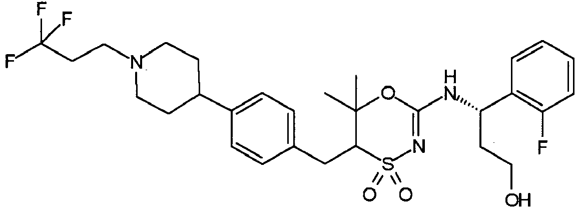 | A | 0.863 | 599.7 | (S)-3-(6,6-Dimethyl-4,4-dioxo-5-{4-[1-(3,3,3-trifluor-propyl)-piperidin-4-yl]-benzyl}-5,6-dihydro-4H-4lambda6-[1,4,3]oxathiazin-2-ylamino)-3-(2-fluor-phenyl)-propan-1-ol |
| 30 | 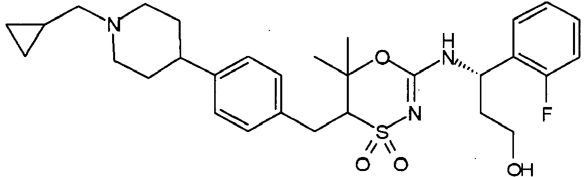 | A | 0.844 | 557.7 | (S)-3-{5-[4-(1-Cyclopropylmethyl-piperidin-4-yl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol |
| 31 | 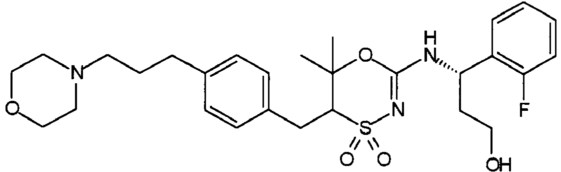 | A | 0.823 | 547.7 | (S)-3-{6,6-Dimethyl-5-[4-(3-morpholin-4-yl-propyl)-benzyl]-4,4-dioxo-5,6-dihydro-4H-4lambda6-[1,4,3]oxathiazin-2-ylamino}-3-(2-fluor-phenyl)-propan-1-ol |
| 32 | 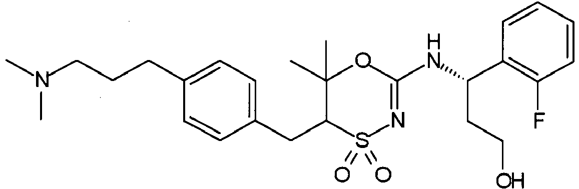 | A | 0.825 | 505.6 | (S)-3-{5-[4-(3-Dimethylamino-propyl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol |
| 33 | 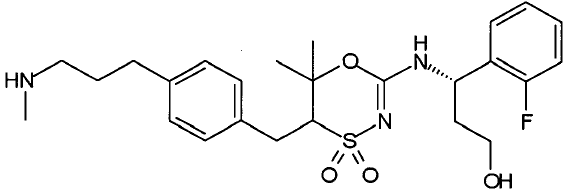 | A | 0.818 | 491.6 | (S)-3-{6,6-Dimethyl-5-[4-(3-methylamino-propyl)-benzyl]-4,4-dioxo-5,6-dihydro-4H-4lambda6-[1,4,3]oxathiazin-2-ylamino}-3-(2-fluor-phenyl)-propan-1-ol |
| 34 | 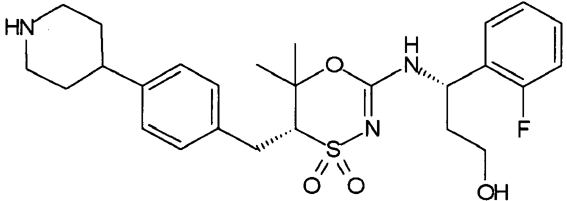 | A | 0.802 | 503.6 | (S)-3-[(R)-6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol * |
| 35 | 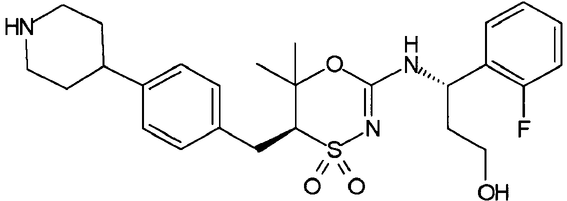 | A | 0.797 | 503.6 | (S)-3-[(S)-6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol * |
| 36 | 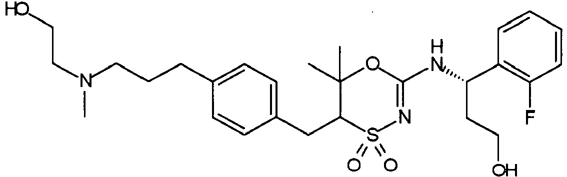 | A | 0.798 | 535.7 | (S)-3-(2-fluor-phenyl)-3-[5-(4-{3-[(2-hydroxy-ethyl)-methyl-amino]-propyl}-benzyl)-6,6-dimethyl-4,4-dioxo-5,6-dihydro-4H-4lambda6-[1,4,3]oxathiazin-2-ylamino]-propan-1-ol |
| 37 | 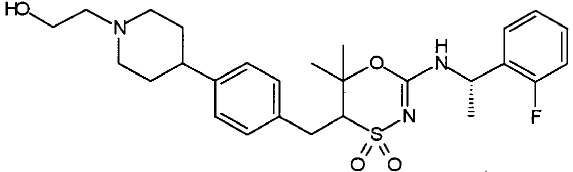 | A | 0.875 | 517.7 | 2-[4-(4-{2-[(S)-1-(2-fluor-phenyl)-ethylamino]-6,6-dimethyl-4,4-dioxo-5,6-dihyd ro-4H-4lambda6-[1,4,3]oxathiazin-5-ylmethyl}-phenyl)-piperidin-1-yl]-ethanol |
| 38 | 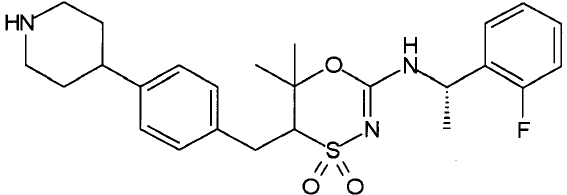 | A | 0.856 | 473.6 | [6,6-Dimethyl-4,4-dioxo-5-(4-piperidin-4-yl-benzyl)-5,6-dihydro-4H-4lambda6-[1,4,3]oxathiazin-2-yl]-[(S)-1-(2-fluor-phenyl)-ethyl]-amin |
| 39 | 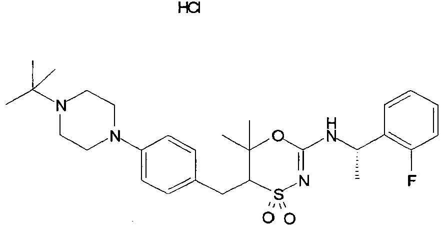 | A | 0.903 | 530.7 | {5-[4-(4-tert-Butyl-piperazin-1-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |
| 40 | 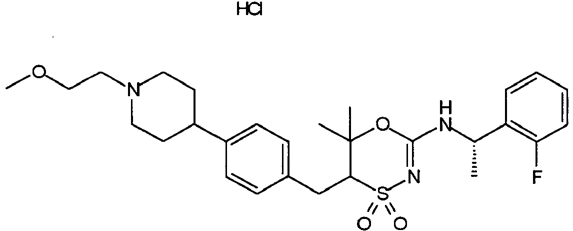 | A | 0.889 | 531.7 | [(S)-1-(2-fluor-phenyl)-ethyl]-(5-{4-[1-(2-methoxy-ethyl)-piperidin-4-yl]-benzyl}-6,6-d imethyl-4,4-dioxo-5,6-dihydro-4H-4lambda6-[1,4,3]oxathiazin-2-yl)-amin |
| 41 | 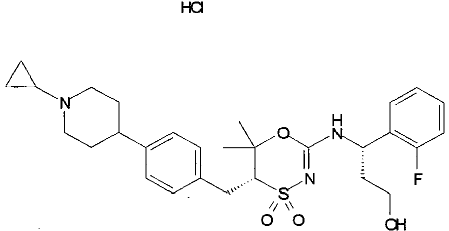 | A | 0.828 | 543.7 | (S)-3-{(R)-5-[4-(1-Cyclopropyl-piperidin-4-yl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol * |
| 42 | 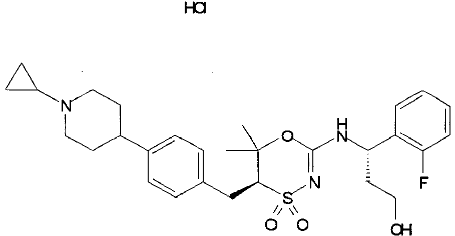 | A | 0.829 | 543.7 | (S)-3-{(S)-5-[4-(1-Cyclopropyl-piperidin-4-yl)-benzyl]-6,6-dimethyl-4,4-dioxo-5,6-dihydro-4H-4lambda6-[1,4,3]oxaihiazin-2-ylamino}-3-(2-fluor-phenyl)-propan-1-ol * |
| 43 | 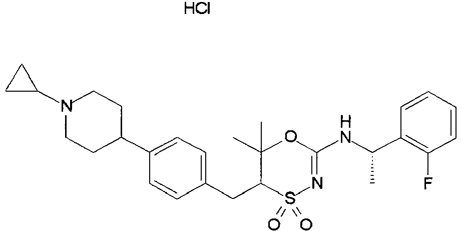 | A | 0.894 | 513.7 | {5-[4-(1-Cyclopropyl-piperidin-4-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |
| 44 | 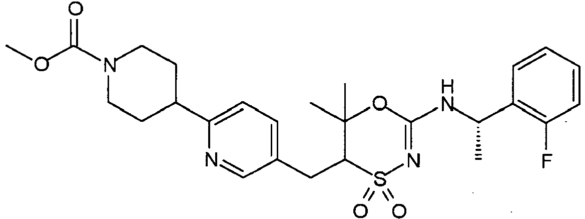 | A | 0.86 | 532.6 | 5-{2-[(S)-1-(2-fluor-phenyl)-ethylamino]-6,6-dimethyl-4,4-dioxo-5,6-dihydro-4H-4lambda6-[1,4,3]oxathiazin-5-ylmethyl}-3',4',5',6'-tetrahydro-2'H-[2,4']bipyridinyl-1'-carbonsäuremethylester |
| 45 | 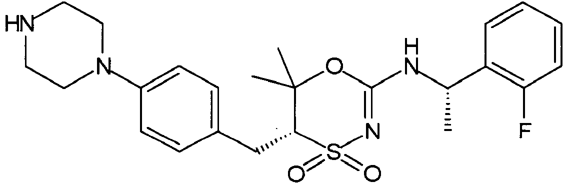 | A | 0.865 | 474.6 | [(R)-6,6-Dimethyl-4,4-dioxo-5-(4-piperazin-1-yl-benzyl)-5,6-dihydro-4H-4lambda6-[1,4,3]oxathiazin-2-yl]-[(S)-1-(2-fluor-phenyl)-ethyl] amin * |
| 46 | 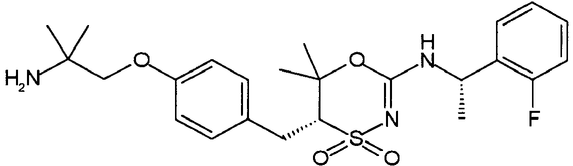 | A | 0.893 | 477.6 | {(R)-5-[4-(2-Amino-2-methyl-propoxy)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin * |
| 47 | 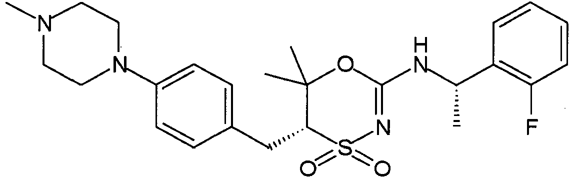 | A | 0.862 | 488.6 | {(R)-6,6-Dimethyl-5-[4-(4-methyl-piperazin-1-yl)-benzyl]-4,4-dioxo-5,6-dihydro-4H-4lambda6-[1,4,3]oxathiazin-2-yl}-[(S)-1-(2-fluor-phenyl)-ethyl]-amin * |
| 48 | 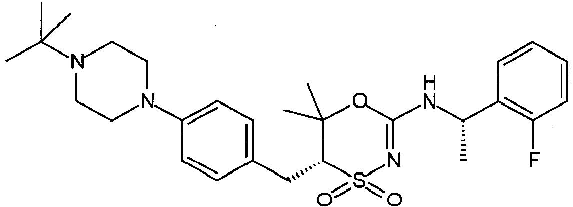 | A | 0.905 | 530.7 | {(R)-5-[4-(4-tert-Butyl-piperazin-1-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin * |
| 49 | 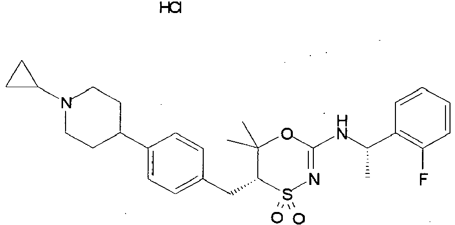 | A | 0.911 | 513.7 | {(R)-5-[4-(1-Cyclopropyl-piperidin-4-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin* |
| 50 | 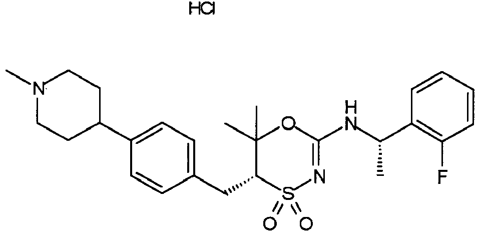 | B | 6.2 | 487.6 | {(R)-6,6-Dimethyl-5-[4-(1-methyl-piperidin-4-yl)-benzyl]-4,4-dioxo-5,6-dihydro-4H-4lambda6-[1,4,3]oxathiazin-2-yl}-[(S)-1-(2-fluor-phenyl)-ethyl]-amin * |
| 51 | 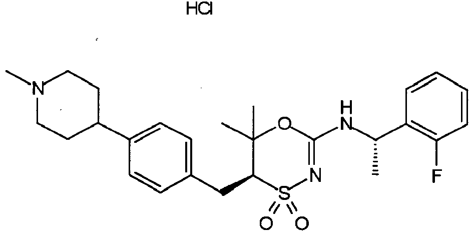 | B | 8.909 | 487.6 | {(S)-6,6-Dimethyl-5-[4-(1-methyl-piperidin-4-yl)-benzyl]-4,4-dioxo-5,6-dihydro-4H-4lambda6-[1,4,3]oxathiazin-2-yl}-[(S)-1-(2-fluor-phenyl)-ethyl]-amin * |
| 52 | 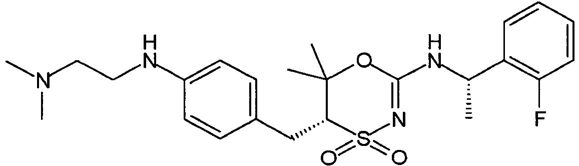 | A | 0.874 | 476.6 | N-(4-{(R)-2-[(S)-1-(2-fluor-phenyl)-ethylamino]-6,6-dimethyl-4,4-dioxo-5,6-dihydro-4H-4lambda6-[1,4,3]oxathiazin-5-ylmethyl}-phenyl)-N',N'-dimethyl-ethan-1,2-diamin * |
| 53 | 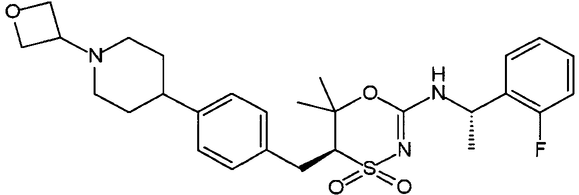 | A | 0.886 | 529.7 | {(S)-6,6-Dimethyl-5-[4-(1-oxetan-3-yl-piperidin-4-yl)-benzyl]-4,4-dioxo-5,6-dihydro-4H-4lambda6-[1,4,3]oxathiazin-2-yl}-[(S)-1-(2-fluor-phenyl)-ethyl]-amin * |
| 54 | 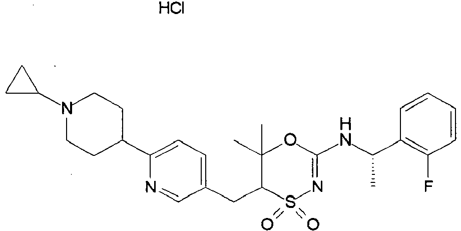 | A | 0.81 | 514.7 | [5-(1'-Cyclopropyl-1',2',3',4',5',6'-hexahydro-[2,4']bipyridinyl-5-ylmethyl-6,6-dimethyl-4,4-dioxo-5,6-dihydro-4H-4lambda6-[1,4,3]oxathiazin-2-yl]-[(S)-1-(2-fluor-phenyl)-ethyl]-amin |
| 55 | 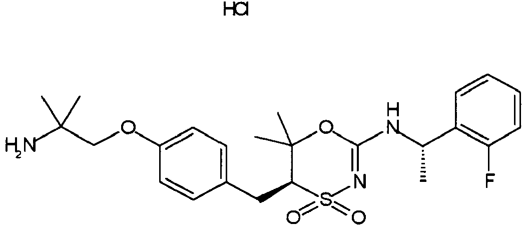 | A | 0.881 | 477.6 | {(S)-5-[4-(2-Amino-2-methyl-propoxy)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin * |
| 56 | 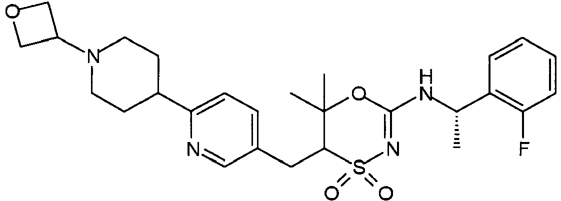 | A | 0.747 | 530.7 | [6,6-Dimethyl-5-(1'-oxetan-3-yl-1',2',3',4',5',6'-hexahydro-[2,4']bipyridinyl-5-ylmethyl)-4,4-dioxo-5,6-dihydro-4H-4lambda6-[1,4,3]oxathiazin-2-yl]-[(S)-1-(2-fluor-phenyl)-ethyl]-amin |
| 57 | 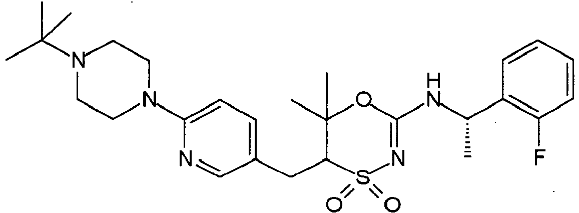 | A | 0.784 | 531.7 | {5-[6-(4-tert-Butyl-piperazin-1-yl)-pyridin-3-ylmethyl]-6,6-dimethyl-4,4-dioxo-5,6-dihydro-4H-4lambda6-[1,4,3]oxathiazin-2-yl}-[(S)-1-(2-fluor-phenyl)-ethyl]-amin |

| | | | | | |
|---|---|---|---|---|---|
| * Isomerenreine Verbindung ** trans Verbindung | | | | | |

### Chromatographie-Methoden:

### Methode A

Säule: YMC J'spere ODS H80, 80 Ǻ, S-4 µm, 20x2,1 mm

Laufmittel: 0 min 90 % H₂O (0,05 % TFA) - 1,9 min 95 % Acetonitril - 2,4 min 95 % Acetonitril - 2,45 min 10% Acetonitril (30 °C, Fluß 1 ml / min)

Die Wirksamkeit der Verbindungen wurde wie folgt getestet:

### Enzymatischer 11beta-HSD1 Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0,2 µg Protein), zubereitet in 50 mM HEPES, 0,1% BSA (w/v), in eine Platte mit 384 Vertiefungen gegeben. Die Testverbindungen (0,09 µl) wurden in 100% DMSO auf die Assayplatte übertragen. Die Reaktion wurde durch Zugabe von 20 µl [1,2-³H] Cortison (0,1 µCi/100 mM) in Assaypuffer mit 25 mM HEPES, 100 mM KCl, 5 mM NaCl, 2 mM MgCl₂ und 0,25 mM NADPH gestartet. Die Platte wurde 1 Stunde lang bei 37°C geschüttelt. Gleichzeitig wurde eine Stopplösung mit 20 mg/ml SPA-PVT-Perlen, 1,6 mg/ml monoklonalem Cortisol-Antikörper und 0,01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 min bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit/Vertiefung). Die Vergleichsverbindung war Glycyrrhetinsäure.

Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität**

| Beispiel | IC₅₀ (nM) |
|---|---|
| 1 | 5 |
| 2 | 7 |
| 3 | 4 |
| 4 | 3 |
| 5 | 5 |
| 6 | 6 |
| 7 | 12 |
| 8 | 7 |
| 9 | 5 |
| 10 | 4 |
| 11 | 8 |
| 12 | 4 |
| 13 | 6 |
| 14 | 4 |
| 15 | 10 |
| 16 | 6 |
| 17 | 4 |
| 18 | 5 |
| 19 | 4 |
| 20 | 5 |
| 21 | 5 |
| 22 | 7 |
| 23 | 17 |
| 25 | 20 |
| 26 | 16 |
| 27 | 5 |
| 28 | 9 |
| 29 | 7 |
| 30 | 29 |
| 31 | 17 |
| 32 | 32 |
| 33 | 30 |
| 34 | 8 |
| 35 | 11 |
| 36 | 38 |
| 37 | 37 |
| 38 | 7 |
| 39 | 17 |
| 40 | 25 |
| 41 | 32 |
| 42 | 5 |
| 43 | 11 |
| 44 | 5 |
| 45 | 44 |
| 50 | 52 |
| 51 | 6 |
| 52 | 62 |
| 53 | 9 |
| 55 | 8 |
| 56 | 24 |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen, und anderen Erkrankungen geeignet sind.

Nachfolgend wird die Herstellung einiger Beispiele detailliert beschrieben, die übrigen Verbindungen der Formel I wurden analog erhalten:Experimenteller Teil:

Trennung von 5-(4-Brom-benzyl)-2-methoxy-6,6-dimethyl-5,6-dihydro-[1,4,3]oxathiazin 4,4-dioxid in die beiden chiralen Verbindungen erfolgte mittels Hochdruck-Flüssig-Chromatographie:

Säule: Chiralcel OJ/H58; Flußrate 1ml/min; Ethanol : Methanol 1:1. Die Säule wurde mit Diethylamin vorkonditioniert. Die Retentionszeiten betrugen 5,05 Minuten und 6,16 Minuten.

### 2-Methyl-propan-2-sulfinsäure 1-(2-chlor-3-fluor-phenyl)-meth-(E)-ylideneamid

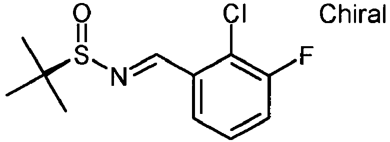

2-Chlor-3-fluor-benzaldehyd (2.5 g) und (R)-(+)-2-Methyl-2-propansulfinamid wurden in Dichlormethan (50 ml) gelöst und dann Titan(IV)-isopropoxid (23.6 ml) zugegeben. Es wurde drei Stunden unter Rückfluß erhitzt, der Ansatz dann auf Eis (150 g) gegossen und zehn Minuten kräftig gerührt. Es wurde über Kieselgur filtiert und das Filtrat mit Dichlormethan (3 x 50 ml) extrahiert. Die organische Phase wurden getrocknet (Na₂SO₄), filtriert und im Vakuum konzentriert. Das Produkt (4 g) wurde ohne weitere Reinigung weiter verwendet.

Auf gleiche Weise wurden folgende Verbindungen hergestellt:
2-Methyl-propan-2-sulfinsäure 1-(2,4-difluor-phenyl)-meth-(E)-ylideneamid 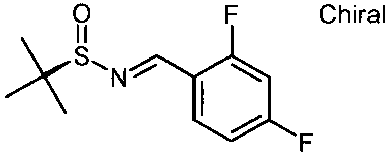
3-Amino-3-(2,5 -difluor-phenyl)-propionsäure

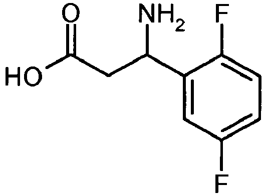

Eine Lösung aus 2,5-Difluor-benzaldehyd (5 g), Malonsäure (3.66 g) und Ammoniumacetat (5.42 g) in Ethanol (50 ml) wurde sechs Stunden unter Rückfluss erhitzt. Der Ansatz wurde abgekühlt und über Nacht stehen gelassen. Die entstandenen Kristalle wurden abfiltriert und mit Ethanol (5 ml) nachgewaschen. Das Produkt (2.94 g) wurde ohne weitere Reinigung verwendet.

Auf gleiche Weise wurden folgende Verbindungen hergestellt:
3-Amino-3-(5-fluor-2-methyl-phenyl)-propionsäure 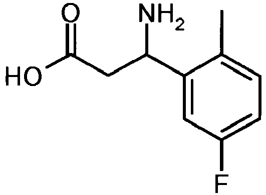
(S)-3-(2-Chlor-3-fluor-phenyl)-3-((R)-2-methyl-propan-2-sulfinylamino)-propionsäure methylester

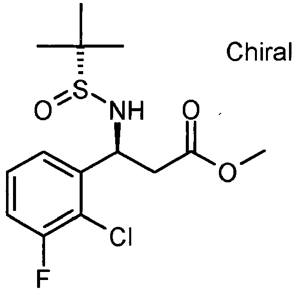

Zu Diisopropylamin (4.13 ml), gelöst in THF (200 ml), wurde langsam Butyllithium 1.6 N (17.55 ml) bei 0 °C zugetropft und dann dreißig Minuten gerührt. Anschließend wurde auf -75 °C abgekühlt und Methylacetat (2.13 ml) gelöst in THF (5 ml) zugegeben und weitere dreißig Minuten gerührt. Nun wurde Chlortitan-trüsopropoxid (56.15 ml, 1 molar in Hexan) bei gleicher Temperatur zugetropft und wieder dreißig Minuten gerührt. 2-Methyl-propan-2-sulfinsäure 1-(2-chlor-3-fluor-phenyl)-meth-(E)-ylideneamid wurde in THF (10 ml) gelöst und bei -75 °C zugetropft und drei Stunden bei gleicher Temperatur gerührt. Die Mischung wurde auf kalte, gesättigte Ammoniumchloridlösung gegossen und mit Essigsäureethylester versetzt und fünfzehn Minuten gerührt. Das Phasengemisch wurde dann über Kieselgur geklärt, die organische Phase abgetrennt und die wässrige Phase mit Ethylacetat (2 x 50 ml) extrahiert. Die vereinigten organischen Phasen wurden getrocknet (Na₂SO₄), filtriert und konzentriert. Der Rückstand wurde chromatographisch (Kieselgel 40-63 µm, Ethylacetat) gereinigt. Man erhielt so das Produkt (4.1 g) mit einem de von 72 % laut NMR

Auf gleiche Weise wurden folgende Verbindungen hergestellt:
(S)-3-(2,4-difluor-phenyl)-3-((R)-2-methyl-propan-2-sulfinylamino)-propionsäure methylester 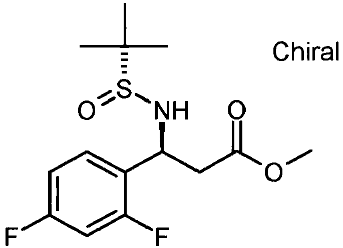 3-Amino-3-(2,5-difluor-phenyl)-propionsäure ethylester

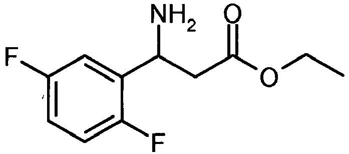

In einen Rundkolben mit Ethanol (50 ml) wurde Acetylchlorid (4 ml) bei Raumtemperatur getropft. Zu der so bereiteten ethanolischen Salzsäure Lösung wurde 3-Amino-3-(2,5-difluor-phenyl)-propionsäure (2.94 g) gegeben und drei Stunden bei 50 °C gerührt. Anschließend wurde im Vakuum konzentriert, der Rückstand mit Natronlauge 1N (100 ml) versetzt und sofort mit Dichlormethan (3 x 50 ml) extrahiert. Die vereinigten organischen Phasen wurden getrocknet (Na₂SO₄), filtriert und konzentriert. Das Produkt (3.2 g) wurde ohne weitere Reinigung verwendet.

Auf gleiche Weise wurden folgende Verbindungen hergestellt:
(S)-3-Amino-3-(5-fluor-2-methyl-phenyl)-propionsäure ethylester 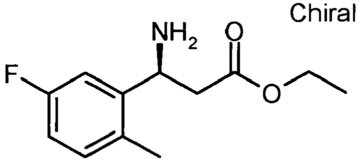
(S)-3-Amino-3-(2,5-difluor-phenyl)-propionsäure methylester 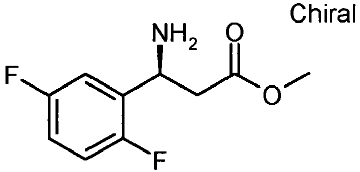 (S)-3-Amino-3-(5-fluor-2-methyl-phenyl)-propionsäure methylester 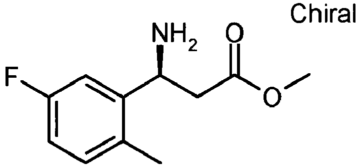
(S)-3-Amino-3-(2,5-difluor-phenyl)-propionsäure

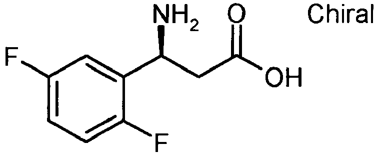

3-Amino-3-(2,5-difluor-phenyl)-propionsäure ethylester (3 g) wurden in Wasser emulgiert (pH = 9.2) und mit Kailumdihydrogenphosphat (5 mg) versetzt (pH = 8.5). Nach Zugabe von Amono Lipase PS (150 mg) wurde über Nacht gerührt. Der pH-Wert bleibt bei 7.5. Der Ansatz wurde nun mit Wasser (30 ml) versetzt und über Kieselgur filtriert und noch mehrmals mit Wasser nachgewaschen. Die wässrige Phase wurde nun mit Dichlormethan extrahiert (3 x 30 ml), die organische Phase getrocknet (Na2S04) und konzentriert. Man erhielt so das Produkt (1.3 g) mit einem ee = 90 % nach HPLC.

Auf gleiche Weise wurden folgende Verbindungen hergestellt:
(S)-3-Amino-3-(5-fluor-2-methyl-phenyl)-propionsäure 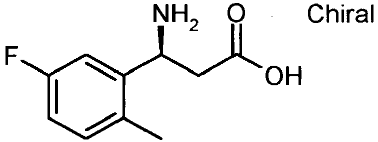
(S)-3-Amino-3-(3-chlor-2-fluor-phenyl)-propionsäure methylester

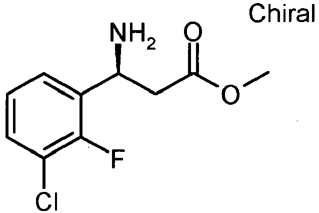

Zu einer Lösung aus Acetylchlorid (4 ml) in Methanol (50 ml) wurde (S)-3-(2-Chlor-3-fluor-phenyl)-3-((R)-2-methyl-propan-2-sulfinylamino)-propionsäure methylester (4.1 g) gelöst in Methanol (10 ml) bei Raumtemperatur zugegeben. Nach einer Stunde wurde die Mischung konzentriert, in Dichlormethan (100 ml) aufgenommen und mit Natronlauge 1N (100 ml) gewaschen. Die wässrige Phase wurde noch zweimal mit Dichlormethan gewaschen. Die vereinigten organischen Phasen wurden getrocknet (Na₂SO₄), filtriert und konzentriert. Der Rückstand (2.8 g) wurde ohne weitere Reinigung verwendet.

Auf gleiche Weise wurden folgende Verbindungen hergestellt:
(S)-3-Amino-3-(2,4-difluor-phenyl)-propionsäure methylester 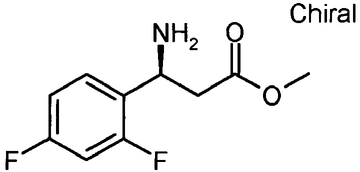
(S)-3-Amino-3-(2-chlor-3-fluor-phenyl)-propan-1-ol

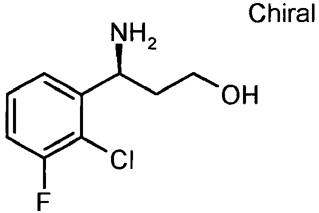

Zu einer Lösung aus Lithiumaluminiumhydrid 1N (24.2 ml in THF) in THF (20 ml) wurde bei 0 °C (S)-3-Amino-3-(3-chlor-2-fluor-phenyl)-propionsäure methylester (2.8 g) gelöst in THF (5 ml) zugetropft und eine Stunde gerührt. Dann wurde der Ansatz auf gesättigte Natriumchloridlösung (50 ml) getropft und fünf Minuten gerührt. Dann wurde Natronlauge 1N (50 ml) zugegeben und weitere fünf Minuten gerührt. Die Mischung wurde dann mit Ethylacatat (3 x 50 ml) extrahiert, getrocknet (Na₂SO₄) und konzentriert. Das Produkt (2.23 g) wurde ohne weitere Aufreinigung verwendet.

Auf gleiche Weise wurden folgende Verbindungen hergestellt:
(S)-3-Amino-3-(2,4-difluor-phenyl)-propan-1-ol 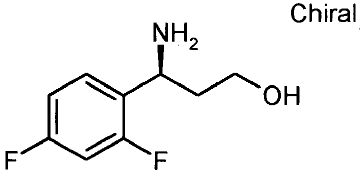
(S)-3-Amino-3-(2,5-difluor-phenyl)-propan-1-ol 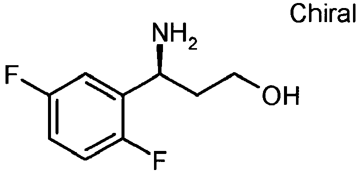
(S)-3-Amino-3-(5-fluor-2-methyl-phenyl)-propan-1-ol 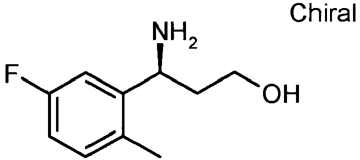
(S)-3-(tert-Butyl-dimethyl-silanyloxy)-1-(5-chlor-2-fluor-phenyl)-propylamin

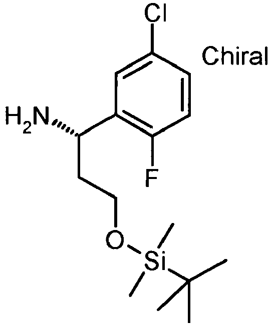

Das Lithiumaluminiumhydrid 1N (100 ml) in THF wurde vorgelegt und (S)-3-Amino-3-(5-chloro-2-fluoro-phenyl)-propionsäuremethylester (in THF unlöslich) portionsweise unter Eiskühlung zugegeben. Die Kühlung wurde dann entfernt und 1 Stunde nachgerührt. Anschließend wurde unter Eis 5ml H₂O und 5 ml 5M NaOH zugetropft und vorsichtig 15ml H₂O zugegeben, dann wurde 4 Tage gerührt. Der Niederschlag wurde über Celite filtriert, mit THF (3 x 30 ml) nachgewewaschen, dann das Filtrat eingeengt. Der Rückstand wurde mit Dichlormethan (50 ml) aufgenommen und über MgSO4 getrocknet, filtriert und einrotiert. Der Rückstand (2.95 g) wurde in Dichlormethan (20 ml) gelöst, mit DIPEA und portionsweise mit t-Butyldimethyl-chlorsilan versetzt. Es wurde bei 25 °C über Nacht gerührt.

Der Ansatz wurde zweimal mit 50 ml 5% NaHCO₃-Lsg. gewaschen, die organische Phase über Na₂SO₄ getrocknet und eingeengt. Auswaage: 5.16 g. Dann wurde mit Hilfe des Flashmasters gereinigt. 70 g Säule (Normalphase); Fraktionsgröße: 20ml; Fluss: 19ml/min Fraktionen 17 - 40 vereinigt. Man erhielt so das Produkt (3.64 g)mit dem Molekulargewicht 317.9 (C₁₅H₂₆ClFNOSi); MS (ESI): 318 (M+H+).

### (S)-3-(tert-Butyl-dimethyl-silanyloxy)-1-phenyl-propylamin

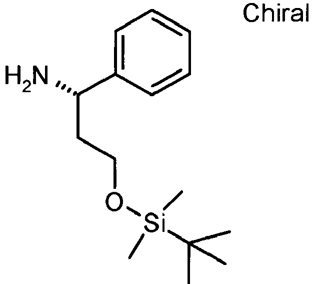

S-3-Amino-3-phenyl-1-propanol (4.72 g) wurde in Dichlormethan (60 mL) gelöst, mit Triethylamin (6.36 g) und tert.-Butyl-dimethyl-chlorosilan (4.1 g) versetzt und 3 Stunden bei Raumtemperatur gerührt. Dann wurde mit Wasser (3 x 50 mL) gewaschen und über eine Phasenseparatorkartusche getrocknet. Man erhielt so das Produkt mit dem Molekulargewicht 265.5 g / mol (C15H27NOSi), MS (ESI): (M+H+) 266 g / mol.

Auf gleiche Weise wurden folgende Verbindungen hergestellt:
(S)-3-(tert-Butyl-dimethyl-silanyloxy)-1-(2-fluor-phenyl)-propylamin 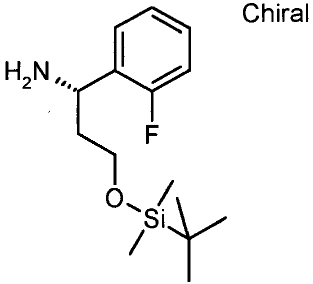
(S)-3-(tert-Butyl-dimethyl-silanyloxy)-1-(3-chlor-phenyl)-propylamin 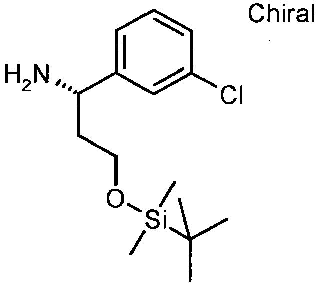
(S)-3 -(tert-Butyl-dimethyl-silanyloxy)-1-(4-chlor-phenyl)-propylamin 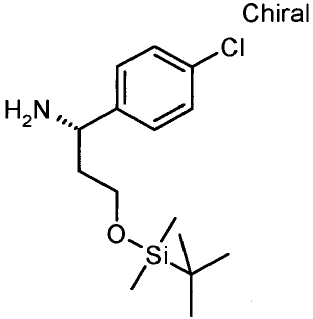
(S)-3-(tert-Butyl-dimethyl-silanyloxy)-1-(2-chlor-phenyl)-propylamin 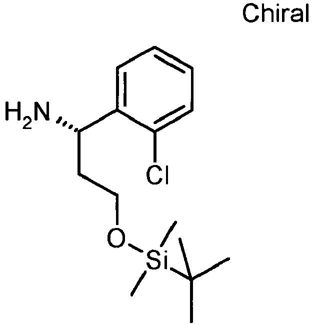
(S)-3-(tert-Butyl-dimethyl-silanyloxy)-1-(2-chlor-3-fluor--phenyl)-propylamin 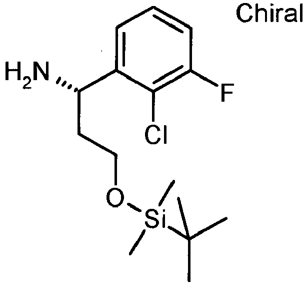
(S)-3-(tert-Butyl-dimethyl-silanyloxy)-1-(2,4-difluor-phenyl)-propylamin 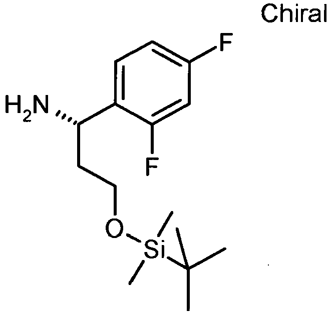
(S)-3-(tert-Butyl-dimethyl-silanyloxy)-1-(2, 5-difluor-phenyl)-propylamin 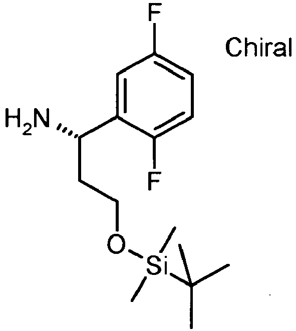
(S)-3-(tert-Butyl-dimethyl-silanyloxy)-1-(5-fluor-2-methyl-phenyl)-propylamin 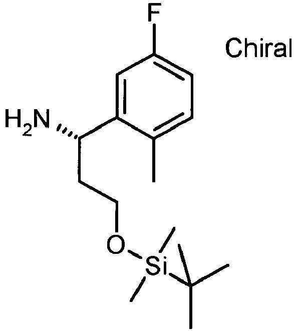
2-((4-Brom-benzyl)-2-methyl-malonsäurediethylester

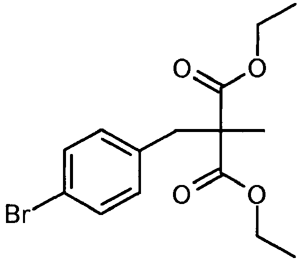

Natriumhydrid in Mineralöl 60 % (6.4 g) wurden in trockenem DMF (100 ml) suspendiert und anschließend bei 0 °C der Methylmalonsäurediethylester (25.2 g) langsam zugetropft. Nach beenderter Gasentwicklung wurde das 4-Brom-benzylbromid (43.7 g) in 40 ml DMF gelöst zugetropft. Es wurde dann eine Stunde bei 100 °C gerührt. Die entstandene Suspension wurde abgekühlt, mit Ethylacetat (100 ml) verdünnt und mit Na₂SO₃ Lösung 5 % (3 x 100 ml) gewaschen. Die organische Phase wurde getrocknet (Na₂SO₄) und im Vakuum konzentriert. Der klare Rückstand (49.6 g) wurde ohne weitere Aufarbeitung weiter verwendet.

### 2-(4-Brom-benzyl)-2-methyl-malonsäure

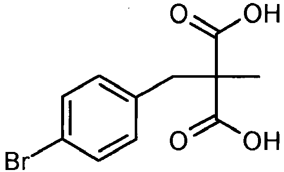

2-(4-Brom-benzyl)-2-methyl-malonsäurediethylester (49.5 g) wurde in Ethanol (100 ml) und Wasser (100 ml) gelöst und der Ansatz mit Natronlauge (28.8 g) versetzt. Die Lösung wurde dann über Nacht unter Rückfluß erhitzt. Anschließend wurde auf Wasser gegossen (200 ml) und mit Dichlormethan (2 x 100 ml) gewaschen. Der Ansatz wurde dann auf Salzsäure konz. (100 ml) und Eis (200 g) gegossen und mit Ethylacetat (2 x 150 ml) extrahiert. Die organische Phase wurde dann mit MgSO₄ getrocknet, filtriert und im Vakuum konzentriert. Der feste Rückstand (41.5 g) wurde ohne weitere Aufarbeitung weiter verwendet.

### 3-(4-Brom-phenyl)-2-methyl-propionsäureethylester

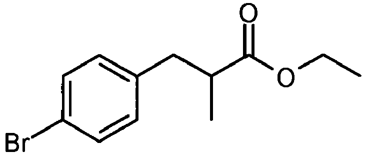

2-(4-Brom-benzyl)-2-methyl-malonsäure (41.5 g) wurde in einem Kolben 45 Minuten auf 150 °C erhitzt. Das Ölbad wurde anschließend entfernt, das klare Öl vorsichtig mit Ethanol (70 g) und Oleum (4 g) versetzt und es wurde vier Stunden unter Rückfluß erhitzt. Das Ethanol wurde im Vakuum entfernt, Der ölige Rückstand mit Ethylacetat (100 ml) versetzt und die organische Phase mit verdünnter Ammoniak-Lösung (2 x 100 ml) gewaschen, getrocknet (MgSO₄), filtriert und im Vakuum konzentriert. Das Produkt wurde durch Destillation bei 0.26 mbar bei 100 °C gereinigt.

Man erhielt so das Produkt (28 g) mit dem Molekulargewicht 271.1 g / mol (C₁₂H₁₅BrO₂), MS (ESI): (M+H+) 272 g / mol.

### 4-(4-Brom-phenyl)-2,3-dimethyl-butan-2-ol

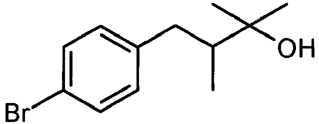

Methylmagnesiumbromid (25.2 g) in Diethylether (150 ml) gelöst wurde tropfenweise unter Kühlung mit 3-(4-Brom-phenyl)-2-methyl-propionsäureethylester (28 g) versetzt. Nach kompletter Zugabe wurde die Kühlung entfernt und der Ansatz über Nacht stehen gelassen. Die Lösung wurde dann mit Ammoniumchlorid-Lösung konz. (10 ml) und Wasser (5 ml) versetzt. Der Diethylether wurde am Rotationsverdampfer entfernt und der Rückstand mit THF (100 ml) versetzt und die Lösung mit MgSO₄ getrocknet. Das MgSO₄ wurde über Celite abfiltriert und die Lösung stark eingeengt und über Nacht stehen gelassen. Die entstandenen Kristalle (13.2 g) wurden abgesaugt und ohne weitere Aufarbeitung weiter verwendet.

### 1-Brom-4-(2,3-dimethyl-but-2-enyl)-benzen

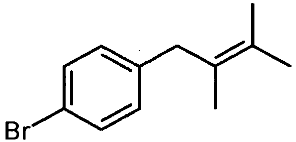

In 100 °C warme Polyphosphorsäure wurde protionweise 4-(4-Brom-phenyl)-2,3-dimethyl-butan-2-ol zugegeben und dann eine Stunde bei dieser Temperatur gerührt. Nach dem Abkühlen wurde mit Eis (50 g) versetzt und mit Ethylacetat (3 x 50 ml) extrahiert. Die organische Phase wurde mit NaHCO₃ Lösung (50 ml) gewaschen, getrocknet (MgSO₄) und konzentriert. Der Rückstand wurde zuerst chromatographisch mit Heptan gereinigt und dann destilliert (0.04 mbar, 58 °C).

Man erhielt so das Produkt (8.7 g). Die Charakterisierung erfolgte über NMR

### 1-Brom-4-(3-methyl-but-2-enyl)-benzen

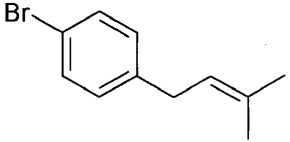

Zimmermann, H. et al. Journal of Organic Chemistry 49; 17; 1984; 3069-3083.

### 5-Benzyl-2-methoxy-6,6-dimethyl-5,6-dihydro-[1,4,3]oxathiazin 4,4-dioxid

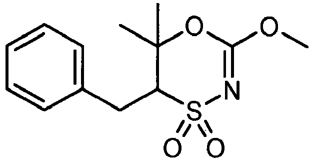

Chlorsulfonylisocyanat (4.2 g) wurden in Dichlormethan vorgelegt und unter Eiskühlung Methanol (1.2 ml) zugetropft. Anschließend wurde 10 Minuten gerührt und dann im Vakuum das Lösungsmittel entfernt. Nach Zugabe von THF (30 ml) wurde der Ansatz auf -78 °C abgekühlt und unter Argon mit Natriumhydrid versetzt (heftige Gasentwicklung). Die Kühlung wurde entfernt und (3-Methyl-but-2-enyl)-benzen (4.34 g) wurden in einer Protion zugegeben und dann der Ansatz auf 35 °C erwärmt. Nach 30 Minuten wurde das THF entfernt, der Rückstand mit Ethylacetat (50 ml) aufgenommen und mit Wasser (3 x 30 ml) gewaschen. Die organische Phase wurde getrocknet (Na₂SO₄) und konzentriert. Das Rohprodukt (7.07 g) wurde mit präparativer HPLC gereinigt.
Man erhielt so das Produkt (0.29 g) mit dem Molekulargewicht 283.3 g / mol (C₁₃H₁₇NO₄S), MS (ESI): (M+H+) 284 g / mol.

Auf gleiche Weise wurden folgende Verbindungen hergestellt:
5-((4-Brom-benzyl)-2-methoxy-5,6,6-trimethyl-5,6-dihydro-[1,4,3]oxathiazin 4,4-dioxid 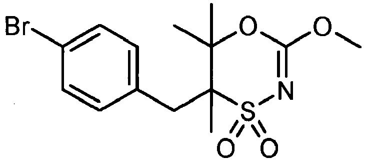
5-((4-Brom-benzyl)-2-methoxy-6,6-dimethyl-5,6-dihydro-[1,4,3]oxathiazin 4,4-dioxid 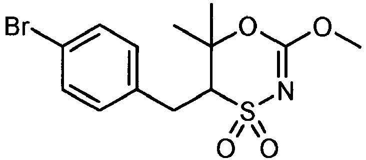
[5-((4-Brom-benzyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin

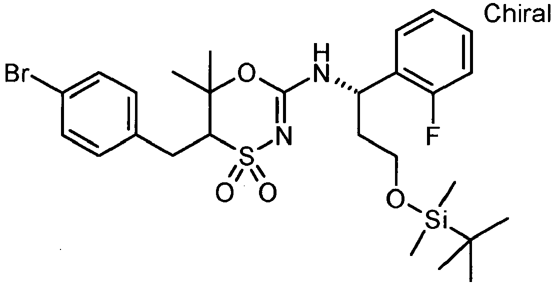

5-(4-Brom-benzyl-2-methoxy-6,6-dimethyl-5,6-dihydro-[1,4,3]oxathiazin 4,4-dioxid (0.33 g) und (S)-3-(tert-Butyl-dimethyl-silanyloxy)-1-(2-fluor-phenyl)-propylamin (0.31 g) wurden in Dichlormethan (2 ml) gelöst und unter leichtem Argonstrom solange gerührt, bis Kristalle entstanden. Dann wurde wieder Lösungsmittel zugesetzt und wieder langsam abgeblasen, bis sich keine Kristalle mehr bildeten. Das Öl wurde dann über Nacht stehen gelassen. Der Rückstand wurde chromatographisch mit Heptan / Ethylacetat gereinigt. Man erhielt so das gewünschte Produkt (250 mg).

Auf gleiche Weise wurden folgende Verbindungen hergestellt:
[5-((6-Chlor-pyridin-3-ylmethyl)-6,6-dimethyl-4,4-dioxo-5,6-dihydro-4H-4lambda6-[1,4,3]oxathiazin-2-yl]-[(S)-1-(2-fluor-phenyl)-ethyl]-amin 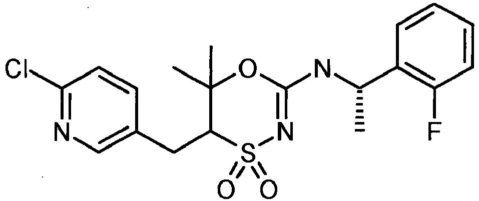
(5-Benzyl-6,6-dimethyl-4,4-dioxo-5,6-dihydro-4H-4lambda6-[1,4,3]oxathiazin-2-yl)-(4-fluor-bicyclo[2.2.2]oct-1-yl)-amin 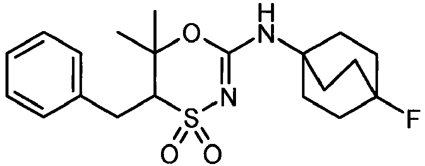
[5-((4-Brom-benzyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin 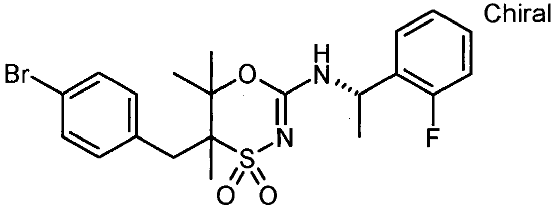
[5-((4-Brom-benzyl)-6,6-dimethyl-4,4-dioxo-5,6-dihydro-4H-4lambda6-[1,4,3]oxathiazin-2-yl]-[(S)-1-(2-fluor-phenyl)-ethyl]-amin 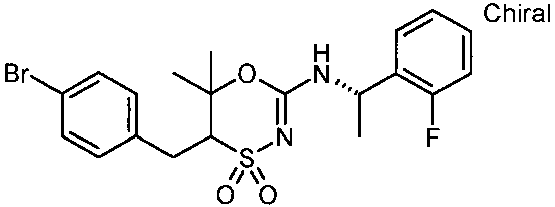
[5-((4-Brom-benzyl)-6,6-dimethyl-4,4-dioxo-5,6-dihydro-4H-4lambda6-[1,4,3]oxathiazin-2-yl]-[(S)-1-(2-chlor-phenyl)-ethyl]-amin 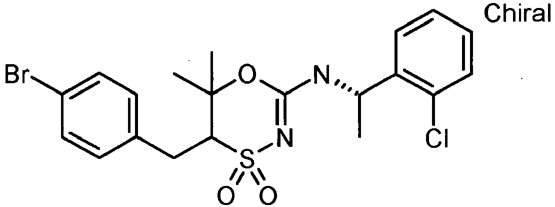
5-((6-Chlor-pyridin-3-ylmethyl)-2-methoxy-6,6-dimethyl-5,6-dihydro-[1,4,3]oxathiazin 4,4-dioxid 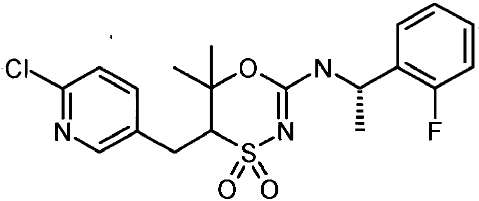
{5-[6-(4-tert-Butyt-piperazin-1-yl)-pyridin-3-ylmethyl]-6,6-dimethyl-4,4-dioxo-5,6-dihydro-4H-4lambda6-[1,4,3]oxathiazin-2-yl}-[(S)-1-(2-fluor-phenyl)-ethyl]-amin 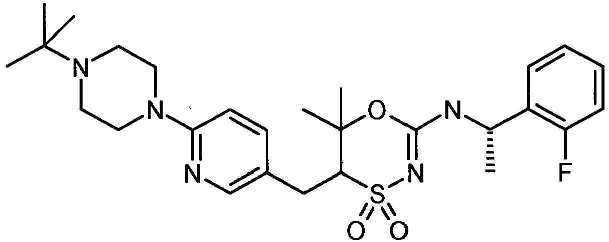
(S)-3-(5-Benzyl-6,6-dimethyl-4,4-dioxo-5,6-dihydro-4H-4lambda6-[1,4,3]oxathiazin-2-ylamino)-3-(2-fluor-phenyl)-propan-1-ol

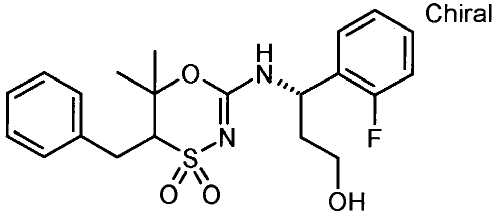

5-Benzyl-2-methoxy-6,6-dimethyl-5,6-dihydro-[1,4,3]oxathiazin 4,4-dioxid (0.2 g) und (S)-3-(tert-Butyl-dimethyl-silanyloxy)-1-(2-fluor-phenyl)-propylamin (0.2 g) wurden in Dichlormethan (2 ml) gelöst und unter leichtem Argonstrom solange gerührt, bis Kristalle entstanden. Dann wurde wieder Lösungsmittel zugesetzt und wieder langsam abgeblasen, bis sich keine Kristalle mehr bildeten. Das Öl wurde dann über Nacht stehen gelassen. Anschließend wurde der Rückstand in Methanol aufgenommen (3 ml) und Salzsäure 5 N (1.5 ml) zugesetzt und die Lösung im Vakuum bei 40 °C konzentriert. Der Rückstand wurde chromatographisch mit Heptan / Ethylacetat gereinigt.

Man erhielt so das Produkt (162 mg) mit dem Molekulargewicht 420.5 g / mol (C₂₁H₂₅FN₂O₄S), MS (ESI): (M+H+) 421 g / mol.

Auf gleiche Weise wurden folgende Verbindungen hergestellt:
(S)-3-(5-Benzyl-6,6-dimethyl-4,4-dioxo-5,6-dihydro-4H-4lambda6-[1,4,3]oxathiazin-2-ylamino)-3-(2-chlor-phenyl)-propan-1-ol 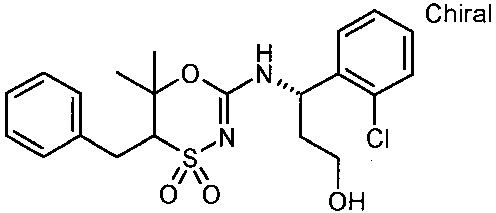
(S)-3-(5-Benzyl-6,6-dimethyl-4,4-dioxo-5,6-dihydro-4H-4lambda6-[1,4,3]oxathiazin-2-ylamino)-3-(3-chlor-phenyl)-propan-1-ol 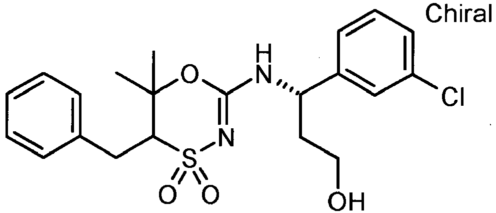
(S)-3-(5-Benzyl-6,6-dimethyl-4,4-dioxo-5,6-dihydro-4H-4lambda6-[1,4,3]oxathiazin-2-ylamino)-3-(4-chlor-phenyl)-propan-1-ol 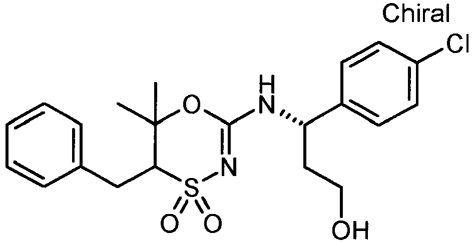
(S)-3-(5-Benzyl-6,6-dimethyl-4,4-dioxo-5,6-dihydro-4H-4lambda6-[1,4,3]oxathiazin-2-ylamino)-3-(2,4-difluor-phenyl)-propan-1-ol 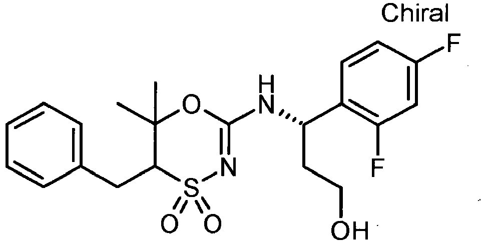
(S)-3-(5-Benzyl-6,6-dimethyl-4,4-dioxo-5,6-dihydro-4H-4lambda6-[1,4,3]oxathiazin-2-ylamino)-3-(2,5-difluor-phenyl)-propan-1-ol 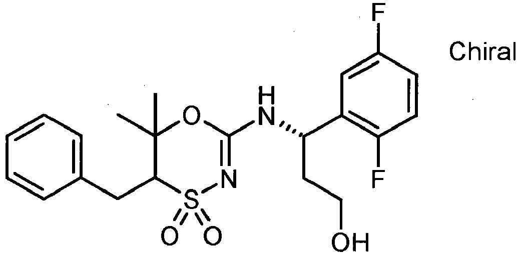
(S)-3-(5-Benzyl-6,6-dimethyl-4,4-dioxo-5,6-dihydro-4H-4lambda6-[1,4,3]oxathiazin-2-ylamino)-3-(2-chlor-3-fluor-phenyl)-propan-1-ol 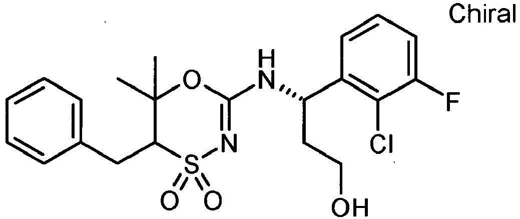
(S)-3-(5-Benzyl-6,6-dimethyl-4,4-dioxo-5,6-dihydro-4H-4lambda6-[1,4,3]]oxathiazin-2-ylamino)-3-(5-chlor-2-fluor-phenyl)-propan-1-ol 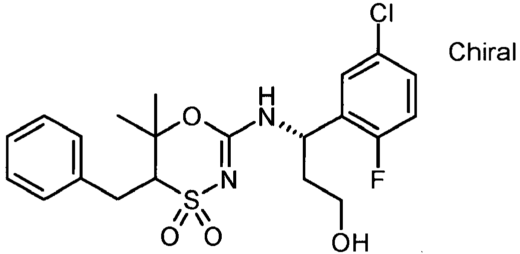
(S)-3-(5-Benzyl-6,6-dimethyl-4,4-dioxo-5,6-dihydro-4H-4lambda6-[1,4,3]oxathiazin-2-ylamino)-3-(5-chlor-2-methyl-phenyl)-propan-1-ol 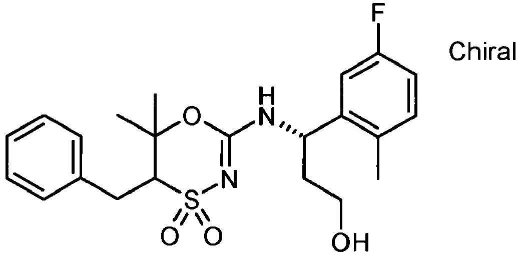
(S)-3-[5-(4-Brom-benzyl)-5,6,6-trimethyl-4,4-dioxo-5,6-dihydro-4H-4lambda6-[1,4,3]oxathiazin-2-ylamino]-3-(2-fluor-phenyl)-propan-1-ol 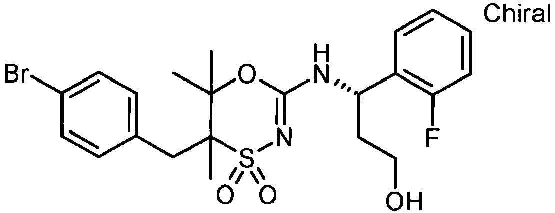
(S)-3-(5-Biphenyl-3-ylmethyl-6,6-dimethyl-4,4-dioxo-5,6-dihydro-4H-4lambda6-[1,4,3]oxathiazin-2-ylamino)-3-(2-fluor-phenyl)-propan-1-ol 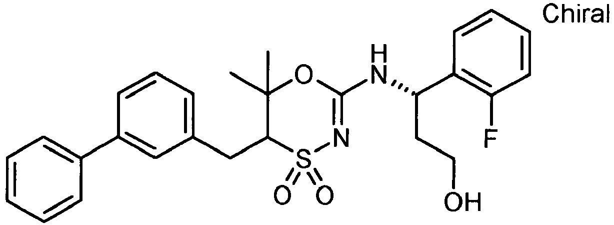
(S)-3-(5-Biphenyl-4-ylmethyl-6,6-dimethyl-4,4-dioxo-5,6-dihydro-4H-4lambda6-[1,4,3]oxathiazin-2-ylamino)-3-(2-fluor-phenyl)-propan-1-ol 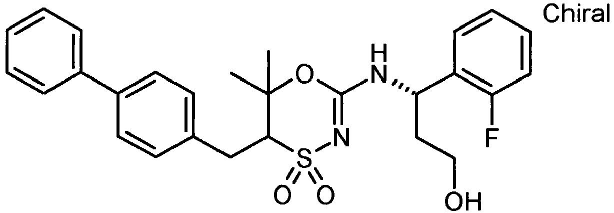
(S)-3-{6,6-Dimethyl-4,4-dioxo-5-[4-(5-trifluoromethyl-pyridin-2-yl)-benzyl]-5,6-dihydro-4H-4lambda6-[1,4,3]oxathiazin-2-ylamino}-3-(2-fluor-phenyl)-propan-1-ol 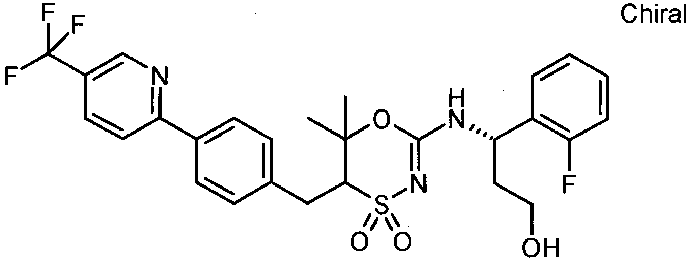
(S)-3-(6,6-Dimethyl-5-naphthalen-1-ylmethyl-4,4-dioxo-5,6-dihydro-4H-4lambda6-[1,4,3]oxathiazin-2-ylamino)-3-(2-fluor-phenyl)-propan-1-ol 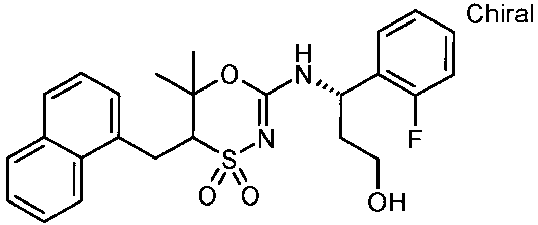
Amino-4-fluor-bicyclo[2.2.2]octan

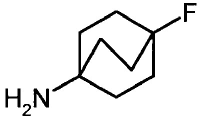

Journal of Organic Chemistry 1982, 47(15), 2951-2957.

### (5-Benzyl-5,6,6-trimethyl-4,4-dioxo-5,6-dihydro-4H-4lambda6-[1,4,3]oxathiazin-2-yl)-[(S)-1-(2-fluor-phenyl)-ethyl]-amin

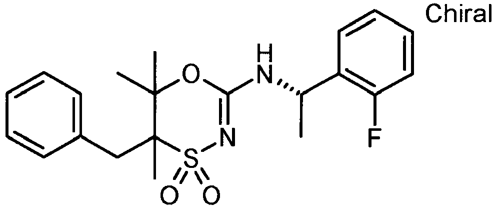

[5-(4-Brom-benzyl)-5,6,6-trimethyl-4,4-dioxo-5,6-dihydro-4H-4lambda6-[1,4,3]oxathiazin-2-yl]-[(S)-1-(2-fluor-phenyl)-ethyl]-amin (13.5 mg) wurden in Ethanol gelöst und mit Pd/C, Wasserstoff im Autoklaven hydriert. Das Palladium wurde abfiltriert und die Lösung konzentriert. Man erhielt so das Produkt (10 mg) mit dem Molekulargewicht 404.5 g / mol (C₂₁H₂₅FN₂O₃S), MS (ESI): (M+H+) 405 g / mol.

### N,N-Bis-(2,4-dimethoxy-benzyl)-methansulfonamid

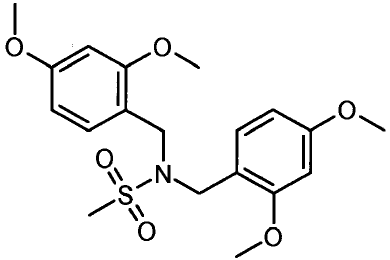

Zu einer Lösung aus Methansulfonylchlorid (3.8 g) in Dichlormethan (50 ml) wurde Bis-(2,4-dimethoxybenzyl)-amin (10 g) portionsweise bei Raumtemperatur zugegeben. Die Suspension wurde noch 30 Minuten gerührt, mit Wasser (100 ml) versetzt und die organische Phase abgetrennt. Die wässrige Phase wurde mit Dichlormethan (2 x 50 ml) extrahiert. Die vereinigten organischen Phasen wurden getrocknet (Na₂SO₄), filtriert und im Vakuum konzentriert. Der Rückstand wurde mit Normalphasenchromatographie (Dichlormethan) gereinigt. Man erhielt so das gewünschte Produkt (10,8 g).

### 2-[4-(5-Trifluormethyl-pyridin-2-yl)-phenyl]-ethansulfonsäure bis-(2,4-dimethoxy-benzyl)-amid

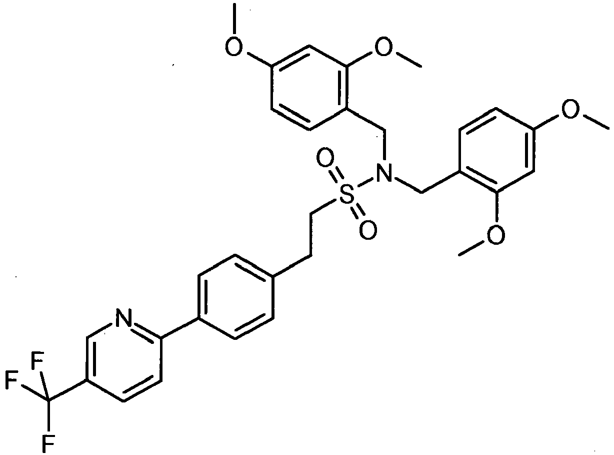

Zu einer Lösung aus N,N-Bis-(2,4-dimethoxy-benzyl)-methansulfonamid (1.3 g) in THF (25 ml) wurde bei -78 °C Butyllithium 1.6 N in Hexan (2.5 ml) zugetropft und dreißig Minuten gerührt. Anschließend wurde 2-(4-Chloromethyl-phenyl)-5-trifluoromethyl-pyridin (1.16 g) zugegeben und die Kühlung entfernt. Nach einer Stunde wurde das Lösungsmittel im Vakuum entfernt, der Rückstand in wenig Ethylacetat aufgenommen und chromatographisch (Ethylacetat / Heptan) gereinigt. Man erhielt so das Produkt (1.1 g) mit dem Molekulargewicht 630.6 g / mol (C₃₂H₃₃F₃N₂O₆S), MS (ESI): (M+H+) 631 g / mol.

Auf gleiche Weise wurden folgende Verbindungen hergestellt:
2-Biphenyl-4-yl-ethansulfonsäure bis-(2,4-dimethoxy-benzyl)-amid 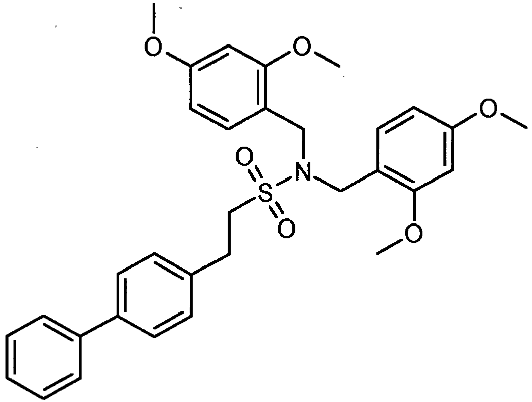
2-Biphenyl-3-yl-ethansulfonsäure bis-(2,4-dimethoxy-benzyl)-amid 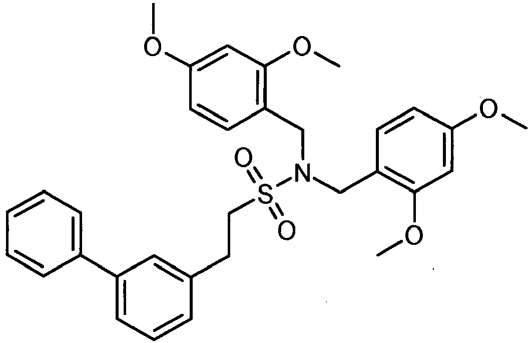
2-((6-Chlor-pyridin-3-yl)-ethansulfonsäure bis-(2,4-dimethoxy-benzyl)-amid 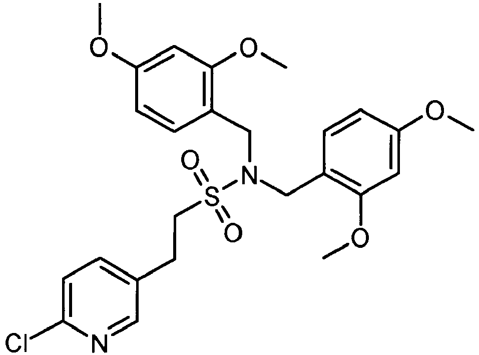
2-[6-(4-tert-Butyl-piperazin-1-yl)-pyridin-3-yl]-2-hydroxy-ethansulfonsäure bis-(2,4-dimethoxybenzyl)-amid 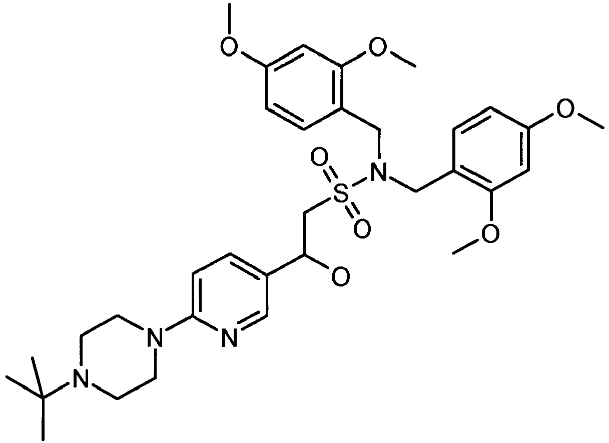
2-[6-(4-tert-Butyl-piperazin-1-yl)-pyridin-3-yl]-ethensulfonsäure bis-(2,4-dimethoxy-benzyl)-amid

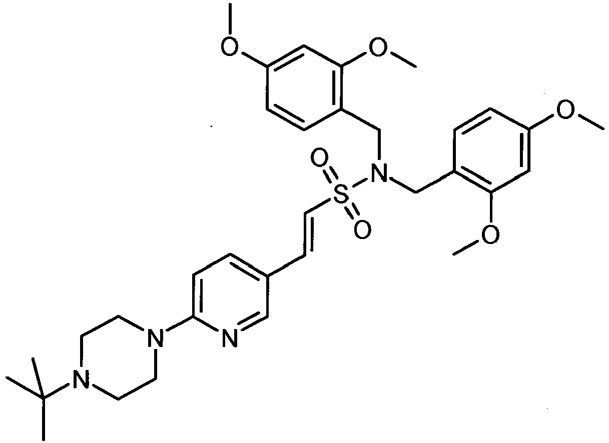

Zu einer Lösung aus 2-[6-(4-tert-Butyl-piperazin-1-yl)-pyridin-3-yl]-2-hydroxy-ethansulfonsäure bis-(2,4-dimethoxy-benzyl)-amid (3,75 g) und Triethylamin (0,7 g) in Methylenchlorid (20 ml) wurde Methylsulfonsäurechlorid (0,8 g) zugetropft und anschließend die Lösung bei 40 °C für zwei Stunden gerührt. Dann wurde die organische Phase gewaschen (NaCl-Lösung, 50 ml), filtriert, getrocknet (Na₂SO₄) und konzentriert. Der Rückstand wurde ohne weiter Aufarbeitung verwendet.
2-[6-(4-tert-Butyl-piperazin-1-yl)-pyridin-3-yl]-ethansulfonsäure bis-(2,4-dimethoxy-benzyl)-amid 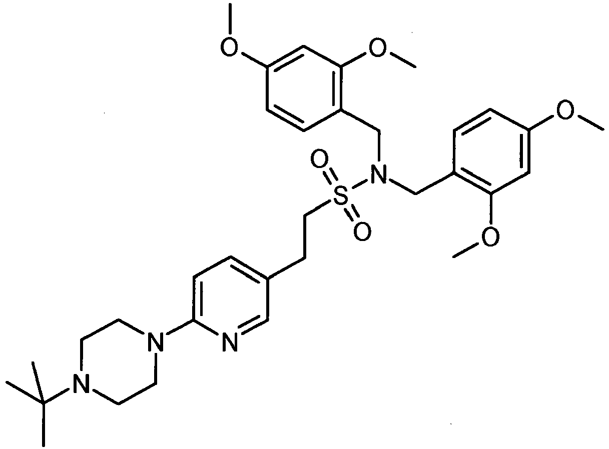
2-[6-(4-tert-Butyl-piperazin-1-yl)-pyridin-3-yl]-ethensulfonsäure bis-(2,4-dimethoxy-benzyl)-amid wurde in Ethanol gelöst, Palladium auf Kohle zugefügt und anschlißend unter 5 bar Wasserstoffatmosphäre 24 Stunden gerührt. Das Palladium wurde abfiltriert, die Lösung konzentriert und der Rückstand chromatographisch (EtOAc / Heptan) gereinigt.
2-Phenyl-ethansulfonsäure 2,4-dimethoxy-benzylamid

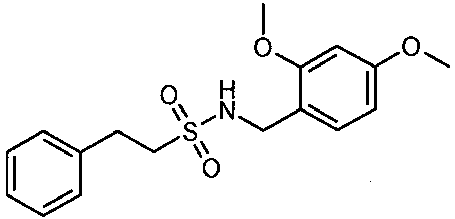

Zu einer Lösung aus 2-Phenylethansulfonylchlorid (3 g) in Dichlormethan (50 ml) wurde bei 0 °C langsam 2,4-Dimethoxybenzylamin (5 g) gelöst in Dichlormethan (5 ml) zugetropft. Die Kühlung wurde entfernt und es wurde noch zwei Stunden gerührt. Die entstandene Suspension wurde mit Wasser (100 ml) versetzt und die organische Phase abgetrennt. Die organische Phase wurde dann mit Salzsäure 2N (100 ml) und Natriumhydrogencarbonat gesättigte Lösung (100 ml) gewaschen, getrocknet (Na₂SO₄), filtriert und konzentriert. Man erhielt so das Produkt (5 g), welches ohne weitere Aufarbeitung verwendet wurde.

Auf gleiche Weise wurden folgende Verbindungen hergestellt:
2-((4-Chlor-phenyl)-ethansulfonsäure 2,4-dimethoxy-benzylamid 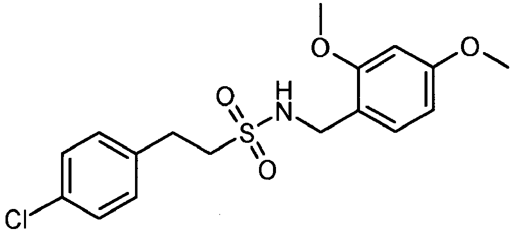
2-Naphthalen-1-yl-ethansulfonsäure 2,4-dimethoxy-benzylamid 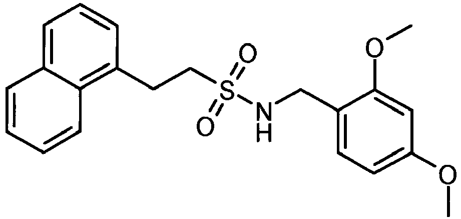
3-Hydroxy-3-methyl-1-[4-(5-trifluormethyl-pyridin-2-yl)-phenyl]-butan-2-sulfonsäureamid

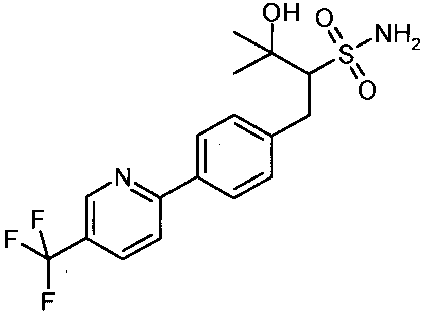

Zu einer Lösung aus 2-[4-(5-Trifluormethyl-pyridin-2-yl)-phenyl]-ethansulfonsäure bis-(2,4-dimethoxy-benzyl)-amid (1.1 g) in THF (10 ml) wurde bei -78 °C Phenyllithium in Hexan 1.8 N (2.9 ml) zugetropft, anschließend wurde der Ansatz auf -20 °C für 5 Minuten erwärmt und dann wieder auf -78 °C abgekühlt. Nach Zugabe des Acetons (0.9 ml) wurde noch fünfzehn Minuten bei dieser Temperatur gerührt und dann mit Trifluoressigsäure (1 ml) gequenscht. Die Lösung wurde im Vakuum konzentriert, in Dichlormethan (10 ml) aufgenommen und mit Trifluoressigsäure (3 ml) versetzt und über Nacht gerührt. Die Lösung wurde konzentriert und ohne weitere Aufarbeitung chromatographiert (Dichlormethan / Methanol). Man erhielt so das Produkt (0.29 g) mit dem Molekulargewicht 388.4 g / mol (C₁₇H₁₉F₃N₂O₃S), MS (ESI): (M+H+) 389 g / mol.

Auf gleiche Weise wurden folgende Verbindungen hergestellt:
1-Biphenyl-4-yl-3-hydroxy-3-methyl-butan-2-sulfonsäureamid 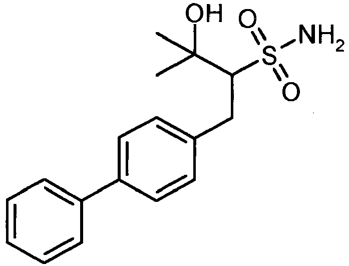
1-Biphenyl-3-yl-3-hydroxy-3-methyl-butan-2-sulfonsäureamid 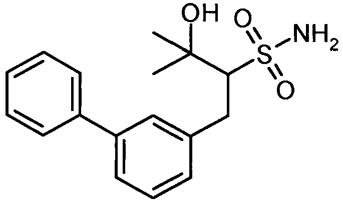
3-Hydroxy-3-methyl-1-phenyl-butan-2-sulfonsäureamid 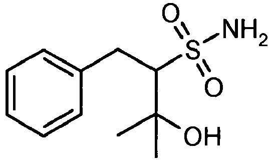
1-((4-Chlor-phenyl)-3-hydroxy-3-methyl-butan-2-sulfonsäureamid 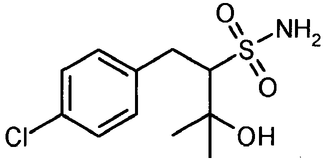
3-Hydroxy-3-methyl-1-naphthalen-1-yl-butan-2-sulfonsäureamid 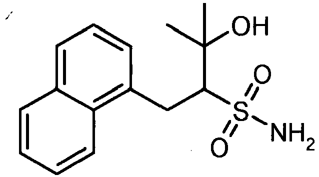
1-((6-Chlor-pyridin-3-yl)-3-hydroxy-3-methyl-butan-2-sulfonsäureamid 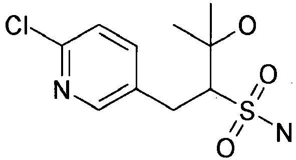
1-[6-(4-tert-Butyl-piperazin-1-yl)-pyridin-3-yl]-3-hydroxy-3-methyl-butan-2-sulfonsäureamid 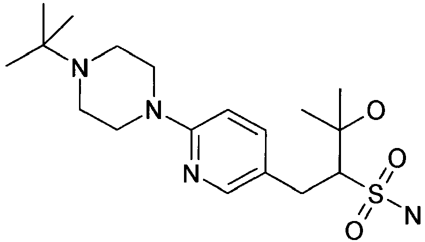
2-Methoxy-6,6-dimethyl-5-[4-(5-trifluormethyl-pyridin-2-yl)-benzyl]-5,6-dihydro[1,4,3]oxathiazin 4,4-dioxid

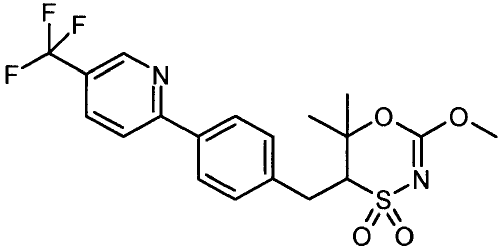

Eine Lösung aus 3-Hydroxy-3-methyl-1-[4-(5-trifluormethyl-pyridin-2-yl)-phenyl]-butan-2-sulfonsäureamid (0.29g) in Essigsäure (1ml) und Tetramethylorthocarbonat (5 g) wurde unter einem Argonstrom fünf Stunden auf 100 °C erwärmt. Dabei wurde entstehendes Methanol aus dem Gemisch entfernt. Anschließend wurde die Lösung mit Diatomeenerde aufgenommen und chromatographisch (Ethylacetat / Heptan) gereinigt. Man erhielt so das Produkt (0.085 g) mit dem Molekulargewicht 428.4 g / mol (C₁₉H₁₉F₃N₂O₄S), MS (ESI): (M+H+) 429 g / mol.

Auf gleiche Weise wurden folgende Verbindungen hergestellt:
5-Biphenyl-4-ylmethyl-2-methoxy-6,6-dimethyl-5,6-dihydro-[1,4,3]oxathiazin 4,4-dioxid 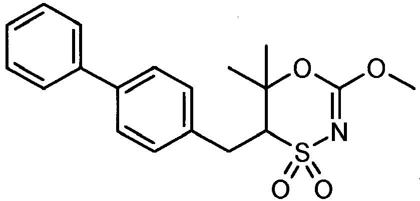
Biphenyl-3-ylmethyl-2-methoxy-6,6-dimethyl-5,6-dihydro-[1,4,3]oxathiazin 4,4-dioxid 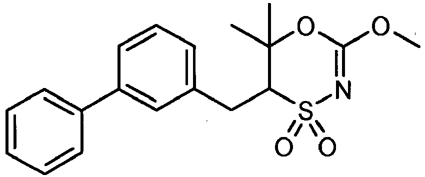
2-Methoxy-6,6-dimethyl-5-naphthalen-1-ylmethyl-5,6-dihydro-[1,4,3]oxathiazin 4,4-dioxid 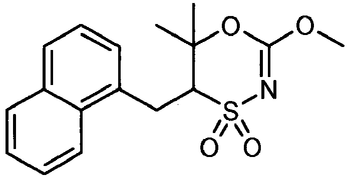
5-((6-Chlor-pyridin-3-ylmethyl)-2-methoxy-6,6-dimethyl-5,6-dihydro-[1,4,3]oxathiazin 4,4-dioxid 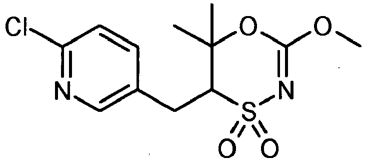
5-[6-(4-tert-Butyl-piperazin-1-yl)-pyridin-3-ylmethyl]-2-methoxy-6,6-dimethyl-5,6-dihydro-[1,4,3]oxathiazin 4,4-dioxid 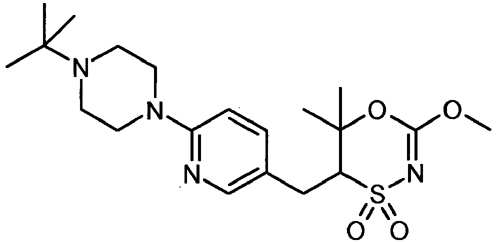
[(S)-3-(tert-Butyl-dimethyl-silanyloxy)-1-(2-fluor-phenyl)-propyl]-[5,6,6-trimethyl-4,4-dioxo-5-(4-pyridin-2-yl-benzyl)-5,6-dihydro-4H-4lambda6-[1,4,3]oxathiazin-2-yl]-amin

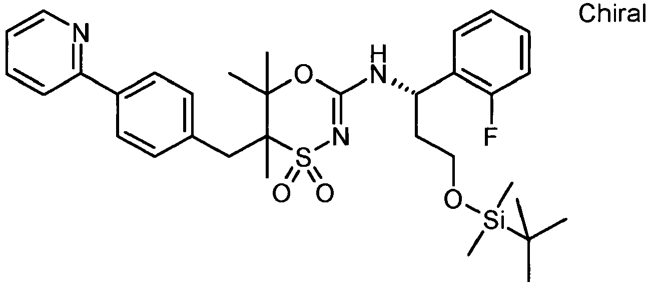

Zu einer Lösung aus Pyridin-2-boronsäure (0.028 g) und Cs₂CO₃ (0.17 g) in entgastem Toluen/Wasser (2.1 ml/ 0.9 ml) wurde [5-(4-Brom-benzyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin (0.11 g) und Bis-(benzyliden-aceton)palladium (0.001 g) für sechs Stunden auf 80 °C erhitzt. Nach dem Abkühlen wurde mit Natriumhydrogencarbonat-Lösung halbkonzentriert (5 ml) versetzt und die Suspension mit Ethylacetat (3 x 20 ml) extrahiert. Die organische Lösung wurde getrocknet (Na₂SO₄), filtriert und konzentriert. Der Ansatz wurde ohne weitere Aufarbeitung weiter verwendet.

Auf gleiche Weise wurden folgende Verbindungen hergestellt:
[(S)-3-(tert-Butyl-dimethyl-silanyloxy)-1-(2-fluor-phenyl)-propyl]-{5,6,6-trimethyl-4,4-dioxo-5-[4-(5-trifluormethyl-pyridin-2-yl)-benzyl]-5,6-dihydro-4H-4lambda6-[1,4,3]oxathiazin-2-yl}-amin 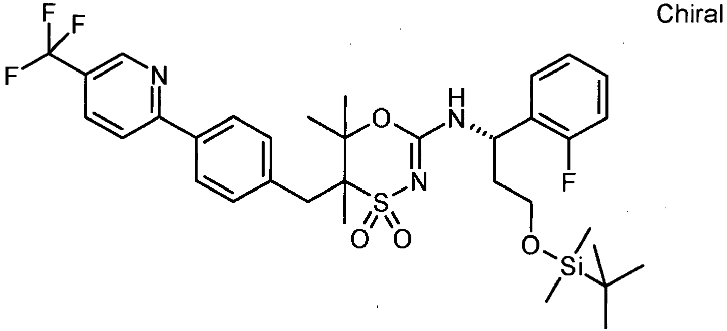
(S)-3-(2-Fluor-phenyl)-3-[5,6,6-trimethyl-4,4-dioxo-5-(4-pyridin-2-yl-benzyl)-5,6-dihydro-4H-4lambda6-[1,4,3]oxathiazin-2-ylamino]-propan-1-ol 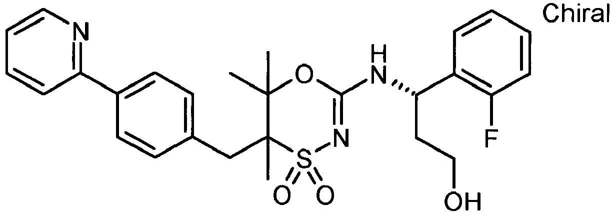
[(S)-3-(tert-Butyl-dimethyl-silanyloxy)-1-(2-fluor-phenyl)-propyl]-[5,6,6-trimethyl-4,4-dioxo-5-(4-pyridin-2-yl-benzyl)-5,6-dihydro-4H-4lambda6-[1,4,3]oxathiazin-2-yl]-amin (0.11 mg) wurde in Methanol (3 ml) aufgenommen, mit konzentrierter Salzsäure (0.3 ml) versetzt und eine Stunde gerührt. Die Reinigung erfolgte aus der Lösung mit präparativer HPLC. Man erhielt so das Produkt (0.007 g) mit dem Molekulargewicht 511.6 g / mol (C₂₅H₂₉FN₂O₄S), MS (ESI): (M+H+) 512 g / mol.

Auf gleiche Weise wurden folgende Verbindungen hergestellt:
(S)-3-(2-Fluor-phenyl)-3-{5,6,6-trimethyl-4,4-dioxo-5-[4-(5-trifluormethyl-pyridin-2-yl)-benzyl]-5,6-dihydro-4H-4lambda6-[1,4,3]oxathiazin-2-ylamino}-propan-1-ol 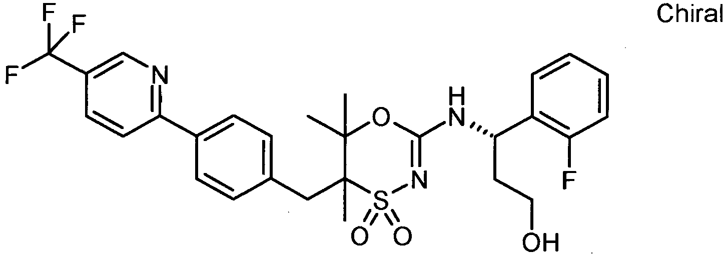
(S)-3-(5-Benzyl-6,6-dimethyl-4,4-dioxo-5,6-dihydro-4H-4lambda6-[1,4,3]oxathiazin-2-ylamino)-3-phenyl-propan-1-ol

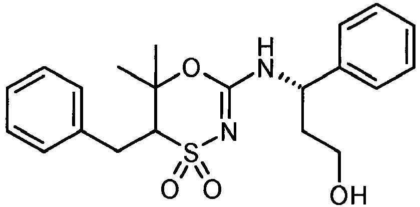

Eine Lösung aus (S)-3-(tert-Butyl-dimethyl-silanyloxy)-1-phenyl-propylamin (0.301 g) in Dichlormethan (8 ml) wurde mit 1,1'-Thiocarbonyldiimidazol (0.234 mg) versetzt. Nach zwanzig Minuten bei Raumtemperatur wurde mit Diethylether (10 ml) und n-Pentan (10 ml) versetzt, mit Wasser (20 ml) gewaschen, getrocknet (Na₂SO₄), filtriert und im Vakuum konzentriert. Der Rückstand wurde in NMP (5 ml) gelöst und mit 3-Hydroxy-3-methyl-1-phenyl-butan-2-sulfonsäureamid (0.24 g) gelöst in NMP (4 ml) versetzt. Dann wurde mit Natriumbistrimethylsilylamid 2N (0.296 ml) versetzt und eine Stunde gerührt. Anschließend wurde N-Bromsuccinimid (0.105 g) zugegeben und eine weitere Stunde gerührt. Die Mischung wurde mit Wasser (80 ml) versetzt und mit Ethylacetat (3 x 50 ml) extrahiert. Die vereinigten organischen Phasen wurden nochmals mit gesättigter Natriumchloridlösung (50 ml) gewaschen, getrocknet (Na₂SO₄), filtriert und konzentriert. Der Rückstand wurde mit präparativer HPLC gereinigt. Man erhielt so das Produkt (0.063 g) mit dem Molekulargewicht 402.5 g / mol (C₂₁H₂₆N₂O₄S), MS (ESI): (M+H+) 403 g / mol.

Auf gleiche Weise wurden folgende Verbindungen hergestellt:
(S)-3-[5-(4-Chlor-benzyl)-6,6-dimethyl-4,4-dioxo-5,6-dihydro-4H-4lambda6-[1,4,3]oxathiazin-2-ylamino]-3-phenyl-propan-1-ol 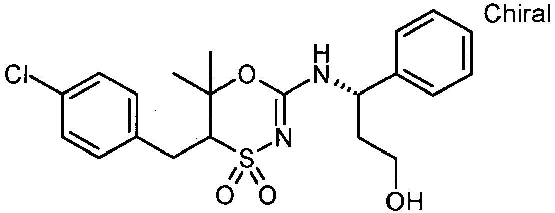
4-((4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-3,6-dihydro-2H-pyridin-1-carbonsäure tert-butyl ester

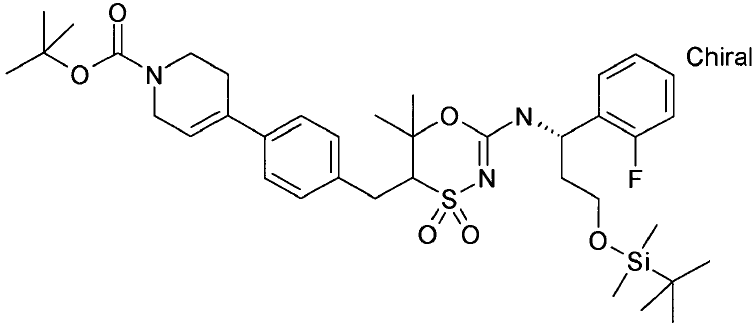

Eine Lösung aus [5-(4-Brom-benzyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin (2,42 g), 4-(4,4,5,5-Tetramethyl-[1,3,2]dioxaborolan-2-yl)-3,6-dihydro-2H-pyridine-1-carbonsäure tert-butyl ester (1,49 g) und Cs₂CO₃ (2,57 g) in Dioxan / Wasser (30 ml / 10 ml) wurde 20 Minuten mit Argon entgast. Anschließend wurde Bis(dibenzylidenacetone)palladium II (0,23 g) und CTC-Q-Phos (0,56 g) zugegeben und 30 Minuten bei 70 °C gerührt. Anschließend wurde mit Wasser (100 ml) versetzt und die wässrige Emulsion mit Etthylacetat (3 x 50 ml) extrahiert. Die organische Phase wurde dann getrocknet (Na₂SO₄), filtriert und konzentriert. Der Rückstand wurde mit Normalphasen Chromatographie (Ethylacetat / Heptan 1 : 4) gereinigt. Man erhielt so das Produkt (2.08 g) mit dem Molekulargewicht 716 g / mol (C37H54FN3O6SSi), MS (ESI): (M+H+) 716 g / mol

Auf gleiche Weise wurden folgende Verbindungen hergestellt:
4-((4-{2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-phenyl)-3,6-dihydro-2H-pyridine-1-carbonsäuretertiärbutylester 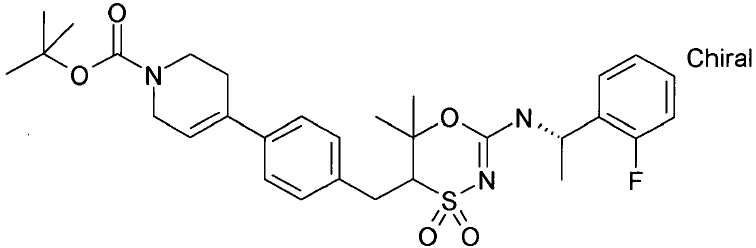
5-{2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-3',6'-dihydro-2'H-[2,4']bipyridinyl-1'-carbonsäuretertiärbutylester 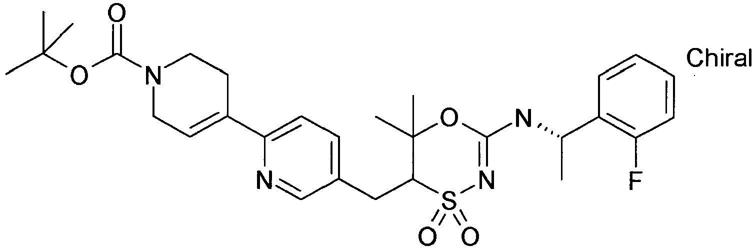
4-((4-{2-[(S)-1-(2-Chlor-phenyl)-ethylamino]-6,6-dimethyl-4,4-dioxo-5,6-dihydro-4H-4lambda6-[1,4,3]oxathiazin-5-ylmethyl}-phenyl)-3,6-dihydro-2H-pyridine-1-carbonsäuretertiärbutylester 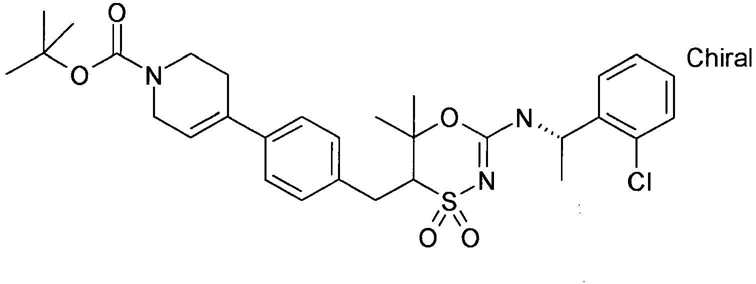
4-((4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-piperidin-1-carbonsäure tert-butyl ester 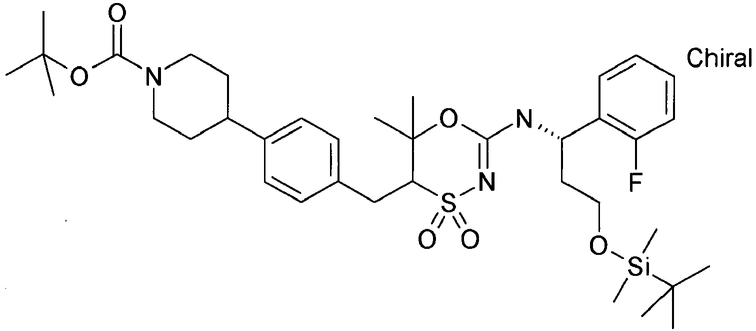
4-((4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-3,6-dihydro-2H-pyridin-1-carbonsäure tert-butyl ester (1,8 g) wurde in Ethanol gelöst, Palladium auf Aktivkohle zugegeben und zwei Stunden unter Wasserstoff-Atmosphäre gerührt. Die Suspension wurde filtriert und im Vakuum konzentriert. Der Rückstand wurde ohne weitere Aufarbeitung weiterverwendet. Man erhielt so das Produkt (1.8 g) mit dem Molekulargewicht 718 g / mol (C₃₇H₅₆FN₃O₆SSi), MS (ESI): (M+H+) 718 g / mol.

Auf gleiche Weise wurden folgende Verbindungen hergestellt:
4-((4-{2-[(S)-1-(2-Fluor-phenyl)-ethylamino]-6,6-dimethyl-4,4-dioxo-5,6-dihydro-4H-4lambda6-[1,4,3]oxathiazin-5-ylmethyl]-phenyl)-piperidine-1-carbonsäuretertiärbutylester 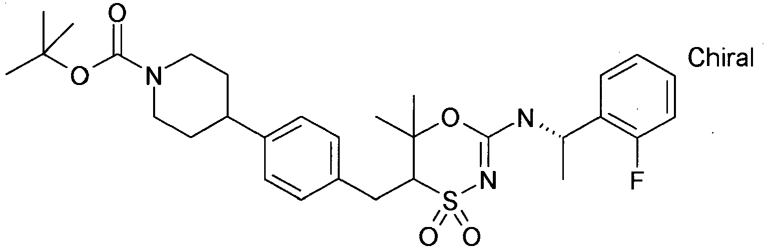
5-{2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-3',4',5',6'-tetrahydro-2'H-[2,4']bipyridinyl-1'-carbonsäuretertiärbutylester 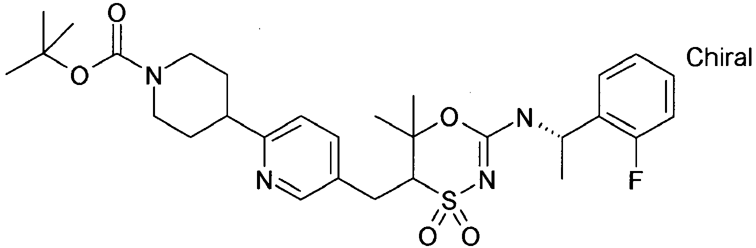
4-((4-{2-[(S)-1-(2-Chlor-phenyl)-ethylamino]-6,6-dimethyl-4,4-dioxo-5,6-dihydro-4H-4lambda6-[1,4,3]oxathiazin-5-ylmethyl}-phenyl)-piperidine-1-carbonsäuretertiärbutylester 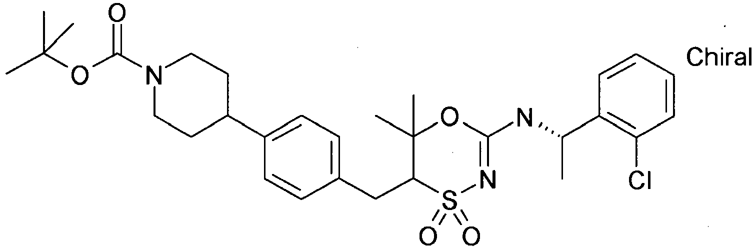
(S)-3-{6,6-Dimethyl-4,4-dioxo-5-[4-(1,2,3,6-tetrahydro-pyridin-4-yl)-benzyl]-5,6-dihydro-4H-4lambda6-[1,4,3]oxathiazin-2-ylamino}-3-(2-fluor-phenyl)-propan-1-ol

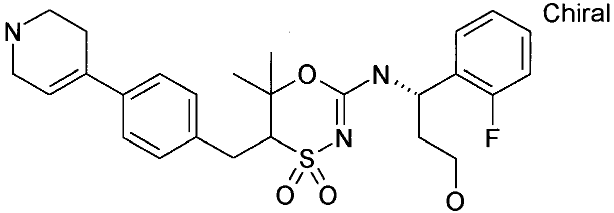

Zu einer Lösung aus 4-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-3,6-dihydro-2H-pyridin-1-carbonsäure tert-butyl ester (0,12 g) in Methanol (2 ml) wurde Salzsäure 37 % (0,1 ml) zugegeben und über Nacht bei Raumtemperatur gerührt. Die Lösung wurde anschließend mit präparativer HPLC gereinigt. Man erhielt so das Produkt (0.085 g) mit dem Molekulargewicht 501 g / mol (C₂₆H₃₂FN₃O₄S), MS (ESI): (M+H+) 502 g / mol.

Auf die gleiche wurde folgende Verbindung hergestellt:
(S)-3-[6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol 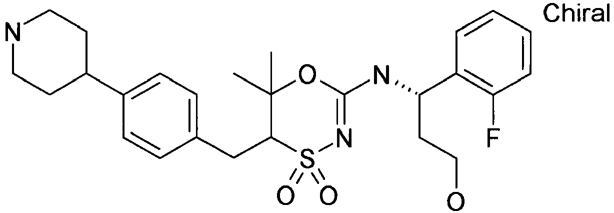
[6,6-Dimethyl-4,4-dioxo-5-(4-piperidin-4-yl-benzyl)-5,6-dihydro-4H-4lambda6-[1,4,3]oxathiazin-2-yl]-[(S)-1-(2-fluor-phenyl)-ethyl]-amin 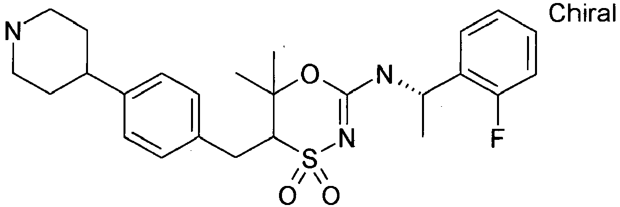
[6,6-Dimethyl-4,4-dioxo-5-(4-piperidin-4-yl-benzyl)-5,6-dihydro-4H-4lambda6-[1,4,3]oxathiazin-2-yl]-[(S)-1-(2-chlor-phenyl)-ethyl]-amin 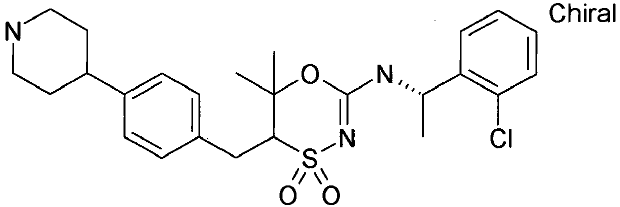
[(R)-6,6-Dimethyl-4,4-dioxo-5-(4-piperazin-1-yl-benzyl)-5,6-dihydro-4H-4lambda6-[1,4,3]oxathiazin-2-yl]-[(S)-1-(2-fluor-phenyl)-ethyl]-amin 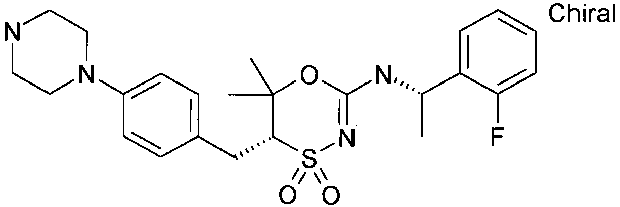
{5-[((R)-4-(2-Amino-2-methyl-propoxy)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin 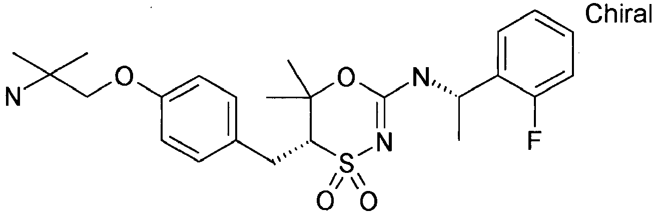
[(S)-1-(2-Fluor-phenyl)-ethyl]-[5-(1',2',3',4',5',6'-hexahydro-[2,4']bipyridinyl-5-ylmethyl)-6,6-dimethyl-4,4-dioxo-5,6-dihydro-4H-4lambda6-[1,4,3]oxathiazin-2-yl]-amin 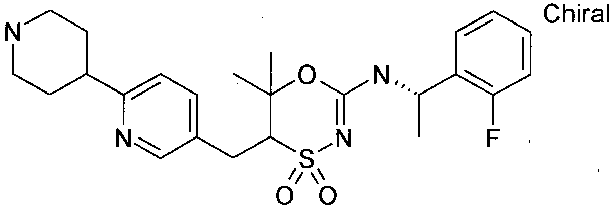
(S)-3-{6,6-Dimethyl-5-[4-(1-methyl-1,2,3,6-tetrahydro-pyridin-4-yl)-benzyl]-4,4-dioxo-5,6-dihydro-4H-4lambda6-[1,4,3]oxathiazin-2-ylamino}-3-(2-fluor-phenyl)-propan-1-ol

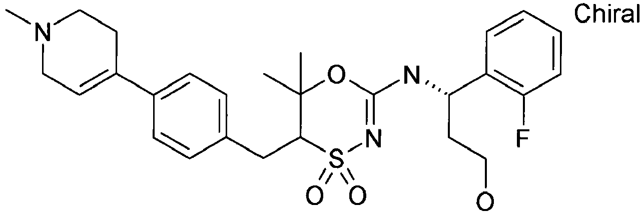

Zu einer Lösung aus (S)-3-{6,6-Dimethyl-4,4-dioxo-5-[4-(1,2,3,6-tetrahydro-pyridin-4-yl)-benzyl]-5,6-dihydro-4H-4lambda6-[1,4,3]oxathiazin-2-ylamino}-3-(2-fluor-phenyl)-propan-1-ol (0.07 g) in Methanol (1 ml) wurde Paraformaldehyd (47 mg) und Natriumcyanoborhydrid (50 mg) gegeben und sechs Stunden unter Rückfluß erhitzt. Nach dem Abkühlen wurde die Suspension filtriert. Das Methanol wurde im Vakuum entfernt, der Rückstand in Ethylacetat (50 ml) gelöst und mit Salzsäure 2N (2 x 30 ml) extrahiert. Die saure Phase wurde dann auf pH >13 mit NaOH 2N gestellt und mit Methylenchlorid (2 x 30 ml) extrahiert. Die organische Lösung wurde getrocknet (Na₂SO₄), filtriert und konzentriert. Man erhielt so das Produkt (0.085 g) mit dem Molekulargewicht 515 g / mol (C₂₇H₃₄FN₃O₄S), MS (ESI): (M+H+) 516 g / mol.

Auf gleiche Weise erhielt man folgendes Produkt:
(S)-3-{6,6-Dimethyl-5-[4-(1-methyl-piperidin-4-yl)-benzyl]-4,4-dioxo-5,6-dihydro-4H-4lambda6-[1,4,3]oxathiazin-2-ylamino}-3-(2-fluor-phenyl)-propan-1-ol 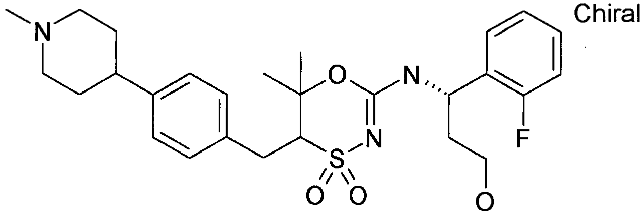
(S)-3-{(S)-6,6-Dimethyl-5-[4-(1-oxetan-3-yl-piperidin-4-yl)-benzyl]-4,4-dioxo-5,6-dihydro-4H-4lambda6-[1,4,3]oxathiazin-2-ylamino}-3-(2-fluor-phenyl)-propan-1-ol 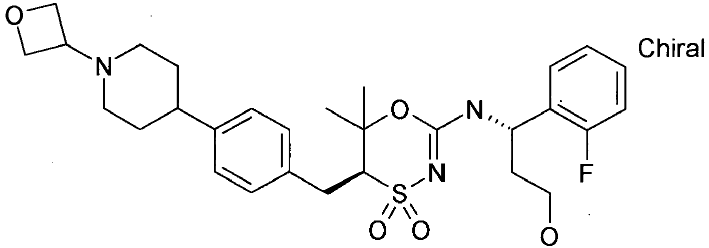
{(S)-6,6-Dimethyl-5-[4-(1-oxetan-3-yl-piperidin-4-yl)-benzyl]-4,4-dioxo-5,6-dihydro-4H-4lambda6-[1,4,3]oxathiazin-2-yl}-[(S)-1-(2-fluor-phenyl)-ethyl]-amin 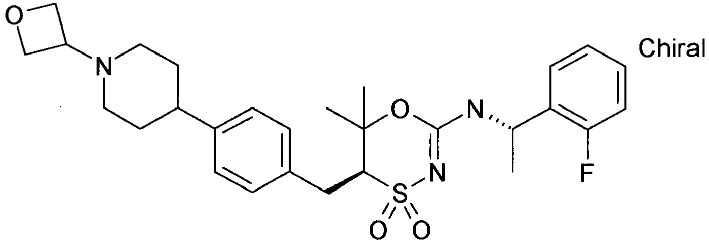
[6,6-Dimethyl-5-(1'-oxetan-3-yl-1',2',3',4',5',6'-hexahydro-[2,4']bipyridinyl-5-ylmethyl)-4,4-dioxo-5,6-dihydro-4H-4lambda6-[1,4,3]oxathiazin-2-yl]-[(S)-1-(2-fluor-phenyl)-ethyl]-amin 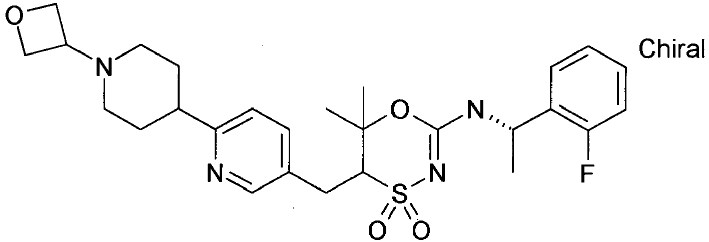
[(S)-1-(2-Chlor-phenyl)-ethyl]-{6,6-dimethyl-5-[4-(1-oxetan-3-yl-piperidin-4-yl)-benzyl]-4,4-dioxo-5,6-dihydro-4H-4lambda6-[1,4,3]oxathiazin-2-yl}-amin 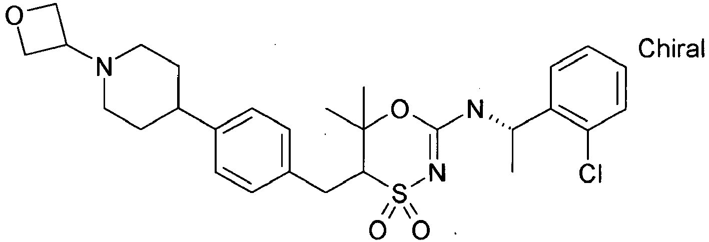
(S)-3-{5-[4-(1-Cyclopropyl-piperidin-4-yl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol

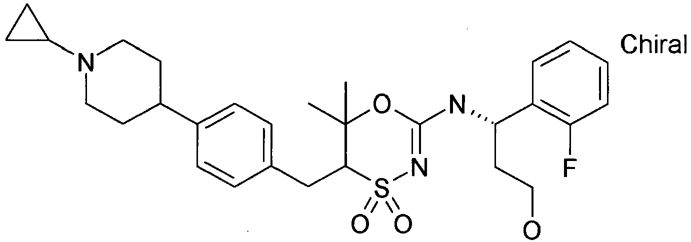

Eine Lösung aus (S)-3-[6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol (0.08 g), Essigsäure (0.065 ml) Natriumcyoanoborhydrid (0.01g) und (1-Ethoxy-cyclopropylmethyl)-trimet hyl-silan (0.065 g) in DMF (1 ml) wurde über Nacht bei 65 °C gerührt. Die Suspension wurde mit Wasser versetzt (20 ml) und auf pH >13 gestellt. Anschließend wurde mit Methylenchlorid (2 x 20 ml) extrahiert, die organische Phase getrocknet (Na₂SO₄), filtriert und konzentriert. Der Rückstand wurde mit präparativer HPLC gereinigt. Man erhielt so das Produkt (0.085 g) mit dem Molekulargewicht 543 g / mol (C₂₉H₃₈FN₃O₄S), MS (ESI): (M+H+) 544 g / mol.

Auf gleiche Weise erhielt man folgende Produkte:
{5-[4-(1-Cyclopropyl-piperidin-4-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin 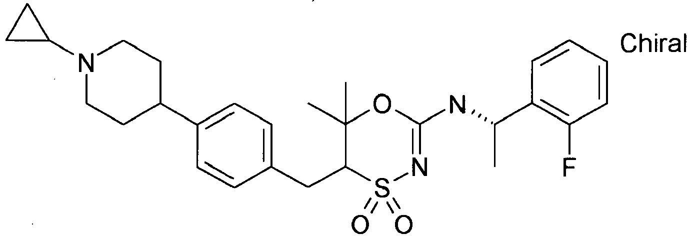
[5-((1'-Cyclopropyl-1',2',3',4',5',6'-hexahydro-[2,4']bipyridinyl-5-ylmethyl)-6,6-dimethyl-4,4-dioxo-5,6-dihydro-4H-4lambda6-[1,4,3]oxathiazin-2-yl]-[(S)-1-(2-fluor-phenyl)-ethyl]-amin 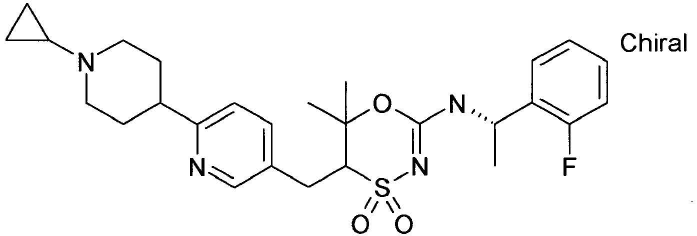
(S)-3-(2-Fluor-phenyl)-3-(5-{4-[1-(2-hydroxy-ethyl)-piperidin-4-yl]-benzyl}-6,6-dimethyl-4,4-dioxo-5,6-dihydro-4H-4lambda6-[1,4,3]oxathiazin-2-ylamino)-propan-1-ol

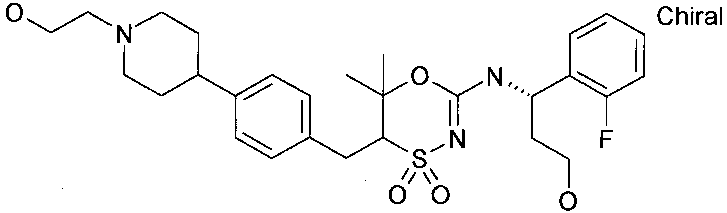

Zu einer Lösung aus (S)-3-[6,6-Dimethyl-4,4-dioxo-5-(4-piperidin-4-yl-benzyl)-5,6-dihydro-4H-4lambda6-[1,4,3]oxathiazin-2-ylamino]-3-(2-fluor-phenyl)-propan-1-ol (0.08 g) in DMF (1 ml) wurde Hünig-Base (0.065 ml) und 2-Bromethanol (0.013 ml) getropft und dann 16 Stunden bei 80 °C gerührt. Nach dem Abkühlen wurde mit Methylenchlorid (20 ml) versetzt und die organische Phase mit Salzsäure 1N (2 x 20 mL) extrahiert. Die wässrige Phase wurde nun auf pH >13 gestellt und mit Methylenchlorid extrahiert. Man erhielt so das Produkt (0.085 g) mit dem Molekulargewicht 547 g / mol (C₂₈H₃₈FN₃O₅S), MS (ESI): (M+H+) 548 g / mol.

Auf gleiche Weise erhielt man folgende Produkte:
(S)-3-(2-Fluor-phenyl)-3-(5-{4-[1-(3-hydroxy-propyl)-piperidin-4-yl]-benzyl}-6,6-dimethyl-4,4-dioxo-5,6-dihydro-4H-4lambda6-[1,4,3]oxathiazin-2-ylamino)-propan-1-ol 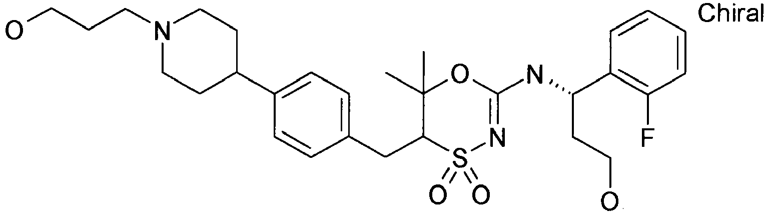
(S)-3-(6,6-Dimethyl-4,4-dioxo-5-{4-[1-(3,3,3-trifluoro-propyl)-piperidin-4-yl]-benzyl}-5,6-dihydro-4H-4lambda6-[1,4,3]oxathiazin-2-ylamino)-3-(2-fluor-phenyl)-propan-1-ol 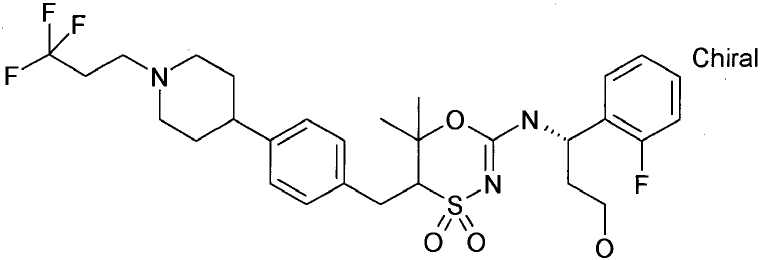
(S)-3-{5-[4-(1-Cyclopropylmethyl-piperidin-4-yl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol 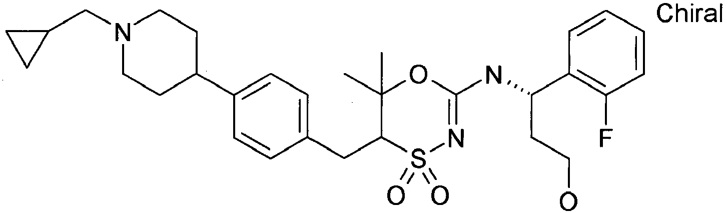
[3-((4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-prop-2-ynyl]-carbaminsäuretertiärbutylester

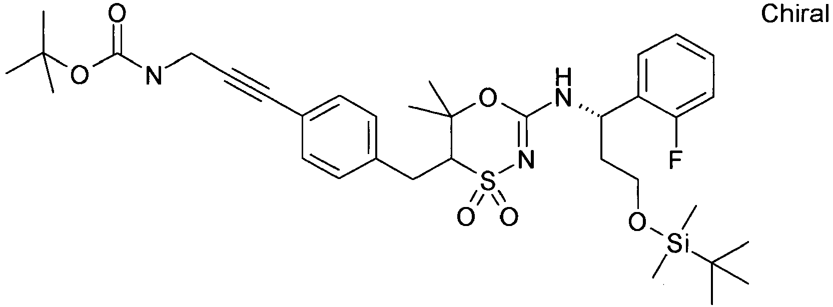

Zu einer Suspension von Bis(triphenylphoshin)palladium(II) dichlorid (10 mg) und Kupfer(I)-iodid (5 mg) in trockenem Dioxan (0.6 mL) wurden nacheinander Triethylamin (120 µL) und eine Lösung von [5-(4-Brom-benzyl)-6,6-dimethyl-4,4-dioxo-5,6-dihydro-4H-4lambda6-[1,4,3]oxathiazin-2-yl]-[(S)-3-(tert-butyl-dimethyl-silanyloxy)-1-(2-fluor-phenyl)-propyl]-amin (100 mg) in trockenem Dioxan (0.7 mL) gegeben und die Mischung wurde für 3 h bei 80 °C und 1 h bei 100 °C gerührt. Anschließend wurde mit Ethylacetat versetzt und mit Wasser gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Man erhielt so das Produkt (79 mg) mit dem Molekulargewicht 687.9 g / mol (C₃₅H₅₀FN₃O₆SSi).

### [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-propyl]-carbaminsäuretertiärbutylester

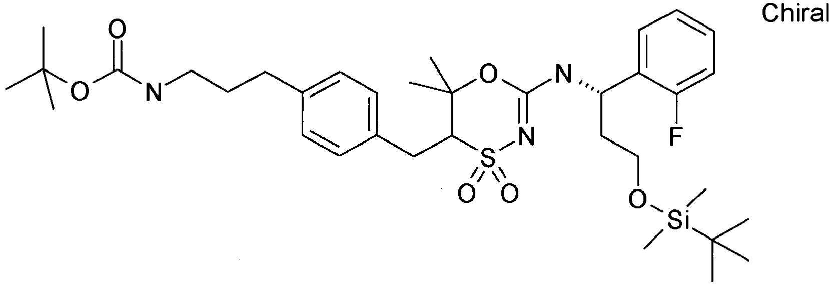

Zu einer Lösung von [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-prop-2-ynyl]-carbaminsäuretertiärbutylester (79 mg) in Methanol (25 mL) wurde Palaldium auf Kohle gegeben (5%, 25 mg) und die Lösung wurde in einer Wasserstoffatmosphäre für 1 h gerührt. Das Gemisch wurde filtriert und das Filtrat wurde eingeengt. Anschließend wurde säulenchromatographisch (Ethylacetat / Heptan) gereinigt. Man erhielt so das Produkt (45 mg) mit dem Molekulargewicht 692.0 g / mol (C₃₅H₅₄FN₃O₆SSi).

### (S)-3-{5-[4-(3-Amino-propyl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol

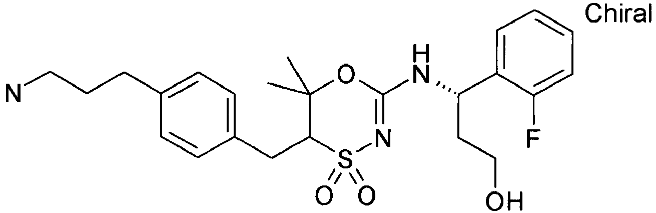

Zu einer Lösung von [3-(4-{2-[(S)-3-(tert-Butyl-dimethyl-silanyloxy)-1-(2-fluor-phenyl)-propylamino]-6,6-dimethyl-4,4-dioxo-5,6-dihydro-4H-4lambda6-[1,4,3]oxathiazin-5-ylmethyl}-phenyl)-propyl]-carbaminsäuretertiärbutylester (45 mg) in Methanol (2 mL) wurde konzentrierte Salzsäure (0.2 mL) gegeben und die Mischung wurde für 2 Tage gerührt. Die entstandene Mischung wurde im Reinigungslabor über präparative HPLC gereinigt. Man erhielt so das Produkt (5 mg als HCl Salz nach Behandeln mit Salzsäure) mit dem Molekulargewicht 477.6 g / mol (C₂₃H₃₂FN₃O₄S); MS (ESI): m / e = 478 (M+H⁺).

Analog wurden folgende Verbindungen erhalten:
(S)-3-{5-[4-(3-Dimethylamino-propyl)-benzyl]-6,6-dimethyl-4,4-dioxo-5,6-dihydro-4H-4lambda6-[1,4,3]oxathiazin-2-ylamino}-3-(2-fluor-phenyl)-propan-1-ol 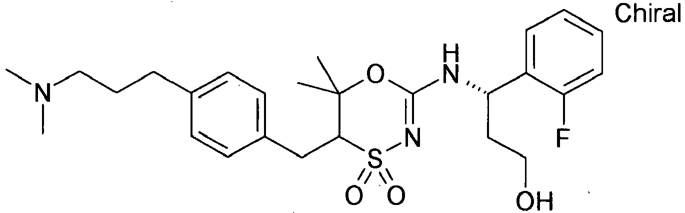
(S)-3-{6,6-Dimethyl-5-[4-(3-morpholin-4-yl-propyl)-benzyl]-4,4-dioxo-5,6-dihydro-4H-4lambda6-[1,4,3]oxathiazin-2-ylamino }-3-(2-fluor-phenyl)-propan-1-ol 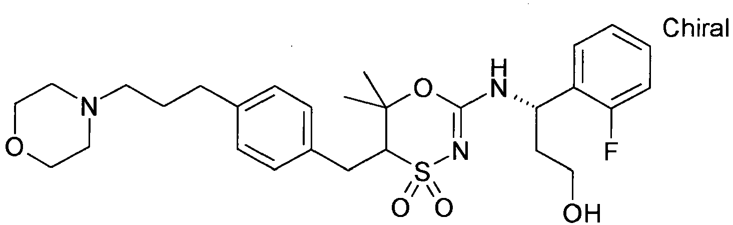
(S)-3-{6,6-Dimethyl-5-[4-(3-methylamino-propyl)-benzyl]-4,4-dioxo-5,6-dihydro-4H-4lambda6-[1,4,3]oxathiazin-2-ylamino}-3-(2-fluor-phenyl)-propan-1-ol 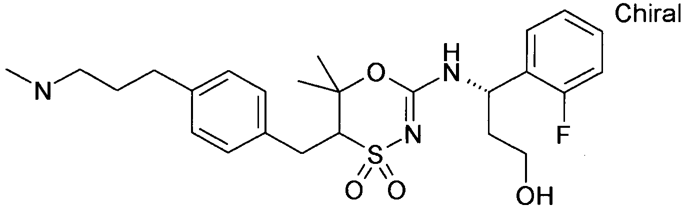
(S)-3-(2-Fluor-phenyl)-3-[5-(4-{3-[(2-hydroxy-ethyl)-methyl-amino]-propyl}-benzyl)-6,6-dimethyl-4,4-dioxo-5,6-dihydro-4H-4lambda6-[1,4,3]oxathiazin-2-ylamino]-propan-1-ol

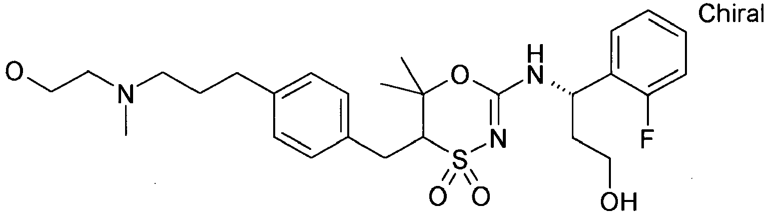

Eine Lösung von (S)-3-{6,6-Dimethyl-5-[4-(3-methylamino-propyl)-benzyl]-4,4-dioxo-5,6-dihydro-4H-4lambda6-[1,4,3]oxathiazin-2-ylamino]-3-(2-fluor-phenyl)-propan-1-ol (170 mg) und 2-Bromethanol (51 mg) in DMF (2.5 mL) wurde bei 50 °C für 8 h gerührt. Die entstandene Mischung wurde im Reinigungslabor über präparative HPLC gereinigt. Man erhielt so das Produkt (73 mg als HCl Salz nach Behandeln mit Salzsäure) mit dem Molekulargewicht 535.7 g / mol (C₂₇H₃₈FN₃O₅S); MS (ESI): m / e = 536 (M+H⁺).

Analog wurden folgende Verbindungen erhalten:
(S)-3-(2-Fluor-phenyl)-3-(5-{4-[1-(2-methoxy-ethyl)-piperidin-4-yl]-benzyl}-6,6-dimethyl-4,4-dioxo-5,6-dihydro-4H-4lambda6-[1,4,3]oxathiazin-2-ylamino)-propan-1-ol 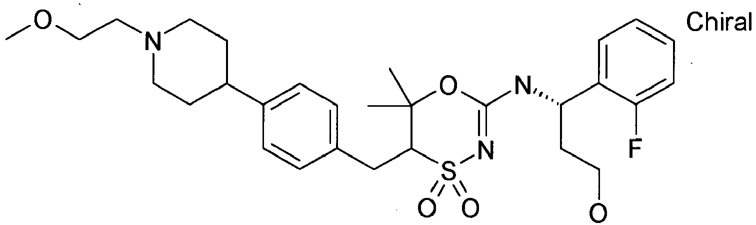
{6,6-Dimethyl-4,4-dioxo-5-[4-(4,4,5,5-tetramethyl-[1,3,2]dioxaborolan-2-yl)-benzyl]-5,6-dihydro-4H-4lambda6-[1,4,3]oxathiazin-2-yl}-[(S)-1-(2-fluor-phenyl)-ethyl]-amin

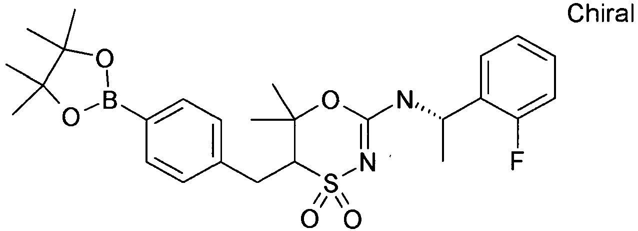

Zu einer Lösung von [5-(4-Brom-benzyl)-6,6-dimethyl-4,4-dioxo-5,6-dihydro-4H-4lambda6-[1,4,3]oxathiazin-2-yl]-[(S)-1-(2-fluor-phenyl)-ethyl]-amin (470 mg) in Dioxan wurde Pd(dppf)Cl₂ (67 mg), Bis(pinacolato)diboron (311 mg) und Kaliumacetat (241 mg) gegeben und die Mischung wurde 1 h bei 80 °C gerührt. Nach dem Verdünnen mit Ethylacetat wurde mit Wasser und Natriumchloridlösung gewaschen, über Kieselgur getrocknet und eingeengt. Anschließend wurde säulenchromatographisch (Ethylacetat / Heptan) gereinigt. Man erhielt so das Produkt (410 mg) mit dem Molekulargewicht 516.4 g / mol (C₂₆H₃₄BFN₂O₅S).

### {6,6-Dimethyl-5-[4-(4-methyl-piperazin-1-yl)-benzyl]-4,4-dioxo-5,6-dihydro-4H-4lambda6-[1,4,3]oxathiazin-2-yl}-[(S)-1-(2-fluor-phenyl)-ethyl]-amin

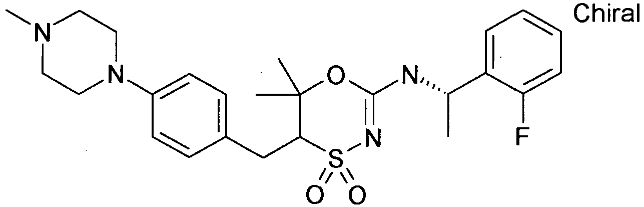

Zu einer Lösung von {6,6-Dimethyl-4,4-dioxo-5-[4-(4,4,5,5-tetramethyl-[1,3,2]dioxaborolan-2-yl)-benzyl]-5,6-dihydro-4H-4lambda6-[1,4,3]oxathiazin-2-yl}-[(S)-1-(2-fluor-phenyl)-ethyl]-amin (230 mg) in Pyridin wurden Kupfer(II)-acetat (76 mg) und Molekularsieb 0,4 nm gegeben und die Mischung wurde für 10 min gerührt. Anschließend wurde N-Methylpiperazin (63 mg) gegeben und die Mischung wurde bei 80 °C für 16 h gerührt. Nach dem Verdünnen mit Ethylacetat wurde mit Natriumbicarbonatlösung und Natriumchloridlösung gewaschen, über Magnsiumsulfat getrocknet und eingeengt. Der Rückstand wurde im Reinigungslabor über präparative HPLC gereinigt. Man erhielt so das Produkt (43 mg als HCl Salz nach Behandeln mit Salzsäure) mit dem Molekulargewicht 488.6 g / mol (C₂₅H₃₃FN₄O₃S); MS (ESI): m / e = 489 (M+H⁺).

Analog wurden folgende Verbindungen erhalten:
4-((4-{(R)-2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-phenyl)-piperazine-1-carbaminsäuretertiärbutylester 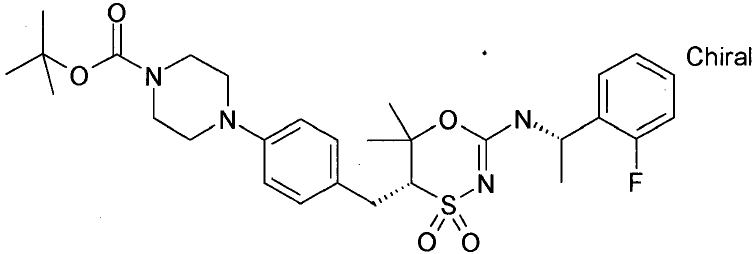
{(R)-5-[4-(4-tert-Butyl-piperazin-1-yl)-benzyl]-6,6-dimethyl-4,4-dioxo-5,6-dihydro-4H-4lambda6-[1,4,3]oxathiazin-2-yl}-[(S)-1-(2-fluor-phenyl)-ethyl]-amin 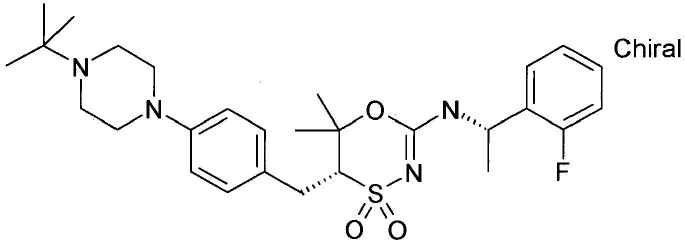
[2-((4-{(R)-2-[(S)-1-(2-Fluoro-phenyl)-ethylamino]-6,6-dimethyl-4,4-dioxo-5,6-dihydro-4H-4lambda6-[1,4,3]oxathiazin-5-ylmethyl}-phenoxy)-1,1-dimethyl-ethyl]-carbaminsäuretertiärbutylester 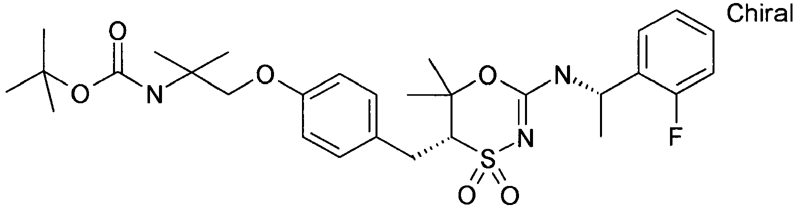
N-(4-{(R)-2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-phenyl)-N',N'-dimethyl-ethane-1,2-diamin 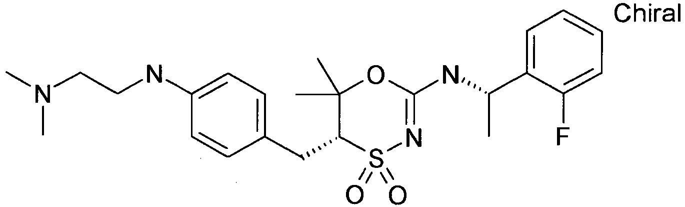
5-{2-[(S)-1-(2-Fluor-phenyl)-ethylamino]-6,6-dimethyl-4,4-dioxo-5,6-dihydro-4H-4lambda6-[1,4,3]oxathiazin-5-ylmethyl}-3',4',5',6'-tetrahydro-2'H-[2,4']bipyridinyl-1'-carbon säuremethyl ester

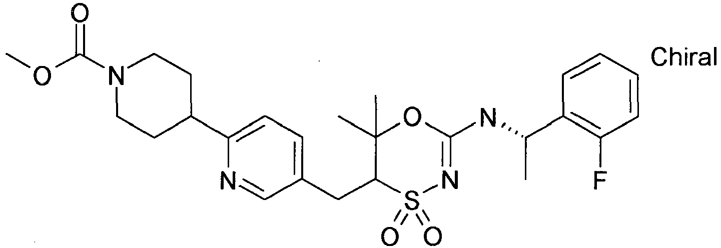

Zu [(S)-1-(2-Fluor-phenyl)-ethyl]-[5-(1',2',3',4',5',6'-hexahydro-[2,4']bipyridinyl-5-ylmethyl)-6,6-dimethyl-4,4-dioxo-5,6-dihydro-4H-4lambda6-[1,4,3]oxathiazin-2-yl]-amin (0,1 g) wurde Tetramethoxymethan (2 ml) zugegeben und auf 100 °C für 2 Stunden erhitzt. Nach dem Verdünnen mit Ethylacetat wurde mit Natriumbicarbonatlösung und Natriumchloridlösung gewaschen, über Magnsiumsulfat getrocknet und eingeengt. Der Rückstand wurde im Reinigungslabor über präparative HPLC gereinigt. Man erhielt so das Produkt (43 mg als HCl Salz nach Behandeln mit Salzsäure) mit dem Molekulargewicht 532.6 g / mol (C₂₆H₃₃FN₄O₅S); MS (ESI): m / e = 533 (M+H⁺).

## Patentansprüche

1. Verbindungen der Formel I, 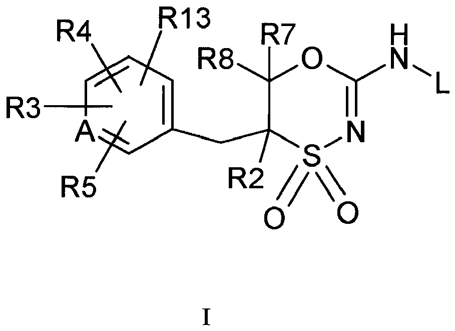 worin bedeuten
A CH, N;
L R1, -CH(R10)(R11);
R10, R11 unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₈)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R6 H, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
R1 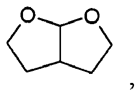 (C₆-C₁₀)-Aryl, (C₃-C₈)-Carbocyclyl, wobei der 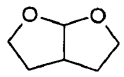 -rest, Arylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, F, (C₁-C₃)-Alkyl, wobei der Alkylrest ein bis 3-fach mit Fluor substituiert sein kann;
R3, R4, R5, R13 unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, -(C₁-C₆)-Alkylen-(C₆-C₁₀)-Aryl, ,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis 12-gliedriger Heterocyclus;
wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
oder R4 und R5 bilden gemeinsam eine -CH=CH-CH=CH- Kette.
R7, R8 unabhängig voneinander H, (C₁-C₃)-Alkyl einfach oder mehrfach mit Fluor substituiert, oder R7 und R8 bilden gemeinsam mit dem Kohlenstoffatom an das sie gebunden sind einen 3-8 gliedrigen Carbocyclus oder Heterocyclus;
R9 H, OH, OCH₃, OCF₃, CHF₂, CF₃;
sowie deren pharmazeutisch verträgliche Salze.

2. Verbindungen der Formel I, gemäß Anspruch 1, **dadurch gekennzeichnet, dass** darin bedeuten
A CH, N;
L R1, -CH(R10)(R11);
R10, R11 unabhängig voneinander H, F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₁-C₆)-Alkylen-(R6), (C₃-C₈)-Cycloalkylen-(R6), (C₁-C₆)-Alkylen-(C₃-C₈)-Cycloalkylen-(R6), (C₆-C₁₀)-Aryl, (C₁-C₆)-Alkylen-(C₆-C₁₀)-Aryl, -(C₆-C₁₀)-Heteroaryl, (C₁-C₆)-Alkylen-(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R6 H, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, O-(CO)-NH₂, SF₅;
R1 (C₆-C₁₀)-Aryl, (C₃-C₈)-Carbocyclyl, wobei der Arylrest oder Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, F, (C₁-C₃)-Alkyl, wobei der Alkylrest ein bis 3-fach mit Fluor substituiert sein kann;
R3, R4, R5, R13 unabhängig voneinander H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)_{2,} -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)_{2,} SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis 12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
oder R4 und R5 bilden gemeinsam eine -CH=CH-CH=CH- Kette;
R7, R8 unabhängig voneinander (C₁-C₃)-Alkyl;
R9 H, OH, OCH₃, OCF₃, CHF₂, CF₃;
sowie deren pharmazeutisch verträgliche Salze.

3. Verbindungen der Formel I, gemäß Anspruch 1 oder 2, **dadurch gekennzeichnet, dass** darin bedeuten
A CH, N;
L R1, -CH(R10)(R11);
R10, R11 unabhängig voneinander (C₁-C₆)-Alkyl, (C₁-C₆)-Alkylen-(R6), (C₆-C₁₀)-Aryl, -(C₆-C₁₀)-Heteroaryl;
wobei der Arylrest oder Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R6 H, OH, O-(CO)-NH₂, SO₂NH₂;
R1 (C₃-C₈)-Carbocyclyl,
wobei der Carbocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2 H, F, (C₁-C₃)-Alkyl, wobei der Alkylrest ein bis 3-fach mit Fluor substituiert sein kann;
R3 F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)_{2,} -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)_{2,}-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)_{2,} SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, 4 bis12-gliedriger Heterocyclus;
wobei der (C₆-C₁₀)-Arylrest, (C₃-C₈)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R4, R5, R13 unabhängig voneinander
H, F, Cl, Br, I, OH, CF₃, NO₂, CN, OCF₃, -(C₁-C₆)-Alkylen-(R9), O-(C₁-C₆)-Alkylen-(R9), NH₂;
R7, R8 unabhängig voneinander (C₁-C₃)-Alkyl;
R9 H, OH, OCH₃, OCF₃, CHF₂, CF₃;
sowie deren pharmazeutisch verträgliche Salze.

4. Verbindungen der Formel I, gemäß einem oder mehreren der Ansprüche 1 bis 3, **dadurch gekennzeichnet, dass** darin bedeuten
A CH, N;
L R1, -CH(R10)(R11);
R10 (C₁-C₆)-Alkyl, (C₁-C₆)-Alkylen-(R6);
R11 (C₆-C₁₀)-Aryl,
wobei der Arylrest oder Heteroarylrest ein bis 3-fach substituiert sein kann mit F, Cl, Br, (C₁-C₆)-Alkyl;
R6 OH;
R1 2,2,2-Bicyclooctyl, wobei der 2,2,2-Bicyclooctylrest ein bis 3-fach substituiert sein kann mit F, Cl, Br;
R2 H, (C₁-C₃)-Alkyl;
R3 F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Pyridin, Tetrahydropyridin, Piperidin, Morpholin, Piperazin,
wobei der Pyridin-, Tetrahydropyridin-, Piperidin-, Morpholin- oder Piperazinrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₈)-Cycloalkyl, -(C₁-C₆)-Alkylen-(C₃-C₈)-Cycloalkyl, 4 bis12-gliedriger Heterocyclus;
R4, R5, R13 unabhängig voneinander
H, F, Cl, Br, I, OH, CF₃, NO₂, CN, OCF₃, -(C₁-C₆)-Alkylen-(R9), O-(C₁-C₆)-Alkylen-(R9), NH₂;
R7, R8 unabhängig voneinander (C₁-C₃)-Alkyl;
R9 H, OH, OCH₃, OCF₃, CHF₂, CF₃;
sowie deren pharmazeutisch verträgliche Salze.

5. Verbindungen der Formel I, gemäß einem oder mehreren der Ansprüche 1 bis 3, **dadurch gekennzeichnet, dass** darin bedeuten
A CH, N;
L R1, -CH(R10)(R11);
R10 (C₁-C₆)-Alkyl, (C₁-C₆)-Alkylen-(R6);
R11 (C₆-C₁₀)-Aryl;
wobei der Arylrest oder Heteroarylrest ein bis 3-fach substituiert sein kann mit F, Cl, Br, (C₁-C₆)-Alkyl;
R6 OH;
R1 2,2,2-Bicyclooctyl,
wobei der 2,2,2-Bicyclooctylrest ein bis 3-fach substituiert sein kann mit F, Cl, Br;
R2 H, (C₁-C₃)-Alkyl;
R3 F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, CF₃;
Pyridin, Tetrahydropyridin, Piperidin, Morpholin, Piperazin, wobei der Pyridin-, Tetrahydropyridin-, Piperidin-, Morpholin- oder Piperazinrest ein bis 3-fach substituiert sein kann mit F, Cl, Br, CF₃, (C₁-C₆)-Alkylen-(R9), COO-(C₁-C₆)-Alkylen-(R9), (C₃-C₈)-Cycloalkyl, Oxetan;
R4, R5, R13 unabhängig voneinander
H, F, Cl, Br, I, OH, CF₃, NO₂, CN, OCF₃, -(C₁-C₆)-Alkylen-(R9), O-(C₁-C₆)-Alkylen-(R9), NH₂;
R7, R8 unabhängig voneinander (C₁-C₃)-Alkyl;
R9 H, OH, OCH₃, OCF₃, CHF₂, CF₃;
sowie deren pharmazeutisch verträgliche Salze.

6. Verbindungen der Formel I, gemäß einem oder mehreren der Ansprüche 1 bis 5, zur Anwendung als Arzneimittel.

7. Arzneimittel enthaltend Verbindungen der Formel I, gemäß einem oder mehreren der Ansprüche 1 bis 5.

8. Arzneimittel enthaltend Verbindungen der Formel I, gemäß einem oder mehreren der Ansprüche 1 bis 5 und mindestens einen weiteren Wirkstoff.

9. Arzneimittel, gemäß Anspruch 8, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten,, CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten , Lipase/Amylase-Inhibitoren, PPAR-Modulatoren, RXR-Modulatoren oder TR-β-Agonisten oder Amphetamine enthält.

10. Verfahren zur Herstellung eines Arzneimittels enthaltend die Verbindungen der Formel I, gemäß einem oder mehreren der Ansprüche 1 bis 5, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen der Formel I, gemäß einem oder mehreren der Ansprüche 1 bis 5 zur Herstellung eines Medikaments zur Behandlung von Hyperglykämie.

12. Verwendung der Verbindungen der Formel I, gemäß einem oder mehreren der Ansprüche 1 bis 5 zur Herstellung eines Medikaments zur Behandlung des Diabetes.

13. Verwendung der Verbindungen der Formel I, gemäß einem oder mehreren der Ansprüche 1 bis 5 zur Herstellung eines Medikaments zur Behandlung von Insulin Resistenz.

14. Set (Kit) bestehend aus getrennten Packungen von
a) einer wirksamen Menge einer Verbindung der Formel I, gemäß einem oder mehreren der Ansprüche 1 bis 5 und
b) einer wirksamen Menge eines weiteren Arzneimittelswirkstoffs.

## Claims

1. Compounds of the formula I 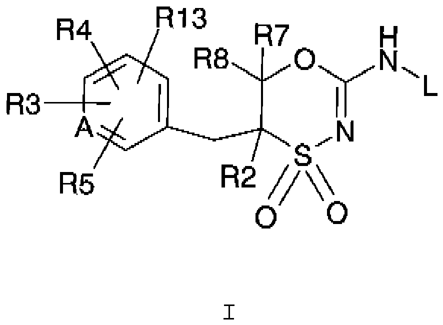 in which
A is CH, N;
L is R1, -CH(R10)(R11);
R10, R11 are each independently H, F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₁-C₆)-alkylene-(R6), (C₃-C₈)-cycloalkylene-(R6), (C₁-C₆)-alkylene-(C₃-C₈)-cycloalkylene-(R6), (C₆-C₈)-aryl, (C₁-C₆)-alkylene-(C₆-C₁₀)-aryl, - (C₆-C₁₀)-heteroaryl, (C₁-C₆)-alkylene-(C₆-C₁₀)-heteroaryl;
where the aryl radical or heteroaryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆) -alkyl, CON((C₁-C₆)-alkyl)₂, SF₅;
R6 is H, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₂-C₆)-alkyl)₂, COOH, COO-(C₁-C₆)-alkyl, CONH₂, CONH(C₁-C₆)-alkyl, CON((C₁-C₆)-alkyl)₂, O-(CO)-NH₂, SF₅;
R1 is 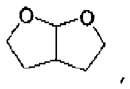 (C₆-C₁₀)-aryl, (C₃-C₈)-carbocyclyl, where the 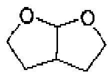 radical, aryl radical or carbocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2 is H, F, (C₁-C₃)-alkyl, where the alkyl radical may be mono- to trisubstituted by fluorine;
R3, R4, R5, R13 are each independently H, F, Cl, Br, I, OH, CF₃, CHF₂, CH₂F, NO₂, CN, OCF₃, OCHF₂, -(C₁-C₆)-alkylene-(R9), O-(C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9)₂, (C₁-C₆)-alkylene-NH₂, (C₁-C₆)-alkylene-NH(C₁-C₆)-alkylene-(R9), (C₁-C₆)-alkylene-N((C₁-C₆)-alkylene-R9)₂, -O-(C₁-C₆)-alkylene-NH₂, -O-(C₁-C₆)-alkylene-NH(C₁-C₆)-alkylene-(R9), -O-(C₁-C₆)-alkylene-N((C₁-C₆)-alkylene-R9)₂, -NH-(C₁-C₆)-alkylene-NH₂, - NH-(C₁-C₆)-alkylene-NH(C₁-C₆)-alkylene-(R9),-NH-(C₁-C₆)-alkylene-N((C₁-C₆)-alkylene-R9)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
(C₆-C₁₀)-aryl, -(C₁-C₆)-alkylene-(C₆-C₁₀)-aryl, where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₈)-cycloalkyl, 4 to 12-membered heterocycle;
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
4 to 12-membered heterocycle, -(C₁-C₆)-alkylene-4 to 12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO-(C₁-C₆)-alkylene- (R9), CONH₂, CONH(C₁-C₆)-alkylene-(R9), CON((C₁-C₆)-alkyl)₂, SF₅, (C₆-C₁₀)-aryl, (C₃-C₈)-cycloalkyl, 4 to 12-membered heterocycle;
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₃, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
or R4 and R5 together form a -CH=CH-CH=CH- chain;
R7, R8 are each independently H, mono- or poly-fluorine-substituted (C₁-C₃)-alkyl, or R7 and R8 together with the carbon atom to which they are bonded form a 3-8-membered carbocycle or heterocycle;
R9 is H, OH, OCH₃, OCF₃, CHF₂, CF₃;
and pharmaceutically acceptable salts thereof.

2. Compounds of the formula I according to Claim 1, **characterized in that**
A is CH, N;
L is R1, -CH(R10)(R11);
R10, R11 are each independently H, F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₁-C₆)-alkylene-(R6), (C₃-C₈)-cycloalkylene-(R6), (C₁-C₆)-alkylene-(C₃-C₈)-cycloalkylene-(R6), (C₆-C₁₀)-aryl, (C₁-C₆)-alkylene-(C₆-C₁₀)-aryl, -(C₆-C₁₀)-heteroaryl, (C₁-C₆)-alkylene-(C₆-C₁₀)-heteroaryl; where the aryl radical or heteroaryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R6 is H, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, O-(CO)-NH₂, SFs;
R1 is (C₆-C₁₀)-aryl, (C₃-C₈)-carbocyclyl, where the aryl radical or carbocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2 is H, F, (C₁-C₃)-alkyl, where the alkyl radical may be mono- to trisubstituted by fluorine;
R3, R4, R5, R13 are each independently H, F, Cl, Br, I, OH, CF₃, CHF₂, CH₂F, NO₂, CN, OCF₃, OCHF₂, -(C₁-C₆)-alkylene-(R9), O-(C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9)₂, (C₁-C₆)-alkylene-NH₂, (C₁-C₆)-alkylene-NH(C₁-C₆)-alkylene-(R9), (C₁-C₆)-alkylene-N((C₁-C₆)-alkylene-R9)₂, -O-(C₁-C₆)-alkylene-NH₂, -O-(C₁-C₆) -alkylene-NH(C₁-C₆)-alkylene-(R9), -0- (C₁-C₆)-alkylene-N((C₁-C₆)-alkylene-R9)₂, -NH- (C₁-C₆)-alkylene-NH₂, -NH-(C₁-C₆)-alkylene-NH(C₁-C₆)-alkylene-(R9), -NH-(C₁-C₆)-alkylene-N((C₁-C₆)-alkylene-R9)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
phenyl,
where the phenyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₈)-cycloalkyl, 4 to 12-membered heterocycle;
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₃-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
4 to 12-membered heterocycle, -(C₁-C₆)-alkylene-4 to 12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene-(R9), N((C₁-C₆-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅, (C₆-C₁₀)-aryl, (C₃-C₈)-cycloalkyl, 4 to 12-membered heterocycle;
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
or R4 and R5 together form a -CH=CH-CH=CH- chain;
R7, R8 are each independently (C₁-C₃)-alkyl;
R9 is H, OH, OCH₃, OCF₃, CHF₂, CF₃;
and pharmaceutically acceptable salts thereof.

3. Compounds of the formula I according to Claim 1 or 2, **characterized in that**
A is CH, N;
L is R1, -CH(R10)(R11);
R10, R11 are each independently (C₁-C₆)-alkyl, (C₁-C₆) -alkylene- (R6), (C₆-C₁₀)-aryl, -(C₆-C₁₀)-heteroaryl;
where the aryl radical or heteroaryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R6 is H, OH, O-(CO)-NH₂, SO₂NH₂;
R1 is (C₃-C₈)-carbocyclyl,
where the carbocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₄)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₃, SF₅;
R2 is H, F, (C₁-C₃)-alkyl, where the alkyl radical may be mono- to trisubstituted by fluorine;
R3 is F, Cl, Br, I, OH, CF₃, CHF₂, CH₂F, NO₂, CN, OCF₃, OCHF₂, -(C₁-C₆)-alkylene-(R9), O-(C₁-C₆)-alkylene-(R9), tert-butyl, isopropylene-(R9), (C=O)-(C₁-C₆)-alkylène- (R9), (C₁-C₆)-alkylene-(R9), NH₂, NH(C₁-C₆)-alkylene-(R9), N(((C₁-C₆)--alkylene-R9)₂, (C₁-C₆)-alkylene-NH₂, (C₁-C₆) -alkylene-NH (C₁-C₆)-alkylene- (R9), (C₁-C₆)-alkylene-N((C₁-C₆)-alkylene-R9)₂. -O- (C₁-C₆) -alkylene-NH₂, -0- (C₁-C₆) -alkylene-NH(C₁-C₆)-alkylene-(R9), -O-(C₁-C₆)-alkylene-N ((C₁-C₆)-alkylene-R9)₂, -NH-(C₁-C₆)-alkylene-NH₂,-NH-(C₁-C₆)-alkylene-NH(C₁-C₆)-alkylene-(R9), -NH- (C₁-C₆)-alkylene-N((C₁-C₆)-alkylene-R9)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
phenyl,
where the phenyl radical may be mono-to trisubstituted by F, Cl, Br, I, OH, CF₃, CHF₂, CH₂F, NO₂, CN, OCF₃, OCHF₂, O- (C₁-C₆)-alkyl, (C₁-C₆)-alkyle , NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₈)-cycloalkyl, 4 to 12-membered heterocycle;
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₃-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
4 to 12-membered heterocycle, -(C₁-C₆)-alkylene-4 to 12-membered heterocycle,
where the heterocyclyl radical may be mono- to trisubstituted by F, Cl, Br, I, OH, CF₃, CHF₂, CH₂F, NO₂, CN, OCF₃, OCHF_{2,} O-(C₁-C₆)-alkylene-(R9), (C₁-C₆)-alkylene-(R9), NH₂, NH(C₁-C₆)-alkylene-(R9), N((C₁-C₆) -alkyl)₂, SO₂-(C₁-C₆) -alkylene-(R9), SO₂-C₂H₂F₃, SO₂-CH₃, SO₂-NH₂,SO₂-NH(C₁-C₆)-alkylene-(R9), SO₂-N((C₁-C₆)-alkyl)₂, COOH, COO-(C₁-C₆) -alkylene- (R9), CONH₂, CONH(C₁-C₆) -alkylene-(R9), CON (C₁-C₆)-alkyl)₂, SF₅, (C₆-C₁₀)-aryl, (C₃-C₈)-cycloalkyl, 4 to 12-membered heterocycle;
R4, R5, R13
where the (C₆-C₁₀)-aryl radical, (C₃-C₈)-cycloalkyl radical, 4 to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl) ₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; are each independently
H, F, Cl, Br, I, OH, CF₃, NO₂, CN, OCF₃, -(C₁-C₆)-alkylene- (R9), 0- (C₁-C₆)-alkylene-(R9), NH₂;
R7, R8 are each independently (C₁-C₃)-alkyl;
R9 is H, OH, OCH₃, OCF₃, CHF₂, CF₃;
and pharmaceutically acceptable salts thereof.

4. Compounds of the formula I according to one or more of Claims 1 to 3, **characterized in that**
A is CH, N;
L is R1, -CH(R10)(R11);
R10 is (C₁-C₆)-alkyl, (C₁-C₆)-alkylene-(R6) ;
R11 is (C₆-C₁₀) -aryl;
where the aryl radical or heteroaryl radical may be mono- to trisubstituted by F, Cl, Br, (C₁-C₆)-alkyl;
R6 is OH;
R1 is 2,2,2-bicyclooctyl,
where the 2,2,2-bicyclooctyl radical may be mono- to trisubstituted by F, Cl, Br;
R2 is H, (C₁-C₃)-alkyl;
R3 is F, Cl, B, I, OH, CF₃, CHF₂, CH₂F, NO₂, CN, OCF₃, OCHF₂, -(C₁-C₆)-alkylene-(R9), O-(C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9)₂, (C₁-C₆)-alkylene-NH₂, (C₁-C₆)-alkylene-NH(C₁-C₆)-alkylene-(R9), (C₁-C₆)-alkylene-N((C₁-C₆)-alkylene-R9)₂, -O-(C₁-C₆)-alkylene-NH₂, -O-(C₁-C₆)-alkylene-NH(C₁-C₆)-alkylene-(R9), -O-(C₁-C₆)-alkylene-N((C₁-C₆)-alkylene-R9)₂, -NH-(C₁-C₆) -alkylene-NH₂, -NH-(C₁-C₆)-alkylene-NH(C₁-C₆) -alkylene- (R9), -NH- (C₁-C₆)-alkylene-N((C₁-C₆)-alkylene-R9)₂, SO₂-CH_{3,} SO₂-NH₂, SO₂-NH(C₁-C₆) -alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
phenyl,
where the phenyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆) -alkyl, SO₂*-*N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
pyridine, tetrahydropyridine, piperidine, morpholine, piperazine,
where the pyridine, tetrahydropyridine, piperidine, morpholine or piperazine radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene-(R9), N((C₁-C₆)-alkyl)₃, SO₂-(C₁-C₆)-alkylene-(R9), SO₂-C₂H₂F₃, SO₂-CH₃, SO₂-NH₂, SO₂-NH(C₁-C₆) -alkylene-(R9), SO₂-N((C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅, (C₆-C₁₀)-aryl, (C₃-C₈)-cycloalkyl, -(C₁-C₆)-alkylene-(C₃-C₈)-cycloalkyl, 4 to 12-membered heterocycle;
R4, R5, R13 are each independently
H, F, Cl, Br, I, OH, CF₃, NO₂, CN, OCF₃, - (C₁-C₆)-alkylene-(R9), 0- (C₁-C₆)-alkylene-(R9), NH₂;
R7, R8 are each independently (C₁-C₃)-alkyl;
R9 is H, OH, OCH₃, OCF₃, CHF₂, CF₃;
and pharmaceutically acceptable salts thereof.

5. Compounds of the formula I according to one or more of Claims 1 to 3, **characterized in that**
A is CH, N;
L is R1, -CH(R10)(R11);
R10 is (C₁-C₆)-alkyl, (C₁-C₆-alkylene-(R6);
R11 is (C₆-C₁₀)-aryl,
where the aryl radical or heteroaryl radical may be mono-to trisubstituted by F, Cl, Br. (C₁-C₆)-alkyl;
R6 is OH;
R1 is 2,2,2-bicyclooctyl,
where the 2,2,2-bicyclooctyl radical may be mono- to trisubstituted by F, Cl, Br;
R2 is H, (C₁-C₃)-alkyl;
R3 is F, Cl, Br, I, OH, CF₃, CHF₂, CH₂F, NO₂, CN, OCHF₃, OCHF₂, -(C₁-C₆)-alkylene-(R9), O-(C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9)₂, (C₁-C₆)-alkylene-NH₂, (C₁-C₆) -alkylene-NH(C₁-C₆)-alkylene- (R9), (C₁-C₆)-alkylene-N((C₁-C₆)alkylene-R9)₂, -0-(C₁-C₆)-alkylene-NH₂, -O-(C₁-C₆)-alkylène-NH(C₁-C₆)-alkylene-(R9), -O-(C₁-C₆)-alkylene-N((C₁-C₆)-alkylene-R9)₂, -NH-(C₁-C₆)-alkylene-NH₂, -NH- (C₁-C₆)-alkylene-NH(C₁-C₆)-alkylène-(R9), -NH-(C₁-C₆)-alkylene-N((C₁-C₆)-alkylene-R9)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
phenyl, where the phenyl radical may be mono- to trisubstituted by F, Cl, Br, CF₃;
pyridine, tetrahydropyridine, piperidine, morpholine, piperazine,
where the pyridine, tetrahydropyridine, piperidine, morpholine or piperazine radical may be mono- to trisubstituted by F, Cl, Br, CF₃, (C₁-C₆)-alkylene-(R9), COO-(C₁-C₆)-alkylene-(R9), (C₃-C₈)-cycloalkyl, oxetane;
R4, R5, R13 are each independently H, F, Cl, Br, I, OH, CF₃, NO₂, CN, OCF₃, - (C₁-C₆) -alkylene- (R9), O-(C₁-C₆)-alkylene-(R9), NH₂;
R7, R8 are each independently (C₁-C₃)-alkyl;
R9 is H, OH, OCH₃, OCF₃, CHF₂, CF₃;
and pharmaceutically acceptable salts thereof.

6. Compounds of the formula I according to one or more of Claims 1 to 5 for use as medicaments.

7. Medicament comprising compounds of the formula I according to one or more of Claims 1 to 5.

8. Medicament comprising compounds of the formula I according to one or more of Claims 1 to 5 and at least one further active ingredient.

9. Medicament according to Claim 8, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR β agonists or amphetamines.

10. Process for producing a medicament comprising the compounds of the formula I according to one or more of Claims 1 to 5, **characterized in that** the active ingredient is mixed with a pharmaceutically suitable carrier and this mixture is converted to a form suitable for administration.

11. Use of the compounds of the formula I according to one or more of Claims 1 to 5 for producing a medicament for treatment of hyperglycemia.

12. Use of the compounds of the formula I according to one or more of Claims 1 to 5 for producing a medicament for treatment of diabetes.

13. Use of the compounds of the formula I according to one or more of Claims 1 to 5 for producing a medicament for treatment of insulin resistance.

14. Set (kit) consisting of separate packages of
a) an effective amount of a compound of the formula I according to one or more of Claims 1 to 5 and
b) an effective amount of a further active medicament ingredient.

## Revendications

1. Composés de formule I 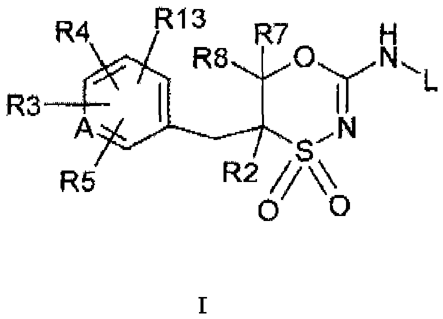 dans laquelle
A signifie CH, N ;
L signifie R1, -CH(R10)(R11);
R10, R11 signifient indépendamment l'un de l'autre H, F, Cl, Br, I, OH, CF₃, OCHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, COOH, COO-alkyle en (C₁-C₆), CONH₂, CONH-alkyle en (C₁-C₆), CON(alkyle en (C₁-C₆)₂, SF₅, alkylène en (C₁-C₆)-(R6), cycloalkylène en (C₃-C₈)-(R6), alkylène en (C₁-C₆)-cycloalkylène en (C₃-C₈)-(R6), aryle en (C₆-C₈), alkylène en (C₁-C₆)-aryle en (C₆-C₁₀), hétéroaryle en (C₆-C₁₀), alkylène en (C₁-C₆)-hétéroaryle en (C₆-C₁₀);
le radical aryle ou le radical hétéroaryle pouvant être substitué une à 3 fois avec F, Cl, Br, I, OH, CF₃, CHF₂, CH₂F, NO₂, CN, OCF₃, OCHF₂, O-(alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R6 signifie H, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆)*,* CON(alkyle en (C₁-C₆))₂, O-(CO)-NH₂, SF₅ :
R1 signifie 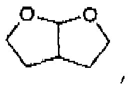 aryle en (C₆-C₁₀), carbocyclyle en (C₃-C₈),
le radical 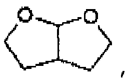 le radical aryle ou le radical carbocyclyle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON (alkyle en (C₁-C₆))₂, SF₅ ;
R2 signifie H, F, alkyle en (C₁-C₃), le radical alkyle pouvant être substitué une à 3 fois avec fluor ;
R3, R4, R5, R13 signifient indépendamment les uns des autres
H, F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkylène en (C₁-C₆)-R9)₂, -NH-alkylène en (C₁-C₆)-NH₂, -NH-alkylène en (C₁-C₆)-NH-alkylène en (C₁-C₆)-(R9), -NH-alkylène en (C1.-C₆) -N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SP₅;
aryle en (C₆-C₁₀), alkylène en (C₁-C₆)-aryle en (C₆-C₁₀),
le radical aryle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆)))₂, SF₅, aryle en (C₆-C₁₀), cycloalkyle en (C₃-C₈), hétérocycle de 4 à 12 éléments ;
le radical aryle en (C₆-C₁₀), le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
hétérocycle de 4 à 12 éléments, alkylène en (C₁-C₆)-hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N (alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅, aryle en (C₆-C₁₀), cycloalkyle en (C₃-C₈), hétérocycle de 4 à 12 éléments ;
le radical aryle en (C₆-C₁₀), le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₃-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
ou R4 et R5 forment ensemble une chaîne -CH=CH-CH=CH-, R7, R8 signifient indépendamment l'un de l'autre H, alkyle en (C₁-C₃) substitué une ou plusieurs fois avec fluor, ou R7 et R8 forment ensemble avec l'atome de carbone auquel ils sont reliés un carbocycle ou hétérocycle de 3 à 8 éléments ;
R9 signifie H, OH, OCH₃, OCF₃, CHF₂, CF₃;
ainsi que leurs sels pharmaceutiquement compatibles.

2. Composés de formule **I** selon la revendication 1,
**caractérisés en ce que**
A signifie CH, N ;
L signifie R1, -CH(R10)(R11) ;
R10, R11 signifient indépendamment l'un de l'autre H, F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, COOH, COO-alkyle en (C₁-C₆), CONH₂, CONH-alkyle en (C₁-C₆) CON (alkyle en (C₁-C₆))₂, SF₅, alkylène en (C₁-C₆)-(R₆), cycloalkylène en (C₃-C₈)-(R6), alkylène en (C₁-C₆)-cycloalkylène en (C₃-C₈)-(R6), aryle en (C₆-C₁₀), alkylène en (C₁-C₆)-aryle en (C₆-C₁₀), hétéroaryle en (C₆-C₁₀), alkylène en (C₁-C₆) - hétéroaryle en (C₆-C₁₀) ;
le radical aryle ou le radical hétéroaryle pouvant être substitué une à 3 fois avec F, Cl, Br, I, OH, CF₃, CHF_{2,} CH₂F, NO₂, CN, OCF₃, OCHF₂, O-alkyle en (C₁-C₆), alkyle en (C₁-C₆), NH₂, NH-alkyle en (C₁-C₆) , N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R6 signifie H, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, O-(CO)-NH₂, SF₅ ;
R1 signifie aryle en (C₆-C₁₀), carbocyclyle en (C₃-C₈),
le radical aryle ou le radical carbocyclyle pouvant être substitué une à 3 fois avec F, Cl, Br, I, OH, CF₃, CHF₂, CH₂F, NO₂, CN, OCF₃ , OCHF₂ O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R2 signifie H, F, alkyle en (C₁-C₃), le radical alkyle pouvant être substitué une à 3 fois avec fluor ;
R3, R4, R5, R13 signifient indépendamment les uns des autres
H, F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkylène en (C₁-C₆)-R9)₂, -NH-alkylène en (C₁-C₆)-NH₂, -NH-alkylène en (C₁-C₆)-NH-alkylène en (C₁-C₆))-(R9), -NH-alkylène en (C₁-C₆)-N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
phényle,
le radical phényle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆, NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅, aryle en (C₆-C₁₀), cycloalkyle en (C₃-C₈), hétérocycle de 4 à 12 éléments ;
le radical aryle en (C₆-C₁₀), le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
hétérocycle de 4 à 12 éléments, alkylène en (C₁-C₆)-hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆) - (R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂₋N(alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅, aryle en (C₆-C₁₀), cycloalkyle en (C₃-C₈), hétérocycle de 4 à 12 éléments ;
le radical aryle en (C₆-C₁₀), le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
ou R4 et R5 forment ensemble une chaîne -CH=CH-CH=CH-, R7, R8 signifient indépendamment l'un de l'autre alkyle en (C₁-C₃) ;
R9 signifie H, OH, OCH₃, OCF₃, CHF₂, CF₃ ;
ainsi que leurs sels pharmaceutiquement compatibles.

3. Composés de formule I selon la revendication 1 ou 2, **caractérisés en ce que**
A signifie CH, N ;
L signifie R1, -CH(R10)(R11) ;
R10, R11 signifient indépendamment l'un de l'autre alkyle en (C₁-C₆), alkylène en (C₁-C₆)-(R6), aryle en (C₆-C₁₀), hétéroaryle en (C₆-C₁₀) ;
le radical aryle ou le radical hétéroaryle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆) , SO₂-N(alkyle en (C₁-C₆) )₂, COOH, COO-alkyle en (C₁-C₆), CONH₂, CONH-alkyle en (C₁-C₆), CON(alkyle en (C₁-C₆))₂, SF₅ ;
R6 signifie H, OH, O-(CO)-NH₂, SO₂NH₂ ;
R1 signifie carbocyclyle en (C₃-C₈),
le radical carbocyclyle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆) SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R2 signifie H, F, alkyle en (C₁-C₃), le radical alkyle pouvant être substitué une à 3 fois avec fluor ;
R3 signifie F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆), N(alkylène en (C₁-C₆)-R9)₂, -NH-alkylène en (C₁-C₆)-NH₂, -NH-alkylène en (C₁-C₆)-NH-alkylène en (C₁-C₆)-(R9), -NH-alkylène en (C₁-C₆)-N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₂-C₆), CONH₂, CONH-alkyle en (C₁-C₆), CON(alkyle en (C₁-C₆))₂, SF₅ ;
phényle,
le radical phényle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅, aryle en (C₆-C₁₀), cycloalkyle en (C₃-C₈), hétérocycle de 4 à 12 éléments ;
le radical aryle en (C₆-C₁₀), le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
hétérocycle de 4 à 12 éléments, alkylène en (C₁-C₆)-hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON (alkyle en (C₁-C₆))₂, SF₅, aryle en (C₆-C₁₀), cycloalkyle en (C₃-C₈), hétérocycle de 4 à 12 éléments ;
le radical aryle en (C₆-C₁₀) , le radical cycloalkyle en (C₃-C₈), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R4, R5, R13 signifient indépendamment les uns des autres
H, F, Cl, Br, I, OH, CF₃, NO₂, CN, OCF₃, alkylène en (C₁-C₆)-(R9), O-alkylène en (C₁-C₆)-(R9), NH₂ ;
R7, R8 signifient indépendamment l'un de l'autre alkyle en (C₁-C₃);
R9 signifie H, OH, OCH₃, OCF₃, CHF₂, CF₃ ;
ainsi que leurs sels pharmaceutiquement compatibles.

4. Composés de formule I selon une ou plusieurs des revendications 1 à 3, **caractérisés en ce que**
A signifie CH, N ;
L signifie R1, -CH(R10)(R11) ;
R10 signifie alkyle en (C₁-C₆), alkylène en (C₁-C₆)-(R6) ;
R11 signifie aryle en (C₆-C₁₀) ;
le radical aryle ou le radical hétéroaryle pouvant être substitué une à 3 fois avec F, Cl, Br, alkyle en (C₁-C₆) ;
R6 signifie OH ;
R1 signifie 2,2,2-bicyclooctyle,
le radical 2,2,2-bicyclooctyle pouvant être substitué une à 3 fois avec F, Cl, Br ;
R2 signifie H, alkyle en (C₁-C₃) ;
R3 signifie F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkyléne en (C₁-C₆)-R9)₂, -NH-alkylène en (C₁-C₆)-NH₂, -NH-alkylène en (C₁-C₆)-NH-alkylène en (C₁-C₆)-(R9), -NH-alkylène en (C₁-C₆)-N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON (alkyle en (C₁-C₆))₂, SF₅ ;
phényle,
le radical phényle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
pyridine, tétrahydropyridine, pipéridine, morpholine, pipérazine,
le radical pyridine, tétrahydropyridine, pipéridine, morpholine ou pipérazine pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆), (R9), N (alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9) , CON(alkyle en (C₁-C₆))₂, SF₅, aryle en (C₆-C₁₀), cycloalkyle en (C₃-C₈), alkylène en (C₁-C₆)-cycloalkyle en (C₃-C₈), hétérocycle de 4 à 12 éléments ;
R4, R5, R13 signifient indépendamment les uns des autres
H, F, Cl, Br, I, OH, CF₃, NO₂, CN, OCF₃, alkylène en (C₁-C₆)-(R9), O-alkylène en (C₁-C₆)-(R9), NH₂ ;
R7, R8 signifient indépendamment l'un de l'autre alkyle en (C₁-C₃) ;
R9 signifie H, OH, OCH₃, OCF₃, CHF₂, CF₃ ;
ainsi que leurs sels pharmaceutiquement compatibles.

5. Composés de formule I selon une ou plusieurs des revendications 1 à 3, **caractérisés en ce que** A signifie CH, N ;
L signifie R1, -CH(R10)(R11) ;
R10 signifie alkyle en (C₁-C₆), alkylène en (C₁-C₆)-(R6) ;
R11 signifie aryle en (C₆-C₁₀) ;
le radical aryle ou le radical hétéroaryle pouvant être substitué une à 3 fois avec F, Cl, Br, alkyle en (C₁-C₆) ;
R6 signifie OH ;
R1 signifie 2,2,2-bicyclooctyl,
le radical 2,2,2-bicyclooctyle pouvant être substitué une à 3 fois avec F, Cl, Br ;
R2 signifie H, alkyle en (C₁-C₃) ;
R3 signifie F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkylène en (C₁-C₅)-R9)₂, -NH-alkylène en (C₁-C₆)-NH₂, -NH-alkylène en (C₁-C₆)-NH-alkylène en (C₁-C₆)-(R9), -NH-alkylène en (C₁-C₆)-N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
phényle,
le radical phényle pouvant être substitué une à 3 fois avec F, Cl, Br, CF₃ ;
pyridine, tétrahydropyridine, pipéridine, morpholine, pipérazine,
le radical pyridine, tétrahydropyridine, pipéridine, morpholine ou pipérazine pouvant être substitué une à 3 fois avec F, Cl, Br, CF₃, alkylène en (C₁-C₆)-(R9), COO-alkylène en (C₁-C₆)-(R9), cycloalkyle en (C₃-C₈), oxétane ;
R4, R5, R13 signifient indépendamment les uns des autres
H, F, Cl, Br, I, OH, CF₃, NO₂, CN, OCF₃, alkylène en (C₁-C₆)-(R9), O-alkylène en (C₁-C₆)-(R9, NH₂ ;
R7, R8 signifient indépendamment l'un de l'autre alkyle en (C₁-C₃) ;
R9 signifie H, OH, OCH₃, OCF₃, CHF₂, CF₃ ;
ainsi que leurs sels pharmaceutiquement compatibles.

6. Composés de formule I selon une ou plusieurs des revendications 1 à 5, pour une utilisation en tant que médicament.

7. Médicament contenant des composés de formule I selon une ou plusieurs des revendications 1 à 5.

8. Médicament contenant des composés de formule I selon une ou plusieurs des revendications 1 à 5 et au moins un agent actif supplémentaire.

9. Médicament selon la revendication 8, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption d'acide biliaire, inhibiteurs de CETP, adsorbeurs polymères d'acide biliaire, inducteurs des récepteurs de LDL, inhibiteurs d'ACAT, antioxydants, inhibiteurs de lipoprotéine lipase, inhibiteurs d'ATP citrate lyase, inhibiteurs de squalène synthétase, antagonistes de lipoprotéine (a), agonistes du récepteur HM74A, inhibiteurs de lipase, insulines, sulfonylurées, biguanides, méglitinides, thiazolidinediones, inhibiteurs d'α-glucosidase, agents actifs agissant sur le canal potassique ATP-dépendant des cellules bêta, inhibiteurs de glycogène phosphorylase, antagonistes des récepteurs de glucagon, activateurs de glucokinase, inhibiteurs de gluconéogenèse, inhibiteurs de fructose 1,6-biphosphatase, modulateurs du transporteur de glucose 4, inhibiteurs de glutamine fructose 6-phosphate amidotransférase, inhibiteurs de dipeptidylpeptidase IV, inhibiteurs de 11-bêta-hydroxystéroïde déshydrogénase 1, inhibiteurs de protéine tyrosine phosphatase 1B, modulateurs du transporteur de glucose 1 ou 2 dépendant du sodium, modulateurs de GPR40, inhibiteurs de la lipase hormonosensible, inhibiteurs d'acétyl-CoA carboxylase, inhibiteurs de phosphoénolpyruvate-carboxykinase, inhibiteurs de glycogène synthase kinase-3 bêta, inhibiteurs de protéine kinase C bêta, antagonistes du récepteur d'endothéline A, inhibiteurs de I kappaB kinase, modulateurs du récepteur de glucocorticoïdes, agonistes de CART, agonistes de NPY, agonistes de MC4, agonistes d'orexine, agonistes d'H3, agonistes de TNF, antagonistes de CRF, antagonistes de CRF BP, agonistes d'urocortine, agonistes de β3, antagonistes du récepteur de CB1, agonistes de MSH (hormone mélanotrope), agonistes de CCK, inhibiteurs du recaptage de la sérotonine, composés mixtes sérotoninergiques et noradrénergiques, agonistes de 5HT, agonistes de bombésine, antagonistes de galanine, hormones de croissance, composés libérant des hormones de croissance, agonistes de TRH, modulateurs 2 ou 3 des protéines découplantes, agonistes de leptine, agonistes de DA, inhibiteurs de lipase/amylase, modulateurs de PPAR, modulateurs de RXR ou agonistes de TR-β ou amphétamines.

10. Procédé de fabrication d'un médicament contenant les composés de formule I selon une ou plusieurs des revendications 1 à 5, **caractérisé en ce que** l'agent actif est mélangé avec un support pharmaceutiquement approprié et ce mélange est mis sous une forme appropriée pour l'administration.

11. Utilisation des composés de formule I selon une ou plusieurs des revendications 1 à 5 pour la fabrication d'un médicament pour le traitement de l'hyperglycémie.

12. Utilisation des composés de formule I selon une ou plusieurs des revendications 1 à 5 pour la fabrication d'un médicament pour le traitement du diabète.

13. Utilisation des composés de formule I selon une ou plusieurs des revendications 1 à 5 pour la fabrication d'un médicament pour le traitement de la résistance à l'insuline.

14. Ensemble (kit) constitué d'emballages séparés de
a) une quantité efficace d'un composé de formule I selon une ou plusieurs des revendications 1 à 5 et
b) une quantité efficace d'un agent actif médicamenteux supplémentaire.
